# EUROPEAN PATENT APPLICATION

(11) **EP 4 023 636 A1**
(43) Date of publication of application: **06.07.2022**
(21) Application number: 20857543.1
(22) Date of filing: 28.07.2020
(51) Int. Cl.: C07C 309/06, C07C 309/12, C07C 309/17, C07C 311/14, C07C 311/48, C07C 311/51, C07C 381/12, G03F 7/004, G03F 7/038, G03F 7/039, G03F 7/20

(54) **ACTINIC RAY-SENSITIVE OR RADIATION-SENSITIVE RESIN COMPOSITION, PATTERN FORMATION METHOD, RESIST FILM, AND ELECTRONIC DEVICE PRODUCTION METHOD**

(30) Priority: 26.08.2019 JP 2019153513; 25.06.2020 JP 2020109700
(71) Applicant: FUJIFILM Corporation, Tokyo 106-8620 (JP)
(72) Inventor: KOJIMA, Masafumi, Haibara-gun, Shizuoka 421-0396 (JP); USHIYAMA, Aina, Haibara-gun, Shizuoka 421-0396 (JP); GOTO, Akiyoshi, Haibara-gun, Shizuoka 421-0396 (JP); SHIRAKAWA, Michihiro, Haibara-gun, Shizuoka 421-0396 (JP); KATO, Keita, Haibara-gun, Shizuoka 421-0396 (JP); MARUMO, Kazuhiro, Haibara-gun, Shizuoka 421-0396 (JP); OKA, Hironori, Haibara-gun, Shizuoka 421-0396 (JP)
(74) Representative: HGF
(86) International application number: PCT/JP2020/028844
(87) International publication number: WO 2021/039244

(57) **Abstract**

Provided is an actinic ray-sensitive or radiation-sensitive resin composition that is capable of obtaining a pattern having excellent LWR performance even in a case where the composition has been stored for a long period of time. In addition, also provided are a resist film, a pattern forming method, and a method for manufacturing an electronic device, each relating to the actinic ray-sensitive or radiation-sensitive resin composition. The actinic ray-sensitive or radiation-sensitive resin composition includes an acid-decomposable resin and a specific compound, in which the specific compound has two or more cationic moieties and the same number of anionic moieties as that of the cationic moieties, and at least one of the cationic moieties has a group represented by General Formula (I).

## Description

### BACKGROUND OF THE INVENTION

### 1. Field of the Invention

The present invention relates to an actinic ray-sensitive or radiation-sensitive resin composition, a pattern forming method, a resist film, and a method for manufacturing an electronic device.

### 2. Description of the Related Art

In processes for manufacturing semiconductor devices such as an integrated circuit (IC) and a large scale integrated circuit (LSI), microfabrication by lithography using a photosensitive composition has been performed. Examples of the lithographic method include a method in which a resist film is formed with a photosensitive composition, and then the obtained film is exposed and then developed.

For example, JP2019-008277A discloses a resist composition including the following salt.

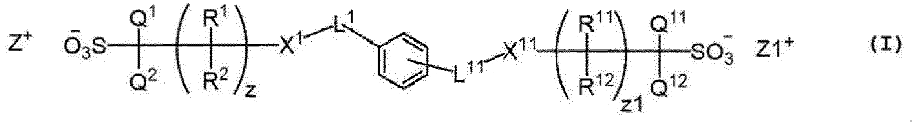

### SUMMARY OF THE INVENTION

The present inventors have specifically investigated the techniques disclosed in JP2019-008277A, and have thus found that in a case where the composition of JP2019-008277A is applied to pattern formation after the composition has been produced and then stored for a long period of time (for example, 3 months), it has room for improvement in the line width roughness (LWR) performance of a pattern thus obtained.

Therefore, an object of the present invention is to provide an actinic ray-sensitive or radiation-sensitive resin composition that is capable of obtaining a pattern having excellent LWR performance even in a case where the composition has been stored for a long period of time.

In addition, another object of the present invention is to provide a resist film, a pattern forming method, and a method for manufacturing an electronic device, each relating to the actinic ray-sensitive or radiation-sensitive resin composition.

The present inventors have found that the objects can be accomplished by the following configurations.
[1] An actinic ray-sensitive or radiation-sensitive resin composition comprising:
   an acid-decomposable resin; and
   a specific compound,
   in which the specific compound has two or more cationic moieties and the same number of anionic moieties as that of the cationic moieties, and at least one of the cationic moieties has a group represented by General Formula (I),

      ^{∗}-Ar^{x}-(RI)ₙᵢ (I)

      in General Formula (I), ^{∗} represents a bonding position,
      Ar^{x} represents an aromatic hydrocarbon ring group,
      ni represents an integer of 1 or more, and
      RI represents a substituent having no cationic functional group.
[2] The actinic ray-sensitive or radiation-sensitive resin composition as described in [1], in which the specific compound has one or more organic cations selected from the group consisting of an organic cation represented by General Formula (II), an organic cation represented by General Formula (III), and an organic cation represented by General Formula (IV), 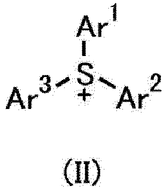
   in General Formula (II), Ar¹, Ar², and Ar³ each independently represent an aromatic hydrocarbon ring group which may have a substituent, and
   at least one of Ar¹, Ar², or Ar³ represents an aromatic hydrocarbon ring group having a substituent having -CF₃,
   provided that all of Ar¹, Ar², and Ar³ have no substituent having a cationic functional group, 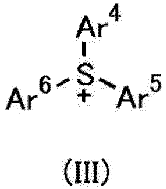
   in General Formula (III), Ar⁴, Ar⁵, and Ar⁶ each independently represent an aromatic hydrocarbon ring group which may have a substituent,
   at least two of Ar⁴, Ar⁵, or Ar⁶ each represent an aromatic hydrocarbon ring group having a substituent,
   at least one of the aromatic hydrocarbon ring groups having a substituent represents an aromatic hydrocarbon ring group having a fluorine atom as the substituent, and
   at least two of Ar⁴, Ar⁵, or Ar⁶ represent groups having different structures,
   provided that all of Ar⁴, Ar⁵, and Ar⁶ have no substituent having a cationic functional group, 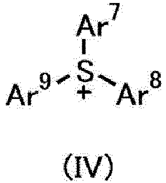
   in General Formula (IV), Ar⁷, Ar⁸, and Ar⁹ each independently represent an aromatic hydrocarbon ring group which may have a substituent,
   at least one of Ar⁷, Ar⁸, or Ar⁹ represents an aromatic hydrocarbon ring group having one or more specific hydrocarbon groups selected from the group consisting of an alkyl group and a polycyclic cycloalkyl group as the substituent,
   provided that all of Ar⁷, Ar⁸, and Ar⁹ have no substituent having a cationic functional group, and
   all of Ar⁷, Ar⁸, and Ar⁹ have no substituent having a fluorine atom.
[3] The actinic ray-sensitive or radiation-sensitive resin composition as described in [2],
   in which in General Formula (II), at least two of Ar¹, Ar², or Ar³ each represent an aromatic hydrocarbon ring group having a substituent.
[4] The actinic ray-sensitive or radiation-sensitive resin composition as described in any one [1] to [3],
   in which the specific compound has an anionic moiety A_{B}⁻ and the anionic moiety A_{B}⁻is a group represented by any of General Formulae (BX-1) to (BX-4), 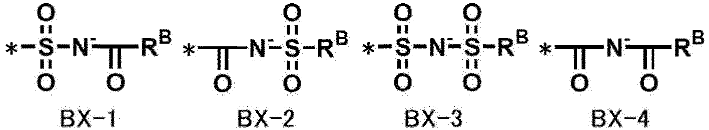 in General Formulae (BX-1) to (BX-4), ^{∗} represents a bonding position, and in General Formulae (BX-1) to (BX-4), R^{B} represents an organic group.
[5] The actinic ray-sensitive or radiation-sensitive resin composition as described in [4],
   in which the specific compound further has an anionic moiety A_{A}⁻ and the anionic moiety A_{A}⁻ is a group represented by either of General Formula (AX-1) and (AX-2), 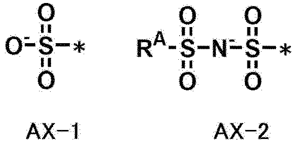
   in General Formulae (AX-1) and (AX-2), ^{∗} represents a bonding position, and
   in General Formula (AX-2), R^{A} represents an organic group.
[6] The actinic ray-sensitive or radiation-sensitive resin composition as described in any one of [1] to [5],
   in which the cationic moiety has at least one acid group.
[7] The actinic ray-sensitive or radiation-sensitive resin composition as described in any one of [1] to [6],
   in which a content of the specific compound is more than 16.0% by mass with respect to a total solid content of the actinic ray-sensitive or radiation-sensitive resin composition.
[8] A resist film formed of the actinic ray-sensitive or radiation-sensitive resin composition as described in any one of [1] to [7].
[9] A pattern forming method comprising:
   a step of forming a resist film on a substrate, using the actinic ray-sensitive or radiation-sensitive resin composition as described in any one of [1] to [7];
   a step of exposing the resist film; and
   a step of developing the exposed resist film, using a developer, to form a pattern.
[10] A method for manufacturing an electronic device, comprising the pattern forming method as described in [9].

According to the present invention, it is possible to provide an actinic ray-sensitive or radiation-sensitive resin composition that is capable of obtaining a pattern having excellent LWR performance even in a case where the composition has been stored for a long period of time.

In addition, the present invention can also provide a resist film, a pattern forming method, and a method for manufacturing an electronic device, each relating to the actinic ray-sensitive or radiation-sensitive resin composition.

### DESCRIPTION OF THE PREFERRED EMBODIMENTS

Hereinafter, an example of a form for carrying out the present invention will be described.

Furthermore, in the present specification, a numerical value range expressed using "to" means a range that includes the preceding and succeeding numerical values of "to" as a lower limit value and an upper limit value, respectively.

In notations for a group (atomic group) in the present specification, in a case where the group is noted without specifying whether it is substituted or unsubstituted, the group includes both a group having no substituent and a group having a substituent. For example, an "alkyl group" includes not only an alkyl group having no substituent (unsubstituted alkyl group), but also an alkyl group having a substituent (substituted alkyl group).

The substituent is preferably a monovalent substituent unless otherwise specified.

An "organic group" in the present specification refers to a group including at least one carbon atom.

In the present specification, examples of the halogen atom include a fluorine atom, a chlorine atom, a bromine atom, and an iodine atom.

The bonding direction of divalent groups noted in the present specification is not limited unless otherwise specified. For example, in a case where Y in a compound represented by General Formula "X-Y-Z" is -COO-, Y may be -CO-O- or -O-CO-. In addition, the compound may be "X-CO-O-Z" or "X-O-CO-Z".

"(Meth)acryl" in the present specification is a generic term encompassing acryl and methacryl, and means "at least one of acryl or methacryl". Similarly, "(meth)acrylic acid" means "at least one of acrylic acid or methacrylic acid".

"Actinic rays" or "radiation" in the present specification means, for example, a bright line spectrum of a mercury lamp, far ultraviolet rays typified by an excimer laser, extreme ultraviolet rays (EUV light), X-rays, electron beams (EB), or the like. "Light" in the present specification means actinic rays or radiation.

Unless otherwise specified, "exposure" in the present specification encompasses not only exposure by a bright line spectrum of a mercury lamp, far ultraviolet rays typified by an excimer laser (an ArF excimer laser and the like), extreme ultraviolet rays (EUV light), X-rays, or the like, but also lithography by particle beams such as electron beams and ion beams.

In the present specification, a weight-average molecular weight (Mw), a number-average molecular weight (Mn), and a dispersity (also referred to as a molecular weight distribution) (Mw/Mn) of a resin are defined as values expressed in terms of polystyrene by means of gel permeation chromatography (GPC) measurement (solvent: tetrahydrofuran, flow amount (amount of a sample injected): 10 µL, columns: TSK gel Multipore HXL-M manufactured by Tosoh Corporation, column temperature: 40°C, flow rate: 1.0 mL/min, and detector: differential refractive index detector) using a GPC apparatus (HLC-8120GPC manufactured by Tosoh Corporation).

1 Å is 1 × 10⁻¹⁰ m.

In the present specification, an acid dissociation constant (pKa) represents a pKa in an aqueous solution, and is specifically a value determined by computation from a value based on a Hammett's substituent constant and database of publicly known literature values, using the following software package 1. Any of the pKa values described in the present specification indicate values determined by computation using the software package.

Software Package 1: Advanced Chemistry Development (ACD/Labs) Software V 8.14 for Solaris (1994 - 2007 ACD/Labs).

On the other hand, the pKa can also be determined by a molecular orbital computation method. Examples of a specific method therefor include a method for performing calculation by computing H⁺ dissociation free energy in a solvent based on a thermodynamic cycle. (Furthermore, in the present specification, water is usually used as the solvent, and in a case where a pKa is not determined with water, dimethyl sulfoxide (DMSO) is used.)

With regard to a computation method for H⁺ dissociation free energy, the H⁺ dissociation free energy can be computed by, for example, density functional theory (DFT), but various other methods have been reported in literature and the like, and are not limited thereto. Furthermore, there are a plurality of software applications capable of performing DFT, and examples thereof include Gaussian 16.

As described above, the pKa in the present specification refers to a value determined by computation from a value based on a Hammett's substituent constant and database of publicly known literature values, using the software package 1, but in a case where the pKa cannot be calculated by the method, a value obtained by Gaussian 16 based on density functional theory (DFT) shall be adopted.

### [Actinic Ray-Sensitive or Radiation-Sensitive Resin Composition]

The actinic ray-sensitive or radiation-sensitive resin composition of an embodiment of the present invention (hereinafter also referred to as a "resist composition") will be described.

The resist composition of the embodiment of the present invention may be either a positive tone resist composition or a negative tone resist composition. In addition, the resist composition may be either a resist composition for alkali development or a resist composition for organic solvent development.

The composition of the embodiment of the present invention is typically a chemically amplified resist composition.

The resist composition of the embodiment of the present invention contains an acid-decomposable resin and a specific compound.

The specific compound has two or more cationic moieties and the same number of anionic moieties as that of the cationic moieties, and at least one of the cationic moieties has a group represented by General Formula (I) which will be described later.

Mechanism by which the objects of the present invention can be accomplished through such configurations is not necessarily clear, but is considered to be as follows by the present inventors.

That is, the specific compound usually acts as a photoacid generator. Since the specific compound has two or more cationic moieties, and anionic moieties in one molecule, it can contribute to the improvement of the LWR performance of a pattern formed. Furthermore, it is considered that in a case where at least one of the cationic moieties has an aromatic hydrocarbon ring group having a substituent, the compatibility between the acid-decomposable resin and the specific compound is improved, and thus, good LWR performance can be maintained even in a case where the resist composition has been stored for a long period of time.

Hereinafter, in the present specification, a fact that a pattern having excellent LWR performance can be obtained even in a case where the resist composition has been stored for a long period of time can also be expressed as follows: the effect of the present invention is excellent.

### [Components of Resist Composition]

Hereinafter, components that can be included in the resist composition will be described in detail.

### <Specific Compound>

The resist composition of the embodiment of the present invention includes a specific compound.

The specific compound usually acts as a photoacid generator. The photoacid generator is a compound that generates an acid upon irradiation (exposure) with actinic rays or radiation (preferably EUV light or ArF).

The specific compound has two or more (preferably two or three) cationic moieties and the same number of anionic moieties as that of the cationic moieties.

Hereinafter, the specific configurations of the cationic moiety and the anionic moiety will be described, and then the specific configurations that the specific compound can take will be described.

### (Cationic Moiety)

The cationic moiety is a structural moiety including a positively charged atom or atomic group, and for example, in a case where the specific compound has an organic cation, the organic cation may be used as the cationic moiety.

### • General Formula (I)

At least one (preferably all) of two or more cationic moieties (preferably organic cations) of the specific compound has a group represented by General Formula (I).

^{∗}-Ar^{x}-(RI)ₙᵢ (I)

In General Formula (I), ^{∗} represents a bonding position. It is preferable that ^{∗} is a bonding position with respect to a cationized atom (S⁺, I⁺, and the like). That is, it is preferable that Ar^{x} in General Formula (I) is directly bonded to a cationized atom (S⁺, I⁺, and the like).

In General Formula (I), Ar^{x} represents an aromatic hydrocarbon ring group.

The aromatic hydrocarbon ring group has no substituent other than -(RI)ni.

The aromatic hydrocarbon ring group may be a monocycle or a polycycle, and is preferably the monocycle.

Examples of the aromatic hydrocarbon ring group include a benzene ring group, a naphthalene ring group, and an anthracene ring group. Among those, the aromatic hydrocarbon ring group is preferably the benzene ring group or the naphthalene ring group, and more preferably the benzene ring group.

In General Formula (I), ni represents an integer of 1 or more.
ni is preferably an integer of 1 to 5.

In General Formula (I), RI represents a substituent having no cationic functional group.

RI which is a substituent is not limited as long as it does not have a cationic functional group (a cationized atom such as >S⁺-, -I⁺-, >C⁺-, or >N⁺<, and a group including the atom), and is preferably, for example, a halogen atom, an alkyl group (which may be linear or branched, and has, for example, 1 to 6 carbon atoms), a cycloalkyl group (which may be a monocycle or a polycycle, and has, for example, 5 to 20 carbon atoms), an alkoxy group (which may be linear or branched, and has, for example, 1 to 6 carbon atoms), or a cycloalkylsulfonyl group (which has a cycloalkyl group moiety that may be a monocycle or a polycycle, and has, for example, 5 to 20 carbon atoms). The RI may have an additional substituent as allowable, and the additional substituent is preferably a halogen atom (preferably a fluorine atom). It is also preferable that such the substituent have no additional substituent other than a halogen atom (for example, a fluorine atom). For example, RI may be a perhalogenated alkyl group (preferably a perfluoroalkyl group) in which an alkyl group is further substituted with a halogen atom. In addition, RI may also be, for example, a cycloalkylalkoxy group. The total number of carbon atoms of one RI (in the case where a substituent is contained, the total number means the number of carbon atoms included in the substituent) is preferably 1 to 20.

In a case where ni is 2 or more, a plurality of RI's that are present may be bonded to each other to form a non-aromatic ring.

It is also preferable that the cationic moiety has at least one (preferably one to six) acid group.

It is also preferable that at least one (preferably all) cationic moiety among the two or more cationic moieties contained in the specific compound is a cationic moiety having at least one (preferably one to six) acid group.

As the acid group which can be contained in the cationic moiety, an acid group having an acid dissociation constant (pKa) of 13 or less is preferable. An acid dissociation constant of the acid group is preferably 13 or less, more preferably 3 to 13, and still more preferably 5 to 10, as described above.

Examples of the acid group include a carboxyl group, a phenolic hydroxyl group, a fluorinated alcohol group (a hexafluoroisopropanol group and the like), a sulfonic acid group, and a sulfonamide group.

As a form in which the cationic moiety has an acid group, it is also preferable that the cationic moiety has an acid group by incorporating an acid group-containing group thereinto. Among those, it is preferable that the cationic moiety has an acid group by adopting an acid group-containing group as at least one RI in General Formula (I).

The acid group-containing group is a general term for an acid group and a group having an acid group in a part thereof, and the acid group-containing group may be a group having the above-mentioned acid group in a part thereof, and may also be a carboxyl group, a phenolic hydroxyl group, a fluorinated alcohol group, or an acid group itself, such as a sulfonic acid group. Furthermore, in a case where RI in General Formula (I) is a hydroxyl group, RI which is the hydroxyl group is directly bonded to Ar^{x} (aromatic hydrocarbon ring group), and thus, RI which is the hydroxyl group is a phenolic hydroxyl group.

Examples of the group having an acid group in a part thereof include "^{∗}-X-RA (^{∗} is a bonding position; X is a divalent linking group such as an ether group; and RA is a monovalent acid group such as a carboxyl group, a fluorinated alcohol group, or a sulfonic acid group)", and "^{∗}-U-XA-R (^{∗} is a bonding position; U is a single bond or a divalent linking group; XA is a divalent acid group such as a sulfonamide group; and R is a substituent such as an alkyl group or an alkylcarbonyl group)".

The total number of atoms other than a hydrogen atom in the acid group-containing group is preferably 1 to 20, and more preferably 1 to 6.

It is preferable that at least one (preferably all) cationic moiety among the two or more cationic moieties of the specific compound has at least one (preferably one to six) acid group-containing group.

It is also preferable that in at least one (preferably all) cationic moiety of the two or more cationic moieties of the specific compound, at least one (preferably one to six) of all RI's of that cationic moiety is an acid group-containing group.

It is also preferable that the cationic moiety has at least one (preferably one to six) acid-decomposable group.

The acid-decomposable group will be described later in the description of the repeating unit having an acid-decomposable group with regard to the acid-decomposable resin (resin (A)).

It is also preferable that at least one (preferably all) cationic moiety among the two or more cationic moieties contained in the specific compound is a cationic moiety having at least one (preferably one to six) acid-decomposable group.

As a form in which the cationic moiety has an acid-decomposable group, it is also preferable that the cationic moiety has an acid-decomposable group by incorporating an acid-decomposable group-containing group. Among those, it is preferable that the cationic moiety has an acid-decomposable group because at least one RI in General Formula (I) is an acid-decomposable group-containing group.

The acid-decomposable group-containing group is a general term for an acid-decomposable group and a group having an acid-decomposable group in a part thereof.

Examples of the group having an acid-decomposable group in a part thereof include "^{∗}-XB-RB (^{∗} is a bonding position; XB is a divalent linking group such as an alkylene group, a thioether group, an ether group, or a combination thereof; and RB is an acid-decomposable group)".

The total number of atoms other than a hydrogen atom in the acid-decomposable group-containing group is preferably 5 to 30, and more preferably 5 to 15.

It is preferable that at least one (preferably all) cationic moiety among the two or more cationic moieties of the specific compound has at least one (preferably one to six) acid-decomposable group-containing group.

It is also preferable that in at least one (preferably all) cationic moiety of the two or more cationic moieties of the specific compound, at least one (preferably one to six) of all RI's of that cationic moiety is an acid-decomposable group-containing group.

At least one (preferably all) of two or more cationic moieties of the specific compound is preferably an organic cation having a group represented by General Formula (I). Hereinafter, the organic cation having the group represented by General Formula (I) is also referred to as a specific organic cation.

The specific organic cation is preferably an organic cation represented by General Formula (II), an organic cation represented by General Formula (III), or an organic cation represented by General Formula (IV).

That is, the specific compound preferably has one or more organic cations selected from the group consisting of the organic cation represented by General Formula (II), the organic cation represented by General Formula (III), and the organic cation represented by General Formula (IV).

The organic cations represented by General Formulae (II) to (IV) have specific substituents and structures, whereby the entire specific compound is hydrophobic. Therefore, the compatibility with the acid-decomposable resin is further improved, the specific compound is likely to be uniformly dispersed even in a resist film formed using the resist composition, and the LWR performance is more excellent. In addition, since the compatibility between the specific compound and the acid-decomposable resin is good, the dispersibility of the specific compound in the resist composition is more excellent, the specific compound is less likely to aggregate during storage of the resist composition, the excellent LWR performance is easily maintained for a long period of time, and the effect of the present invention is more excellent.

### • General Formula (II)

The organic cation represented by General Formula (II) is shown below.

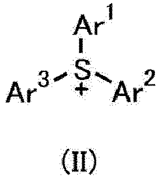

In General Formula (II), Ar¹, Ar², and Ar³ each independently represent an aromatic hydrocarbon ring group which may have a substituent.

Examples of the aromatic hydrocarbon ring group include a benzene ring group, a naphthalene ring group, and an anthracene ring group, and the benzene ring group is preferable.

The substituent which may be contained in the aromatic hydrocarbon ring group is preferably a halogen atom, an alkyl group, a cycloalkyl group, an alkoxy group, the above-mentioned acid group-containing group, or the above-mentioned acid-decomposable group-containing group. The substituent may be of only one kind or of two or more kinds.

The alkyl group may be linear or branched. The alkyl group has, for example, 1 to 6 carbon atoms.

The cycloalkyl group may be a monocycle or a polycycle. The cycloalkyl group has, for example, 5 to 20 carbon atoms.

The alkoxy group may be linear or branched. The alkyl group has, for example, 1 to 6 carbon atoms.

The alkyl group, the cycloalkyl group, and the alkoxy group may each independently have an additional substituent. The additional substituent is preferably a halogen atom (preferably a fluorine atom). It is also preferable that such the substituent have no additional substituent other than a halogen atom (for example, a fluorine atom). The alkyl group may be a perhalogenated alkyl group (preferably a perfluoroalkyl group).

In a case where the aromatic hydrocarbon ring group has a substituent, the number of the substituents of the aromatic hydrocarbon ring groups are each independently preferably 1 to 5.

At least one of Ar¹, Ar², or Ar³ in General Formula (II) is an aromatic hydrocarbon ring group having a substituent, and preferably an aromatic hydrocarbon ring group having at least two (two or three) substituents.

At least one (one to three) of Ar¹, Ar², or Ar³ in General Formula (II) represents an aromatic hydrocarbon ring group having a substituent having -CF₃.

In the substituent having -CF₃, the substituent itself may be -CF₃, or the substituent may have -CF₃ as a part of the entire substituent.

The aromatic hydrocarbon ring group having a substituent having -CF₃ preferably has 1 to 5 substituents having -CF₃.

The aromatic hydrocarbon ring group having a substituent having -CF₃ may further have a substituent having no -CF₃.

One or two of Ar¹, Ar², or Ar³ may be may be aromatic hydrocarbon ring groups having -CF₃ and having no substituent.

The organic cation represented by General Formula (II) preferably has 1 to 8 -CF₃'s which are present in a form where they are directly bonded to a ring member atom of the aromatic hydrocarbon ring group.

Any of the aromatic hydrocarbon ring groups which may have a substituent represented by each of Ar¹, Ar², and Ar³ in General Formula (II) have no substituent having a cationic functional group. In other words, the organic cation represented by General Formula (II) has no cationic functional group other than S⁺ which is directly bonded to Ar¹, Ar², or Ar³.

Furthermore, as for the substituent having a cationic functional group, the substituent itself may be a cationic functional group, or the substituent may have a cationic functional group as a part of the entire substituent.

The organic cation represented by General Formula (II) is preferably different from the organic cation represented by General Formula (III) which will be described later. For example, it is also preferable that the organic cation represented by General Formula (II) has no fluorine atom in a form of being directly bonded to a ring member atom of the aromatic hydrocarbon ring group represented by Ar¹, Ar², or Ar³.

### • General Formula (III)

The organic cation represented by General Formula (III) is shown below.

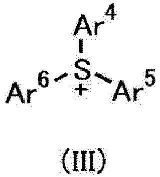

In General Formula (III), Ar⁴, Ar⁵, and Ar⁶ each independently represent an aromatic hydrocarbon ring group which may have a substituent.

The substituent which may be contained in the aromatic hydrocarbon ring group is preferably a halogen atom (preferably a fluorine atom), an alkyl group, a cycloalkyl group, an alkoxy group, the above-mentioned acid group-containing group, or the above-mentioned acid-decomposable group-containing group. The substituent may be of only one kind or of two or more kinds.

The alkyl group may be linear or branched. The alkyl group has, for example, 1 to 6 carbon atoms.

The cycloalkyl group may be a monocycle or a polycycle. The cycloalkyl group has, for example, 5 to 20 carbon atoms.

The alkoxy group may be linear or branched. The alkyl group has, for example, 1 to 6 carbon atoms.

The alkoxy group may be linear or branched. The alkyl group has, for example, 1 to 6 carbon atoms.

The alkyl group, the cycloalkyl group, and the alkoxy group may each independently have an additional substituent.

In a case where the aromatic hydrocarbon ring group has a substituent, the number of the substituents of the aromatic hydrocarbon ring groups are each independently preferably 1 to 5.

At least two (two or three) of Ar⁴, Ar⁵, or Ar⁶ in General Formula (III) is an aromatic hydrocarbon ring group having a substituent.

In addition, at least one (preferably one to three) of Ar⁴, Ar⁵, or Ar⁶ in General Formula (III) is an aromatic hydrocarbon ring group having a fluorine atom as a substituent.

In other words, in Ar⁴, Ar⁵, and Ar⁶ in General Formula (III), at least one (one or two of the two, or one to three of the three) of the "aromatic hydrocarbon ring group having a substituent" among the "aromatic hydrocarbon ring groups having a substituent", which are present in number of at least 2 (2 or 3), is an "aromatic hydrocarbon ring group having a fluorine atom as a substituent".

The "aromatic hydrocarbon ring group having a fluorine atom as a substituent" means an aromatic hydrocarbon ring group having at least one (preferably one to five) fluorine atom (the fluorine atom which is the substituent itself) directly bonded to a ring member atom in the aromatic hydrocarbon ring group.

The "aromatic hydrocarbon ring group having a fluorine atom as a substituent" may have a substituent other than the substituent which is a fluorine atom.

In General Formula (III), at least two of Ar⁴, Ar⁵, or Ar⁶ represent groups having different structures. In other words, all of Ar⁴, Ar⁵, and Ar⁶ are not groups having the same structure.

Furthermore, an expression that all of Ar⁴, Ar⁵, and Ar⁶ are groups having the same structure means that, for example, any of the aromatic hydrocarbon ring groups of Ar⁴, Ar⁵, and Ar⁶ are benzene ring groups, and each of the types and arrangements (bonding positions) of groups contained in the benzene ring groups of Ar⁴, Ar⁵, and Ar⁶ are the same. In other words, it is not mentioned that all of Ar⁴, Ar⁵, and Ar⁶ are groups having the same structure in a case where all of the arrangements of those groups are not the same even with combinations of the types of the groups contained in the benzene ring groups of Ar⁴, Ar⁵, and Ar⁶ being all the same.

Any of the aromatic hydrocarbon ring groups which may have a substituent represented by each of Ar⁴, Ar⁵, and Ar⁶ in General Formula (III) have no substituent having a cationic functional group. In other words, the organic cation represented by General Formula (III) has no cationic functional group other than S⁺ which is directly bonded to Ar⁴, Ar⁵, and Ar⁶.

The organic cation represented by General Formula (III) is preferably different from the organic cation represented by General Formula (II). For example, it is also preferable that any of the aromatic hydrocarbon ring groups which may have a substituent represented by each of Ar⁴, Ar⁵, and Ar⁶ have no substituent having -CF₃.

### • General Formula (IV)

The organic cation represented by General Formula (IV) is shown below.

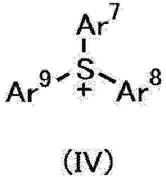

In General Formula (IV), Ar⁷, Ar⁸, and Ar⁹ each independently represent an aromatic hydrocarbon ring group which may have a substituent.

Examples of the substituent which may be contained in the aromatic hydrocarbon ring group include a halogen atom other than a fluorine atom, an alkyl group, a cycloalkyl group, an alkoxy group, the above-mentioned acid group-containing group, or the above-mentioned acid-decomposable group-containing group. The substituent may be of only one kind or of two or more kinds.

The alkyl group may be linear or branched. The alkyl group has, for example, 1 to 6 carbon atoms.

The cycloalkyl group may be a monocycle or a polycycle. The cycloalkyl group has, for example, 5 to 20 carbon atoms.

The alkoxy group may be linear or branched. The alkyl group has, for example, 1 to 6 carbon atoms.

The alkyl group, the cycloalkyl group, and the alkoxy group may each independently have an additional substituent.

In a case where the aromatic hydrocarbon ring group has a substituent, the number of the substituents of the aromatic hydrocarbon ring groups are each independently preferably 1 to 5.

At least one (one to three) of Ar⁷, Ar⁸, or Ar⁹ in General Formula (IV) is an aromatic hydrocarbon ring group having one or more specific hydrocarbon groups selected from the group consisting of an alkyl group and a polycyclic cycloalkyl group as the substituent.

An alkyl group in the specific hydrocarbon group may be linear or branched. The alkyl group has, for example, 1 to 6 carbon atoms.

The polycyclic cycloalkyl group for the specific hydrocarbon group is not particularly limited as long as it is a polycycle, may be a crosslinked ring-based polycycle, a fused ring-based polycycle, or a spiro ring-based polycycle, and may be a polycycle having a plurality of modes of these rings. The number of the ring member atoms of the polycyclic cycloalkyl group is preferably 8 to 20. Examples of the polycyclic cycloalkyl group include a norbornyl group, an adamantyl group, a bicyclooctanyl group, and a tricyclo [5,2,1,0^{2,6}] decanyl group, and the adamantyl group is preferable.

The specific hydrocarbon group may or may not have an additional substituent. It should be noted that the additional substituent is other than a fluorine atom and a group having a fluorine atom in a part thereof.

The specific hydrocarbon group is directly bonded to a ring member atom of the aromatic hydrocarbon ring group.

In a case where the aromatic hydrocarbon ring groups have the specific hydrocarbon groups, the numbers of the specific hydrocarbon groups are each independently preferably 1 to 5.

In addition, the aromatic hydrocarbon ring group having a specific hydrocarbon group may or may not have a substituent other than the specific hydrocarbon group.

Any of the aromatic hydrocarbon ring groups which may have a substituent represented by each of Ar⁷, Ar⁸, and Ar⁹ in General Formula (IV) have no substituent having a cationic functional group. In other words, the organic cation represented by General Formula (IV) has no cationic functional group other than S⁺ which is directly bonded to Ar⁷, Ar⁸, or Ar⁹.

Any of the aromatic hydrocarbon ring groups which may have a substituent represented by Ar⁷, Ar⁸, or Ar⁹ in General Formula (IV) have no substituent having a fluorine atom. In other words, the organic cation represented by General Formula (IV) has no fluorine atom.

That is, a fluorine atom and a group having a fluorine atom in a part thereof are excluded from the substituents which may be contained in the aromatic hydrocarbon ring groups of Ar⁷, Ar⁸, and Ar⁹ in General Formula (IV).

The specific organic cation may be another specific organic cation other than the above-mentioned organic cations represented by General Formulae (II) to (IV).

### • General Formula (ZaII)

Examples of such another specific organic cation include a cation represented by General Formula (ZaII) (cation (ZaII)).

R²⁰⁴-I⁺-R²⁰⁵ (ZaII)

In General Formula (ZaII), R²⁰⁴ and R²⁰⁵ each independently represent an aryl group, an alkyl group, or a cycloalkyl group.

As the aryl group of each of R²⁰⁴ and R²⁰⁵, a phenyl group or a naphthyl group is preferable, and the phenyl group is more preferable. The aryl group of each of R²⁰⁴ and R²⁰⁵ may be an aryl group having a heteroatom, which has an oxygen atom, a nitrogen atom, a sulfur atom, or the like. Examples of the skeleton of the aryl group having a heteroatom include pyrrole, furan, thiophene, indole, benzofuran, and benzothiophene.

As the alkyl group and the cycloalkyl group of each of R²⁰⁴ and R²⁰⁵, a linear alkyl group having 1 to 10 carbon atoms or a branched alkyl group having 3 to 10 carbon atoms (for example, a methyl group, an ethyl group, a propyl group, a butyl group, and a pentyl group), or a cycloalkyl group having 3 to 10 carbon atoms (for example, a cyclopentyl group, a cyclohexyl group, and a norbornyl group) is preferable.

The aryl group, the alkyl group, and the cycloalkyl group of each of R²⁰⁴ and R²⁰⁵ may each independently have a substituent. Examples of the substituent which may be contained in each of the aryl group, the alkyl group, and the cycloalkyl group of each of R²⁰⁴ and R²⁰⁵ include an alkyl group (for example, having 1 to 15 carbon atoms), a cycloalkyl group (for example, having 3 to 15 carbon atoms), an aryl group (for example, having 6 to 15 carbon atoms), an alkoxy group (for example, having 1 to 15 carbon atoms), a halogen atom, a hydroxyl group, a phenylthio group, the above-mentioned acid group-containing group, and the above-mentioned acid-decomposable group-containing group.

It should be noted that at least one of R²⁰⁴ or R²⁰⁵ is an aryl group having a substituent having no cationic functional group and having no heteroatom.

For example, one or both of R²⁰⁴ and R²⁰⁵ are preferably each a group represented by General Formula (I).

### • General Formula (ZaI-3b)

Examples of such another specific organic cation include a cation represented by General Formula (ZaI-3b) (cation (ZaI-3b)).

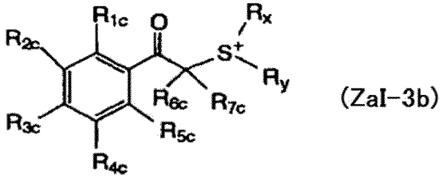

In General Formula (ZaI-3b),
Ric to R_{5c} each independently represent a hydrogen atom, an alkyl group, a cycloalkyl group, an aryl group, an alkoxy group, an aryloxy group, an alkoxycarbonyl group, an alkylcarbonyloxy group, a cycloalkylcarbonyloxy group, a cycloalkylalkoxy group, a halogen atom, a hydroxyl group, a nitro group, an alkylthio group, an arylthio group, and the above-mentioned acid-decomposable group-containing group.

It should be noted that at least one (one to five) of R_{1c} to R_{5c} represents a group other than a hydrogen atom. Furthermore, the group other than a hydrogen atom is not a substituent having a cationic functional group.

R6c and R_{7c} each independently represent a hydrogen atom, an alkyl group (a t-butyl group or the like), a cycloalkyl group, a halogen atom, a cyano group, an aryl group, or the above-mentioned acid-decomposable group-containing group.

Rₓ and R_{y} each independently represent an alkyl group, a cycloalkyl group, a 2-oxoalkyl group, a 2-oxocycloalkyl group, an alkoxycarbonylalkyl group, an allyl group, a vinyl group, or the above-mentioned acid-decomposable group-containing group.

Any two or more of R_{1c} to R_{5c}, R_{5c} and R_{6c}, R_{6c} and R_{7c}, R_{5c} and Rₓ, or Rₓ and R_{y} may each be bonded to each other to form a ring, and the ring may each independently include an oxygen atom, a sulfur atom, a ketone group, an ester bond, or an amide bond.

Examples of the ring include an aromatic or non-aromatic hydrocarbon ring, an aromatic or non-aromatic heterocyclic ring, and a polycyclic fused ring formed by combination of two or more of these rings. Examples of the ring include a 3- to 10-membered ring, and the ring is preferably a 4- to 8-membered ring, and more preferably a 5- or 6-membered ring.

Examples of the group formed by the bonding of any two or more of R_{1c} to R_{5c}, R_{6c} and R_{7c}, and Rₓ and R_{y} include an alkylene group such as a butylene group and a pentylene group. The methylene group in this alkylene group may be substituted with a heteroatom such as an oxygen atom.

As the group formed by the bonding of R_{5c} and R_{6c}, and R_{5c} and Rₓ, a single bond or an alkylene group is preferable. Examples of the alkylene group include a methylene group and an ethylene group.

### • General Formula (ZaI-4b)

Examples of such another specific organic cation include a cation represented by General Formula (ZaI-4b) (cation (ZaI-4b)).

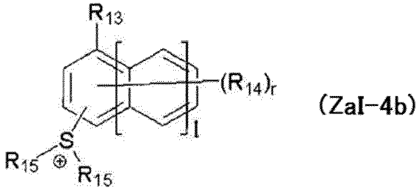

In General Formula (ZaI-4b),
1 represents an integer of 0 to 2.
r represents an integer of 0 to 8.
R₁₃ represents a hydrogen atom, a halogen atom (a fluorine atom, an iodine atom, and the like), a hydroxyl group, an alkyl group, an alkoxy group, an alkoxycarbonyl group, a group having a cycloalkyl group (which may be the cycloalkyl group itself or a group including the cycloalkyl group in a part thereof), or the above-mentioned acid-decomposable group-containing group. These groups may have a substituent.
R₁₄ represents a hydroxyl group, a halogen atom (a fluorine atom, an iodine atom, and the like), an alkyl group (an alkyl halide group is also preferable), an alkoxy group, an alkoxycarbonyl group, an alkylcarbonyl group, an alkylsulfonyl group, a cycloalkylsulfonyl group, a group having a cycloalkyl group (which may be the cycloalkyl group itself or a group including the cycloalkyl group in a part thereof), or the above-mentioned acid-decomposable group-containing group. These groups may have a substituent. In a case where R₁₄'s are present in a plural number, R₁₄'s each independently represent the group such as a hydroxyl group.

It should be noted that General Formula (ZaI-4b) satisfies at least one of a requirement that "R₁₃ is a group other than a hydrogen atom" or a requirement that "r represents an integer of 1 to 8". Furthermore, each group of R₁₃ and R₁₄ is not a substituent having a cationic functional group.

R₁₅'s each independently represent an alkyl group, a cycloalkyl group, a naphthyl group, or the above-mentioned acid-decomposable group-containing group. These groups may have a substituent. Two R₁₅'s may be bonded to each other to form a ring. In a case where two R₁₅'s are bonded to each other to form a ring, the ring skeleton may include a heteroatom such as an oxygen atom and a nitrogen atom.

In one aspect, it is preferable that two R₁₅'s are bonded to each other to form an alkylene group, thereby forming a ring structure. One or more of -CH₂₋'s constituting the alkylene group that forms the ring structure may be substituted with a heteroatom such as an oxygen atom or a nitrogen atom.

In addition, it is also preferable that two R₁₅'s are bonded to each other to form a group represented by "^{∗}-aromatic ring group-L15-aromatic ring group-* (two ^{∗}'s are bonding positions with S⁺; and L15 is a single bond, or a divalent linking group such as an ether group or a thioether group)", thereby forming a ring structure. It is preferable that the aromatic ring groups in the "^{∗}-aromatic ring group-L15-aromatic ring group- ^{∗}" are each independently a phenylene group which may have a substituent.

In General Formula (ZaI-4b), the alkyl groups of each of R₁₃, R₁₄, and R₁₅ are linear or branched. The alkyl group preferably has 1 to 10 carbon atoms. As the alkyl group, a methyl group, an ethyl group, an n-butyl group, a t-butyl group, or the like is more preferable.

### • Organic Cation Other than Specific Organic Cation

The specific compound may have an organic cation other than the specific organic cation as long as at least one of the cationic moieties (preferably the organic cations) has a group represented by General Formula (I).

Examples of the organic cation other than the specific organic cation include an unsubstituted triarylsulfonium cation, an iodonium cation which is the same as the above-mentioned cation (ZaII), except that it does not satisfy a requirement "at least one of R²⁰⁴ or R²⁰⁵ has an aryl group that has a substituent having no cationic functional group and has no heteroatom", a phenacyl-based sulfonium cation which is the same as to the above-mentioned cation (ZaI-3b), except that all of R_{1c} to R_{5c} are hydrogen atoms, and a naphthyl-based sulfonium cation which is the same as the above-mentioned cation (ZaI-4b), except that R₁₃ is a hydrogen atom and r is 0.

The two or more cationic moieties (preferably organic cations, and more preferably specific organic cations) of the specific compound may be the same as or different from each other.

### (Anionic Moiety)

The anionic moiety is a structural moiety including a negatively charged atom or atomic group, and for example, an anionic functional group that may be present in a specific compound may be used as the anionic moiety.

The specific compound preferably has an organic anion having the same number of anionic functional groups as that of the cationic moiety (preferably the organic cation) contained in the specific compound.

As described above, the specific compound has two or more (preferably two or three) cationic moieties and the same number of anionic moieties as that of the cationic moieties.

That is, the specific compound has two or more (preferably two or three) anionic moieties (preferably anionic functional groups).

Examples of the anionic functional group include -SO₃⁻ and a group having -SO₃⁻ in a part thereof, -COO⁻ and a group having -COO⁻ in a part thereof, a group having -N⁻- in a part thereof, and a group having a carbanion (-C⁻<) in a part thereof.

Among those, groups represented by General Formulae (B-1) to (B-13) are preferable as the anionic moiety.

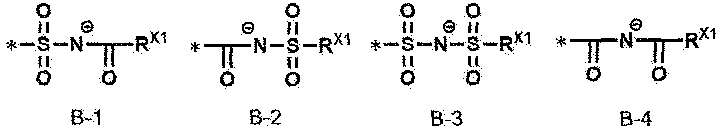

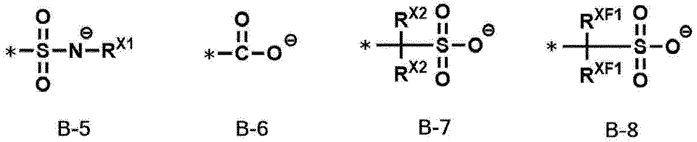

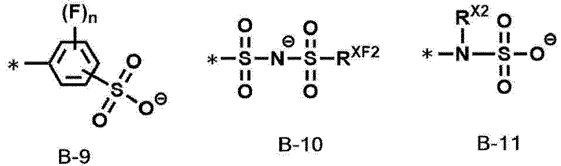

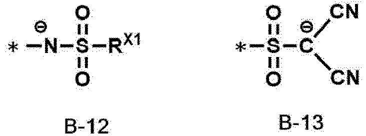

In General Formulae (B-1) to (B-13), ^{∗} represents a bonding position.

Furthermore, it is also preferable that ^{∗} in General Formula (B-12) is a bonding position to a group that is neither -CO- nor -SO₂-.

In General Formulae (B-1) to (B-5), and (B-12), R^{X1} represents an organic group.

As R^{X1}, an alkyl group (which may be linear or branched, and preferably has 1 to 15 carbon atoms), a cycloalkyl group (which may be a monocycle or a polycycle, and preferably has 3 to 20 carbon atoms), an aryl group (which may be a monocycle or a polycycle, and preferably has 6 to 20 carbon atoms), or the above-mentioned acid-decomposable group-containing group is preferable.

Furthermore, in General Formula (B-5), the atom directly bonded to N⁻ in R^{X1} is preferably neither a carbon atom in -CO- nor a sulfur atom in -SO₂-.

The cycloalkyl group in R^{X1} may be a monocycle or a polycycle.

Examples of the cycloalkyl group in R^{X1} include a norbornyl group and an adamantyl group.

The substituent which may be contained in the cycloalkyl group in R^{X1} is preferably an alkyl group (which may be linear or branched, and preferably has 1 to 5 carbon atoms).

One or more of the carbon atoms which are ring member atoms of the cycloalkyl group in R^{X1} may be substituted with carbonyl carbon atoms.

The alkyl group in R^{X1} preferably has 1 to 10 carbon atoms, and more preferably has 1 to 5 carbon atoms.

The substituent which may be contained in the alkyl group in R^{X1} is preferably a cycloalkyl group, a fluorine atom, or a cyano group.

Examples of the cycloalkyl group as the substituent include those of the cycloalkyl group described in a case where R^{X1} is the cycloalkyl group.

In a case where the alkyl group in R^{X1} has a fluorine atom as the substituent, the alkyl group may be a perfluoroalkyl group.

In addition, in the alkyl group in R^{X1}, one or more -CH₂-'s may be substituted with a carbonyl group.

The aryl group in R^{X1} is preferably a benzene ring group.

The substituent which may be contained in the aryl group in R^{X1} is preferably an alkyl group, a fluorine atom, a cyano group, an alkoxycarbonyl group, an iodine atom, or the above-mentioned acid-decomposable group-containing group. Examples of the alkyl group as the substituent include the alkyl group described in the case where R^{X1} is the alkyl group, a perfluoroalkyl group is preferable, and a perfluoromethyl group is more preferable.

R^{X1} may be a group having a group represented by each of General Formulae (B-6) to (B-9), or (B-11). R^{x1} in such a case is preferably a group represented by "-fluoroalkylene group-SO₃⁻". The fluoroalkylene group is preferably a perfluoroalkylene group, and preferably has 1 to 5 carbon atoms. The group represented by General Formula (B-1) to (B-5), or (B-12), in which R^{X1} is a group having a group represented by General Formula (B-6) to (B-9), or (B-11), is singly counted as two anionic moieties (anionic functional groups). In a case where R^{X1} in the group represented by General Formula (B-1) to (B-5), or (B-12) is a group having the group represented by General Formula (B-6) to (B-9), or (B-11), it is preferable that the specific compound further has an anionic moiety (anionic functional group), in addition to the two anionic moieties (anionic functional groups) in the group represented by General Formula (B-1) to (B-5), or (B-12).

In General Formulae (B-7) and (B-11), R^{X2} represents a hydrogen atom, or a substituent other than a fluorine atom and a perfluoroalkyl group.

The substituent other than a fluorine atom and a perfluoroalkyl group, represented by R^{X2}, is preferably an alkyl group other than a perfluoroalkyl group, or a cycloalkyl group.

Examples of the alkyl group include an alkyl group except for a perfluoroalkyl group from the alkyl group in R^{X1}. In addition, it is preferable that the alkyl group has no fluorine atom.

Examples of the cycloalkyl group include the cycloalkyl group in R^{X1}. In addition, the cycloalkyl group preferably has no fluorine atom.

In General Formula (B-8), R^{XF1} represents a hydrogen atom, a fluorine atom, or a perfluoroalkyl group. It should be noted that at least one of the plurality of R^{XF1}'s represents a fluorine atom or a perfluoroalkyl group.

The perfluoroalkyl group represented by R^{XF1} preferably has 1 to 15 carbon atoms, more preferably has 1 to 10 carbon atoms, and still more preferably has 1 to 6 carbon atoms.

In General Formula (B-10), R^{XF2} represents a fluorine atom or a perfluoroalkyl group.

The perfluoroalkyl group represented by R^{XF2} preferably has 1 to 15 carbon atoms, more preferably has 1 to 10 carbon atoms, and still more preferably has 1 to 6 carbon atoms.

In General Formula (B-9), n represents an integer of 0 to 4.

The specific compound preferably has at least two anionic functional groups (preferably two anionic functional groups selected from General Formulae (B-1) to (B-13)). In this case, a combination of the anionic functional groups of the specific compound is not particularly limited.

For example, in a case where the specific compound has the group represented by General Formula (B-8) or (B-10), it may further have the group represented by General Formulae (B-1) to (B-7), (B-9), or (B-11) to (B-13). In addition, in a case where the specific compound is the group represented by General Formula (B-7), it may further have the group represented by General Formula (B-6). These specific compounds may further have different anionic functional groups.

Among those, it is preferable that the specific compound has an anionic moiety A_{B}⁻(anionic functional group A_{B}⁻) as an anionic moiety.

The anionic moiety A_{B}⁻ (anionic functional group A_{B}⁻) is a group represented by any of General Formulae (BX-1) to (BX-4).

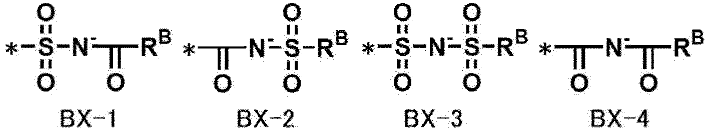

In General Formulae (BX-1) to (BX-4), ^{∗} represents a bonding position.

In General Formulae (BX-1) to (BX-4), R^{B} represents an organic group.

Examples of the organic group in R^{B} include the examples of the organic group in R^{X1} in General Formulae (B-1) to (B-5), and (B-12).

Moreover, it is preferable that the specific compound further has an anionic moiety A_{A}⁻(anionic functional group A_{A}⁻), in addition to the anionic moiety A_{B}⁻ (anionic functional group A_{B}⁻) as an anionic moiety.

The anionic moiety A_{A}⁻ (anionic functional group A_{A}⁻) is a group represented by any of General Formulae (AX-1) to (AX-2).

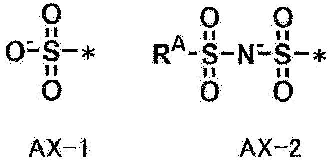

In General Formulae (AX-1) and (AX-2), ^{∗} represents a bonding position.

In General Formula (AX-2), R^{A} represents an organic group.

R^{A} is preferably an alkyl group.

The alkyl group may be linear or branched.

The alkyl group preferably has 1 to 10 carbon atoms, and more preferably has 1 to 5 carbon atoms.

The substituent which may be contained in the alkyl group is preferably a fluorine atom.

The alkyl group having a fluorine atom as the substituent may or may not be a perfluoroalkyl group.

The specific compound may or may not further have an additional anionic moiety (preferably an additional anionic functional group), in addition to the anionic moiety A_{B}⁻

(anionic functional group A_{B}⁻) and the anionic moiety A_{A}⁻ (anionic functional group A_{A}⁻) as an anionic moiety.

### (Configuration of Specific Compound)

The specific compound is preferably a compound represented by General Formula (AD0).

L₀-(A₀⁻)ₙₖ(M₀⁺)ₙₖ General Formula (ADO)

In General Formula (ADO), nk represents an integer of 2 or more.
nk is preferably 2 to 10, and more preferably 2 or 3.

Two nk's that are present in General Formula (ADO) each have the same value.

It should be noted that in a case where N pieces out of nk pieces of anionic functional groups A₀⁻'s are each a "group represented by General Formula (B-1) to (B-5), or (B-12), in which R^{X1} is a group having a group represented by General Formula (B-6) to (B-9), or (B-11)", a value of nk which represents the number of M₀⁺'s in General Formula (ADO) is a value obtained by adding N to nk which represents the number of A₀⁻'s.

In General Formula (ADO), L₀ represents an nk-valent linking group.

It should be noted that in a case where nk is 2, L₀ represents a single bond or a divalent linking group.

Examples of the divalent organic group include -COO-, -CONH-, -CO-, -O-, an alkylene group (which preferably has 1 to 6 carbon atoms, and may be linear or branched), a cycloalkylene group (which preferably has 3 to 15 carbon atoms), an alkenylene group (which preferably has 2 to 6 carbon atoms), and a divalent linking group formed by combination of a plurality of these groups.

The alkylene group and the cycloalkylene group may have the above-mentioned acid-decomposable group-containing group as a substituent.

One or more of the methylene groups constituting a cycloalkane ring of the cycloalkylene group may be substituted with a carbonyl carbon and/or a heteroatom (an oxygen atom and the like).

It is also preferable that the divalent linking group has a group selected from the group consisting of -S-, -SO-, -SO₂-, and -NR^{N}- (R^{N} is a hydrogen atom or substituent).

Examples of the nk-valent linking group include a group obtained by combination of each group which can be taken as a single bond and/or the divalent linking group with -CR^{nk}<, -N<, >C<, a trivalent or higher hydrocarbon ring group, and/or a trivalent or higher heterocyclic group. R^{nk} represents a hydrogen atom or a substituent. The substituent represented by R^{nk} may be the above-mentioned acid-decomposable group-containing group. The hydrocarbon ring group and the heterocyclic group may have the above-mentioned acid-decomposable group-containing group as a substituent.

In a case where L₀ is other than a single bond, the total number of atoms other than a hydrogen atom constituting L₀ is preferably 1 to 100, and more preferably 1 to 50.

In General Formula (ADO), A₀⁻ represents an anionic functional group. The anionic functional group is as described above. A₀⁻'s which are present in a plural number may be the same as or different from each other.

A₀⁻'s which are present in a plural number may have, for example, at least "a group represented by General Formula (B-8) or (B-10) and a group represented by General Formula (B-1) to (B-7), (B-9), or (B-11) to (B-13)", may have at least "a group represented by General Formula (B-7) and a group represented by General Formula (B-6)", and may have at least "a group represented by any of General Formulae (BX-1) to (BX-4) and a group represented by any of General Formulae (AX-1) and (AX-2).

In General Formula (ADO), M₀⁺ represents an organic cation (a specific organic cation or an organic cation other than a specific organic cation).

At least one (preferably all) of nk pieces of M₀⁺'s represents the specific organic cation.

The specific organic cation is as described above.

The organic cation other than the specific organic cation is also as described above.

M₀⁺'s which are present in a plural number may be the same as or different from each other.

Furthermore, the specific compound is preferably a compound (I), a compound (II), or a compound (III).

Hereinafter, each of the compounds (I) to (III) will be described.

### • Compound (I)

The compound (I) will be described.

The compound (I) is the following compound.

Compound (I): a compound having each one of the following structural moiety X and the following structural moiety Y, the compound generating an acid including the following first acidic moiety derived from the following structural moiety X and the following second acidic moiety derived from the following structural moiety Y upon irradiation with actinic rays or radiation.

Structural moiety X: a structural moiety which consists of an anionic moiety A₁⁻ and a cationic moiety M₁⁺, and forms a first acidic moiety represented by HA₁ upon irradiation with actinic rays or radiation

Structural moiety Y: a structural moiety which consists of an anionic moiety A₂⁻ and a cationic moiety M₂⁺, and forms a second acidic moiety represented by HA₂, having a structure different from that of the first acidic moiety formed by the structural moiety X, upon irradiation with actinic rays or radiation

It should be noted that the compound (I) satisfies the following condition I.

Condition I: a compound PI formed by substituting the cationic moiety M₁⁺ in the structural moiety X and the cationic moiety M₂⁺ in the structural moiety Y with H⁺ in the compound (I) has an acid dissociation constant a1 derived from an acidic moiety represented by HA₁, formed by substituting the cationic moiety M₁⁺ in the structural moiety X with H⁺, and an acid dissociation constant a2 derived from an acidic moiety represented by HA₂, formed by substituting the cationic moiety M₂⁺ in the structural moiety Y with H⁺, and the acid dissociation constant a2 is larger than the acid dissociation constant a1.

Furthermore, the acid dissociation constant a1 and the acid dissociation constant a2 are determined by the above-mentioned method. More specifically, with regard to the acid dissociation constant a1 and the acid dissociation constant a2 of the compound PI, in a case where the acid dissociation constant of the compound PI is determined, the pKa with which the compound PI (in which the compound PI corresponds to a "compound having HA₁ and HA₂") serves as a "compound having A₁⁻ and HA₂" is the acid dissociation constant a1, and the pKa with which "compound having A₁⁻ and HA₂" serves as a "compound having A₁⁻ and A₂⁻" is the acid dissociation constant a2.

In addition, the compound PI corresponds to an acid generated by irradiating the compound (I) with actinic rays or radiation.

From the viewpoint that the effect of the present invention is more excellent, a difference between the acid dissociation constant a1 and the acid dissociation constant a2 is preferably 0.10 to 20.00, and more preferably 0.50 to 17.00 in the compound PI.

In addition, from the viewpoint that the effect of the present invention is more excellent, the acid dissociation constant a2 is preferably -4.00 to 15.00, and more preferably -2.00 to 12.00 in the compound PI.

In addition, from the viewpoint that the effect of the present invention is more excellent, the acid dissociation constant a1 is preferably -12.00 to 1.00, and more preferably -7.00 to 0.50 in the compound PI.

### • General Formula (Ia)

The compound (I) is not particularly limited, and examples thereof include a compound represented by General Formula (Ia).

M₁₁⁺A₁₁-L₁-A₁₂⁻M₁₂⁺ (Ia)

In General Formula (Ia), "M₁₁⁺A₁₁⁻" and "A₁₂⁻M₁₂⁺" correspond to the structural moiety X and the structural moiety Y, respectively. The compound (Ia) generates an acid represented by HA₁₁-L₁-A₂₁H upon irradiation with actinic rays or radiation. That is, "M₁₁⁺A₁₁⁻" forms a first acidic moiety represented by HA₁₁, and "A₁₂⁻M₁₂⁺" forms a second acidic moiety represented by HA₁₂, which has a structure different from that of the first acidic moiety.

It should be noted that in the compound PIa (HA₁₁-L₁-A₁₂H) formed by substituting organic cations represented by M₁₁⁺ and M₁₂⁺ with H⁺ in General Formula (Ia), the acid dissociation constant a2 derived from the acidic moiety represented by A₁₂H is larger than the acid dissociation constant a1 derived from the acidic moiety represented by HA₁₁. Furthermore, suitable values of the acid dissociation constant a1 and the acid dissociation constant a2 are as described above.

In General Formula (Ia), M₁₁⁺ and M₁₂⁺ each independently represent an organic cation (a specific organic cation or an organic cation other than a specific organic cation).

At least one (preferably both) of M₁₁⁺ and M₁₂⁺ is the specific organic cation.

The specific organic cation is as described above.

The organic cation other than the specific organic cation is as described above.

In General Formula (Ia), A₁₁⁻ and A₁₂⁻ each independently represent an anionic functional group. It should be noted that A₁₂⁻ represents a structure different from the anionic functional group represented by A₁₁⁻.

The anionic functional group is as described above.

The anionic functional groups of A₁₁⁻ and A₁₂⁻ are each independently preferably a group represented by each of General Formulae (B-1) to (B-13).

A combination of the anionic functional groups represented by A₁₁⁻ and A₁₂⁻ is not particularly limited, but for example, in a case where A₁₁⁻ is a group represented by General Formula (B-8) or (B-10), examples of the anionic functional group represented by A₁₂⁻ include a group represented by General Formula (B-1) to (B-7), (B-9), or (B-11) to (B-13); and in a case where A₁₁⁻ is a group represented by General Formula (B-7), examples of the anionic functional group represented by A₁₂⁻ includes a group represented by General Formula (B-6).

In General Formula (Ia), L₁ represents a divalent linking group.

In General Formula (I), the divalent linking group represented by L₁ is not particularly limited, and examples thereof include -CO-, -NR-, -CO-, -O-, an alkylene group (which preferably has 1 to 6 carbon atoms, and may be linear or branched), a cycloalkylene group (which preferably has 3 to 15 carbon atoms), an alkenylene group (which preferably has 2 to 6 carbon atoms), a divalent aliphatic heterocyclic group (which is preferably a 5- to 10-membered ring, more preferably a 5- to 7-membered ring, and still more preferably a 5- or 6-membered ring, each having at least one of an N atom, an O atom, an S atom, or an Se atom in the ring structure), and a divalent linking group formed by combination of a plurality of these groups. Examples of R include a hydrogen atom or a monovalent substituent. The monovalent substituent is not particularly limited, but is preferably for example, an alkyl group (which preferably has 1 to 6 carbon atoms).

Such a divalent linking group may further include a group selected from the group consisting of -S-, -SO-, and -SO₂-.

In addition, the alkylene group, the cycloalkylene group, the alkenylene group, and the divalent aliphatic heterocyclic group may be substituted with a substituent. Examples of the substituent include a halogen atom (preferably a fluorine atom) and the above-mentioned acid-decomposable group-containing group.

### • General Formula (Ib)

Among those, a compound represented by General Formula (Ib) is preferable as the compound (I).

M₁⁺A⁻-L-B⁻M₂⁺ (Ib)

In General Formula (Ib), M₁⁺ and M₂⁺ each independently represent an organic cation (a specific organic cation or an organic cation other than a specific organic cation).

At least one (preferably all) of M₁⁺ or M₂⁺ represents the specific organic cation.

The specific organic cation is as described above.

The organic cation other than the specific organic cation is also as described above.

In General Formula (Ib), L represents a divalent organic group.

Examples of the divalent organic group include -COO-, -CONH-, -CO-, -O-, an alkylene group (which preferably has 1 to 6 carbon atoms, and may be linear or branched), a cycloalkylene group (which preferably has 3 to 15 carbon atoms), an alkenylene group (which preferably has 2 to 6 carbon atoms), and a divalent linking group formed by combination of a plurality of these groups.

The alkylene group and the cycloalkylene group may have the above-mentioned acid-decomposable group-containing group as a substituent.

One or more of the methylene groups constituting a cycloalkane ring of the cycloalkylene group may be substituted with a carbonyl carbon and/or a heteroatom (an oxygen atom and the like).

It is also preferable that the divalent linking group has a group selected from the group consisting of -S-, -SO-, and -SO₂-.

Among those, L is preferably a group represented by General Formula (L).

^{∗}A-LA-LB-LC-LD-LE-^{∗}B (L)

In General Formula (L), ^{∗}A represents a bonding position to A⁻ in General Formula (Ib).

In General Formula (L), ^{∗}B represents a bonding position to B⁻ in General Formula (Ib).

In General Formula (L), LA represents -(C(R_{LA1})(R_{LA2}))_{XA}-.

XA represents an integer of 1 or more, and is preferably 1 to 10, and more preferably 1 to 3.

R_{LA1} and R_{LA2} each independently represent a hydrogen atom or a substituent.

The substituents of R_{LA1} and R_{LA2} are each independently preferably a fluorine atom or a fluoroalkyl group, more preferably the fluorine atom or a perfluoroalkyl group, and still more preferably the fluorine atom or a perfluoromethyl group.

As the substituent represented by each of R_{LA1} and R_{LA2}, the above-mentioned acid-decomposable group is also preferable.

In a case where XA is 2 or more, XA pieces of R_{LA1}'s may be the same as or different from each other.

In a case where XA is 2 or more, XA pieces of R_{LA2}'s may be the same as or different from each other.
-(C(R_{LA1})(R_{LA2}))- is preferably -CH₂-, -CHF-, -CH(CF₃)-, or -CF₂-.

Among those, -(C(R_{LA1})(R_{LA2}))- which is directly bonded to A⁻ in General Formula (Ib) is preferably -CH₂-, -CHF-, -CH(CF₃)-, or -CF₂-.
-(C(R_{LA1})(R_{LA2}))-'s other than -(C(R_{LA1})(R_{LA2}))- which is directly bonded to A⁻ in General Formula (Ib) are each independently preferably -CH₂-, -CHF-, or -CF₂-.

In General Formula (L), LB represents a single bond, an ester group (-COO-), or a sulfonyl group (-SO₂-).

In General Formula (L), LC represents a single bond, an alkylene group, a cycloalkylene group, or a group formed by combination thereof ("-alkylene group-cycloalkylene group-" and the like).

The alkylene group may be linear or branched.

The alkylene group preferably has 1 to 5 carbon atoms, more preferably has 1 or 2 carbon atoms, and still more preferably has one carbon atom.

The cycloalkylene group preferably has 3 to 15 carbon atoms, and more preferably has 5 to 10 carbon atoms.

The cycloalkylene group may be a monocycle or a polycycle.

Examples of the cycloalkylene group include a norbornanediyl group and an adamantandiyl group.

As a substituent which may be contained in the cycloalkylene group, an alkyl group (which may be linear or branched, and preferably has 1 to 5 carbon atoms) is preferable.

One or more of the methylene groups constituting a cycloalkane ring of the cycloalkylene group may be substituted with a carbonyl carbon and/or a heteroatom (an oxygen atom and the like).

In a case where LC is "-alkylene group-cycloalkylene group-", the alkylene group moiety is preferably present on the LB side.

The alkylene group and the cycloalkylene group may have the above-mentioned acid-decomposable group-containing group as a substituent.

In a case where the LB is the single bond, LC is preferably the single bond or the cycloalkylene group.

In General Formula (L), LD represents a single bond, an ether group (-O-), a carbonyl group (-CO-), or an ester group (-COO-).

In General Formula (L), LE represents a single bond or -(C(R_{LE1})(R_{LE2}))_{XE}-.

XE in -(C(R_{LE1})(R_{LE2}))_{XE}- represents an integer of 1 or more, and is preferably 1 to 10, and more preferably 1 to 3.

R_{LE1} and R_{LE2} each independently represent a hydrogen atom or a substituent.

In a case where XE is 2 or more, XE pieces of R_{LE1}'s may be the same as or different from each other.

In a case where XE is 2 or more, XE pieces of R_{LE2}'s may be the same as or different from each other.

As the substituent represented by each of R_{LE1} and R_{LE2}, the above-mentioned acid-decomposable group is also preferable.

Among those, -(C(R_{LE1})(R_{LE2}))- is preferably -CH₂- or -CF₂-.

In a case where LB, LC, and LD are the single bonds in General Formula (L), it is preferable that LE is also the single bond.

In General Formula (Ib), A⁻ and B⁻ each independently represent an anionic functional group.

The anionic functional group is as described above.

Among those, A⁻ is preferably a group represented by either of General Formulae (AX-1) and (AX-2).

B⁻ preferably represents a group represented by any of General Formulae (BX-1) to (BX-4).

It is preferable that A⁻ and B⁻ have different structures.

Among those, it is preferable that A⁻ is the group represented by General Formula (AX-1) and B⁻ is the group represented by any of General Formulae (BX-1) to (BX-4), or that A⁻ is the group represented by General Formula (AX-2) and B⁻ is the group represented by any of General Formulae (BX-1), (BX-3), and (BX-4).

It should be noted that in a compound represented by HA-L-BH in which M₁⁺ and M₂⁺ of the compound represented by General Formula (Ib) are each substituted with a hydrogen atom, a pKa of a group represented by HA is lower than a pKa of a group represented by BH.

More specifically, in a case where an acid dissociation constant is determined for the compound represented by HA-L-BH, the pKa in a case where "HA-L-BH" serves as "A⁻-L-BH" is defined as the "pKa of a group represented by HA", and the pKa in a case where "A⁻-L-BH" serves as "A⁻-L-B⁻" is defined as the "pKa of the group represented by BH".

The "pKa of the group represented by HA" and the "pKa of the group represented by BH" are each determined using "Software Package 1" or "Gaussian 16".

For example, the pKa of the group represented by HA corresponds to the above-mentioned acid dissociation constant a1, and a preferred range thereof is also the same.

The pKa of the group represented by BH corresponds to the above-mentioned acid dissociation constant a2, and a preferred range thereof is also the same.

A difference between the pKa of the group represented by HB and the pKa of the group represented by HA ("pKa of the group represented by HB" - "pKa of the group represented by HA") corresponds to a difference between the acid dissociation constant a1 and the acid dissociation constant a2, and a preferred range thereof is also the same.

### • Compound (II)

Next, the compound (II) will be described.

The compound (II) is the following compound.

Compound (II): a compound having the two or more structural moieties X and the structural moiety Y, the compound generating an acid including the two or more first acidic moieties derived from the structural moieties X and the second acidic moiety derived from the structural moiety Y upon irradiation with actinic rays or radiation

It should be noted that the compound (II) satisfies the following condition II.

Condition II: a compound PII formed by substituting the cationic moiety M₁⁺ in the structural moiety X and the cationic moiety M₂⁺ in the structural moiety Y with H⁺ in the compound (II) has an acid dissociation constant a1 derived from an acidic moiety represented by HA₁, formed by substituting the cationic moiety M₁⁺ in the structural moiety X with H⁺ and an acid dissociation constant a2 derived from an acidic moiety represented by HA₂, formed by substituting the cationic moiety M₂⁺ in the structural moiety Y with H⁺, and the acid dissociation constant a2 is larger than the acid dissociation constant a1.

The acid dissociation constant a1 and the acid dissociation constant a2 are determined by the above-mentioned method.

Here, the acid dissociation constant a1 and the acid dissociation constant a2 of the compound PII will be more specifically described. In a case where the compound (II) is, for example, a compound that generates an acid having two of the first acidic moieties derived from the structural moiety X and one of the second acidic moieties derived from the structural moiety Y, the compound PII corresponds to a "compound having two HA₁'s and HA₂". In a case where the acid dissociation constant of the compound PII was determined, the pKa in a case where the compound PII serves as a "compound having one A₁⁻, one HA₁, and HA₂" is the acid dissociation constant a1, and the pKa in a case where the compound having two A₁⁻'s and HA₂ serves as a "compound having two A₁⁻'s and A₂⁻" is the acid dissociation constant a2. That is, in a case where the compound PII has a plurality of acid dissociation constants derived from the acidic moiety represented by HA₁, formed by substituting the cationic moiety M₁⁺ in the structural moiety X with H⁺, the smallest value is considered as the acid dissociation constant a1.

In addition, the compound PII corresponds to an acid generated by irradiating the compound (II) with actinic rays or radiation.

Furthermore, the compound (II) may have a plurality of the structural moieties Y.

From the viewpoint that the effect of the present invention is more excellent, in the compound PII, the difference between the acid dissociation constant a1 and the acid dissociation constant a2 is preferably 2.00 or more, and more preferably 3.00 or more. Furthermore, an upper limit value of the difference between the acid dissociation constant a1 and the acid dissociation constant a2 is not particularly limited, but is, for example, 15.00 or less.

In addition, from the viewpoint that the effect of the present invention is more excellent, in the compound PII, the acid dissociation constant a2 is preferably 2.00 or less, and more preferably 1.00 or less. Furthermore, a lower limit value of the acid dissociation constant a2 is preferably -2.00 or more.

In addition, from the viewpoint that the effect of the present invention is more excellent, in the compound PII, the acid dissociation constant a1 is preferably 2.00 or less, more preferably 0.50 or less, and still more preferably -0.10 or less. Furthermore, a lower limit value of the acid dissociation constant a1 is preferably -15.00 or more.

The compound (II) is not particularly limited, and examples thereof include a compound represented by General Formula (IIa).

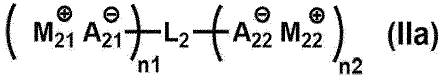

In General Formula (IIa), "M₂₁⁺A₂₁⁻" and "A₂₂⁻M₂₂⁺" correspond to the structural moiety X and the structural moiety Y, respectively. The compound (IIa) generates an acid represented by General Formula (IIa-1) upon irradiation with actinic rays or radiation. That is, "M₂₁⁺A₂₁⁻" forms a first acidic moiety represented by HA₂₁, and "A₂₂⁻M₂₂⁺" forms a second acidic moiety represented by HA₂₂ having a structure different from that of the first acidic moiety.

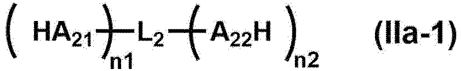

In General Formula (IIa), M₂₁⁺ and M₂₂⁺ each independently represent an organic cation.

A₂₁⁻ and A₂₂⁻ each independently represent an anionic functional group. It should be noted that A₂₂⁻ represents a structure different from the anionic functional group represented by A₂₁⁻.
L₂ represents a (n1 + n2) valent organic group.
n1 represents an integer of 2 or more.
n2 represents an integer of 1 or more.

It should be noted that in the compound PIIa (corresponding to a compound represented by General Formula (IIA-1)), formed by substituting organic cations represented by M₂₁⁺ and M₂₂⁺ with H⁺ in General Formula (IIa), the acid dissociation constant a2 derived from the acidic moiety represented by A₂₂H is larger than the acid dissociation constant a1 derived from the acidic moiety represented by HA₂₁. Furthermore, suitable values of the acid dissociation constant a1 and the acid dissociation constant a2 are as described above.

In General Formula (IIa), M₂₁⁺, M₂₂⁺, A₂₁⁻, and A₂₂⁻ have the same definitions as M₁₁⁺, M₁₂⁺, A₁₁⁻, and A₁₂⁻ in General Formula (Ia), respectively, and suitable aspects thereof are also the same.

In General Formula (IIa), n1 pieces of M₂₁⁺ and n1 pieces of A₂₁⁺ represent the same group as each other.

In General Formula (IIa), the (n1 + n2)-valent organic group represented by L₂ is not particularly limited, and examples thereof include groups represented by (A1) and (A2) below. Furthermore, in (A1) and (A2) below, at least two of ^{∗}'s represent bonding positions to A₂₁⁻, and at least one of ^{∗}'s represents a bonding position to A₂₂⁻.

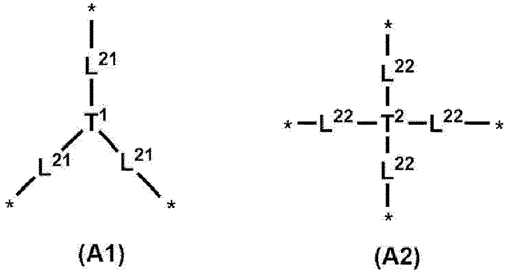

In (A1) and (A2) above, T¹ represents a trivalent hydrocarbon ring group or a trivalent heterocyclic group, and T² represents a carbon atom, a tetravalent hydrocarbon ring group, or a tetravalent heterocyclic group.

The hydrocarbon ring group may be an aromatic hydrocarbon ring group or an aliphatic hydrocarbon ring group. The number of carbon atoms included in the hydrocarbon ring group is preferably 6 to 18, and more preferably 6 to 14.

The heterocyclic group may be either an aromatic heterocyclic group or an aliphatic heterocyclic group. The heterocyclic ring is preferably a 5- to 10-membered ring, more preferably a 5- to 7-membered ring, and still more preferably a 5- or 6-membered ring, each of which has at least one N atom, O atom, S atom, or Se atom in the ring structure.

The above-mentioned hydrocarbon ring group and the above-mentioned heterocyclic group may have the above-mentioned acid-decomposable group-containing group as a substituent.

In addition, in (A1) and (A2), L²¹ and L²² each independently represent a single bond or a divalent linking group.

The divalent linking group represented by each of L²¹ and L²² has the same definition as the divalent linking group represented by L₁ in General Formula (Ia), and a suitable aspect thereof is also the same.

n1 represents an integer of 2 or more. An upper limit thereof is not particularly limited, but is, for example, 6 or less, preferably 4 or less, and more preferably 3 or less.

n2 represents an integer of 1 or more. An upper limit thereof is not particularly limited, but is, for example, 3 or less, and preferably 2 or less.

### • Compound (III)

Next, the compound (III) will be described.

Compound (III): a compound having the two or more structural moieties X and the following structural moiety Z, the compound generating an acid including the two or more first acidic moieties derived from the structural moieties X and the structural moiety Z upon irradiation with actinic rays or radiation

Structural moiety Z: a nonionic moiety capable of neutralizing an acid

The nonionic moiety capable of neutralizing an acid in the structural moiety Z is not particularly limited, and is preferably, for example, a moiety including a functional group having a group or electron which is capable of electrostatically interacting with a proton. It is also preferable that the moiety is an organic moiety having a carbon atom.

Examples of the functional group having a group or electron capable of electrostatically interacting with a proton include a functional group with a macrocyclic structure, such as a cyclic polyether, or a functional group having a nitrogen atom having an unshared electron pair not contributing to π-conjugation. The nitrogen atom having an unshared electron pair not contributing to π-conjugation is, for example, a nitrogen atom having a partial structure represented by the following formula.

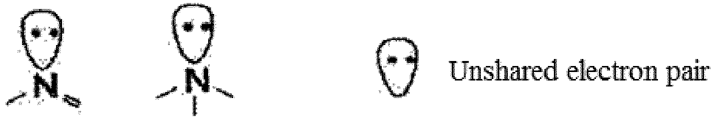

Examples of the partial structure of the functional group having a group or electron capable of electrostatically interacting with a proton include a crown ether structure, an azacrown ether structure, primary to tertiary amine structures, a pyridine structure, an imidazole structure, and a pyrazine structure, and among these, the primary to tertiary amine structures are preferable.

In the compound PIII formed by substituting the cationic moiety M₁⁺ in the structural moiety X with H⁺ in the compound (III), the acid dissociation constant a1 derived from the acidic moiety represented by HA₁, formed by substituting the cationic moiety M₁⁺ in the structural moiety X with H⁺, is preferably 2.0 or less, more preferably 0.5 or less, and still more preferably -0.1 or less, from the viewpoint that the effect of the present invention is more excellent. Furthermore, a lower limit value of the acid dissociation constant a1 is preferably -15.0 or more.

Furthermore, in a case where the compound PIII has a plurality of acid dissociation constants derived from the acidic moiety represented by HA₁, formed by substituting the cationic moiety M₁⁺ in the structural moiety X with H⁺, the smallest value is considered as the acid dissociation constant a1.

That is, in a case where the compound (III) is, for example, a compound that generates an acid having two of the first acidic moieties derived from the structural moiety X and the structural moiety Z, the compound PIII corresponds to a "compound having two of HA₁". In a case where the acid dissociation constant of this compound PIII is determined, the pKa in a case where the compound PIII serves as a "compound having one of A₁⁻ and one of HA₁" is the acid dissociation constant a1. That is, in a case where the compound PIII has a plurality of acid dissociation constants derived from the acidic moiety represented by HA₁, formed by substituting the cationic moiety M₁⁺ in the structural moiety X with H⁺, the smallest value is considered as the acid dissociation constant a1.

Furthermore, for example, in a case where the compound (III) is a compound represented by the compound (IIIa) which will be described later, the compound PIII formed by substituting the cationic moiety M₁⁺ in the structural moiety X with H⁺ in the compound (III) corresponds to HA₃₁-L₃-N(R^{2X})-L₄-A₃₁H.

The compound (III) is not particularly limited, and examples thereof include a compound represented by General Formula (IIIa).

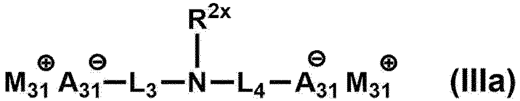

In General Formula (IIIa), "M₃₁⁺A₃₁⁻" corresponds to the structural moiety X. The compound (IIIa) generates an acid represented by HA₃₁-L₃-N(R^{2X})-L₄-A₃₁H upon irradiation with actinic rays or radiation. That is, "M₃₁⁺A₃₁⁻" forms the first acidic moiety represented by HA₃₁.

In General Formula (IIIa), M₃₁⁺ represents an organic cation.

A₃₁" represents an anionic functional group.

L₃ and L₄ each independently represent a divalent linking group.

R^{2X} represents a monovalent organic group.

In General Formula (IIIa), M₃₁⁺ and A₃₁⁻ have the same definitions as M₁₁⁺ and A₁₁⁻ in General Formula (Ia), respectively, and suitable aspects thereof are also the same.

In General Formula (IIIa), L₃ and L₄ have the same definition as L₁ in General Formula (Ia), and suitable aspects thereof are also the same.

In General Formula (IIIa), two M₃₁⁺'s and two A₃₁⁻'s represent the same group as each other.

In General Formula (IIIa), the monovalent organic group represented by R^{2X} is not particularly limited, and examples thereof include an alkyl group (which preferably has 1 to 10 carbon atoms, and may be linear or branched), a cycloalkyl group (which preferably has 3 to 15 carbon atoms), and an alkenyl group (which preferably has 2 to 6 carbon atoms), in which -CH₂- may be substituted with one or a combination of two or more selected from the group consisting of -CO-, -NH-, -O-, -S-, -SO-, and -SO₂-. In addition, the alkylene group, the cycloalkylene group, and the alkenylene group may be substituted with a substituent. The alkylene group, the cycloalkylene group, and the alkenylene group may have the above-mentioned acid-decomposable group-containing group as a substituent.

The molecular weight of the specific compound is preferably 300 to 3,000, more preferably 500 to 2,000, and still more preferably 700 to 1,500.

A lower limit of the content of the specific compound is preferably 0.1% by mass or more, more preferably 1.0% by mass or more, still more preferably 4.0% by mass or more, and particularly preferably more than 16.0% by mass with respect to the total solid content of the composition. An upper limit of the content of the specific compound is preferably 70.0% by mass or less, more preferably 50.0% by mass or less, still more preferably 30.0% by mass or less, and particularly preferably 27.0% by mass or less.

In particular, in a case where the content is more than 16.0% by mass, the resolution of the obtained pattern is more excellent.

In addition, in a case where the composition includes the compound (II) as the specific compound, a lower limit of the content of the compound (II) (a total content thereof the compounds (II) in a case where plural kinds of the compounds are included) is preferably 5. 0% by mass or more, more preferably more than 16.0% by mass, and still more preferably more than 20.0% by mass with respect to the total solid content of the composition. An upper limit of the content of the compound (II) is preferably 70.0% by mass or less, and more preferably 60.0% by mass or less.

Furthermore, the solid content is intended to mean a component forming a resist film, and does not include a solvent. In addition, any of components that form a resist film are regarded as a solid content even in a case where they have a property and a state of a liquid.

The specific compounds may be used singly or in combination of two or more kinds thereof. In a case where two or more kinds of the specific compounds are used, a total content thereof is preferably within the suitable content range.

The organic cation in the specific compound is exemplified below.

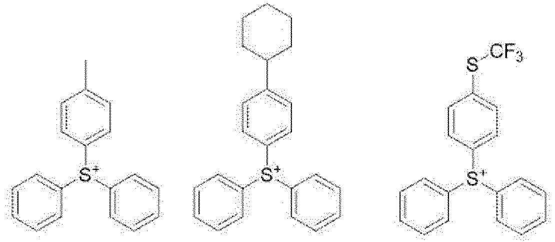

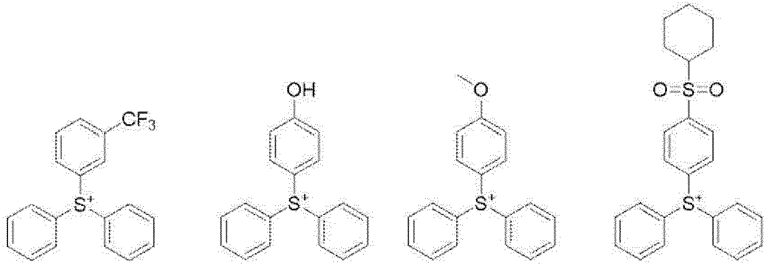

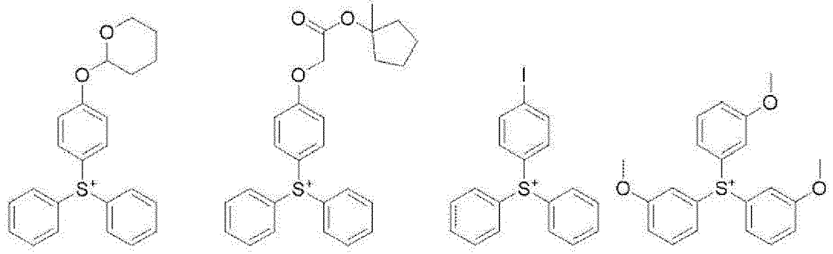

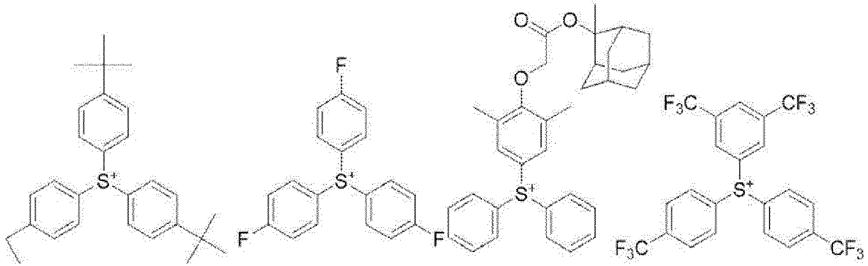

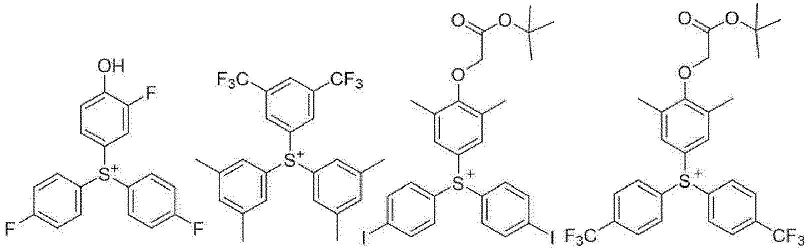

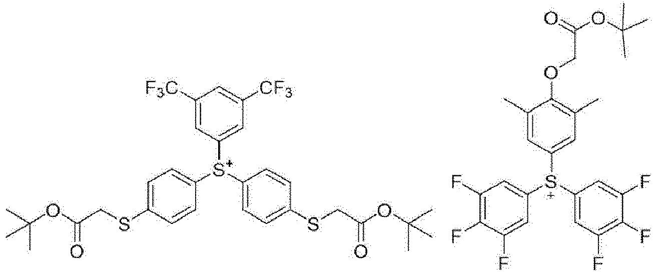

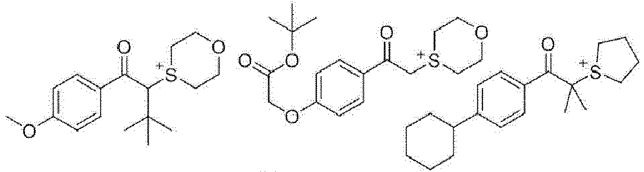

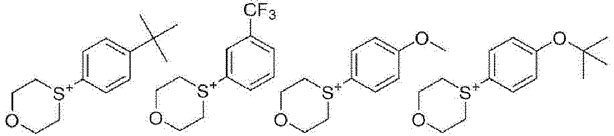

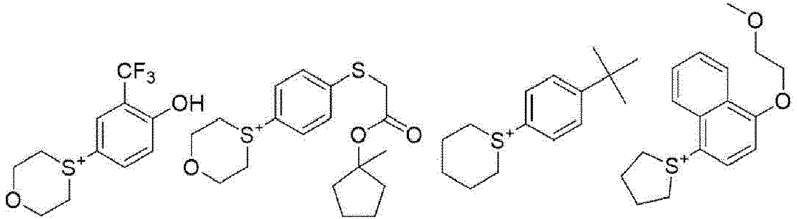

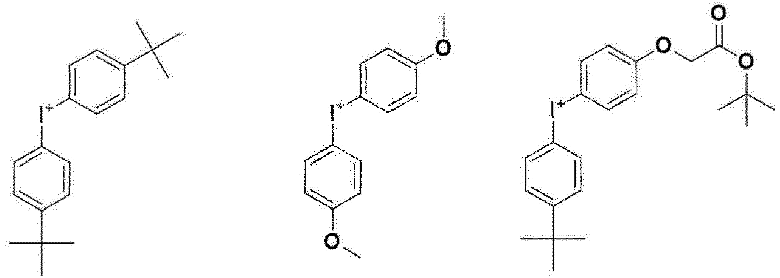

The organic anions in the specific compound are illustrated below.

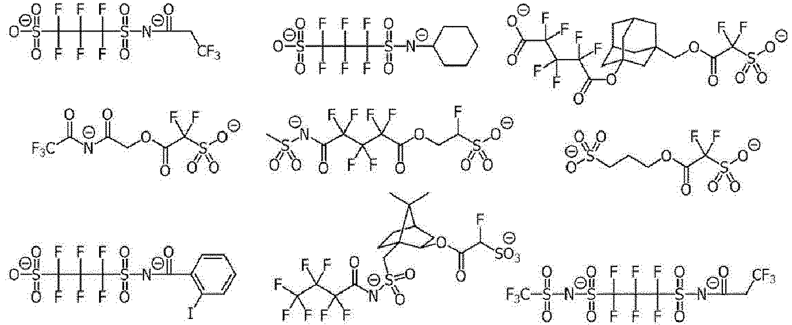

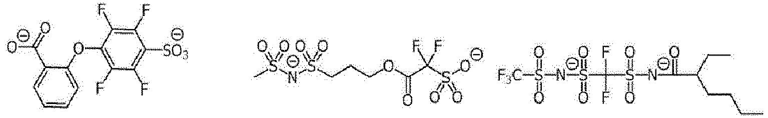

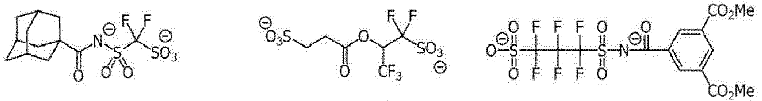

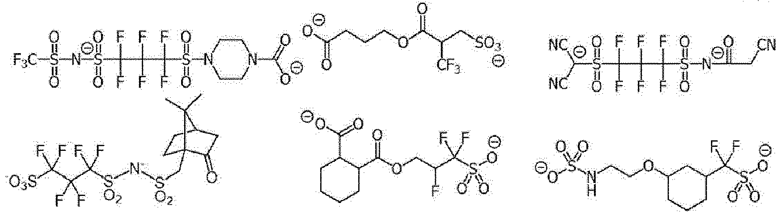

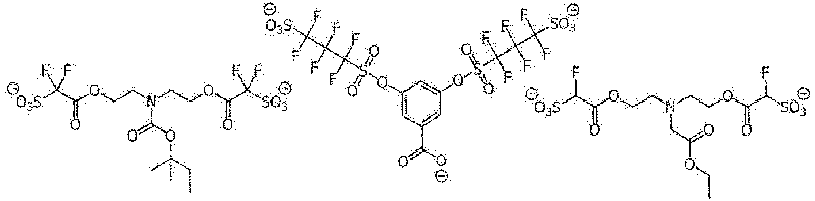

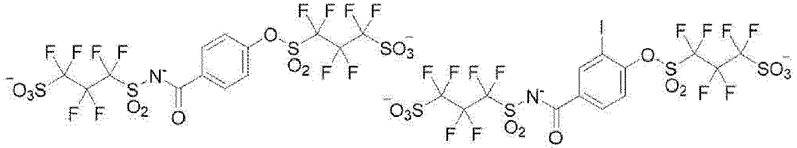

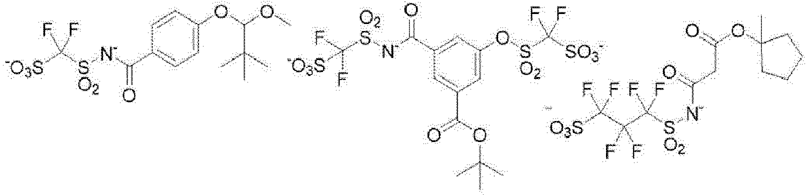

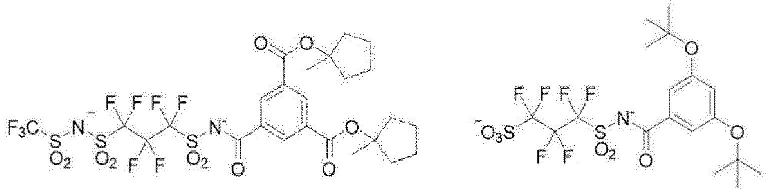

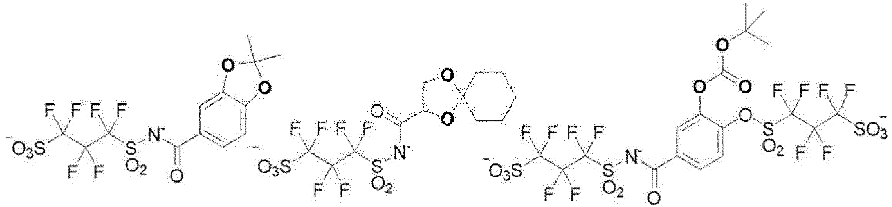

The specific compound is exemplified below.

The combination of the organic cation and the organic anion in the following specific compound may be replaced as appropriate.

The numerical value attached to the organic anion of the specific compound indicates an acid dissociation constant derived from each acidic moiety formed in a case where each anionic functional group in the organic anion is bonded to H⁺ instead of the organic cation.

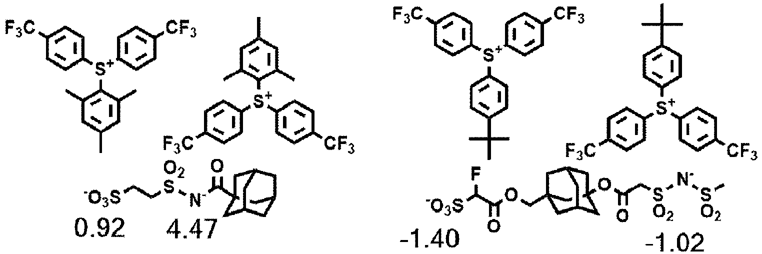

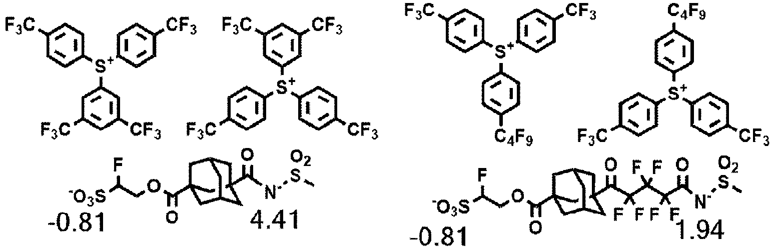

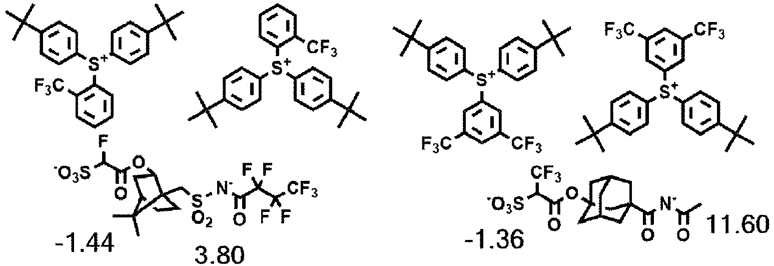

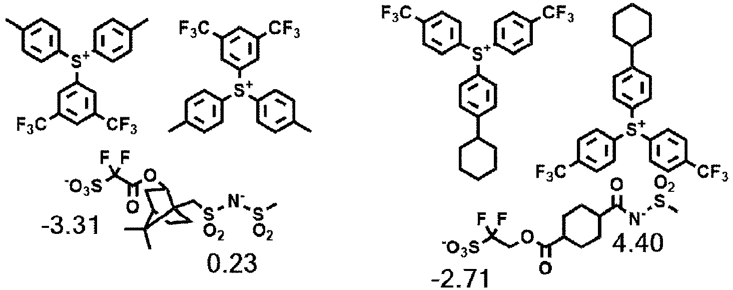

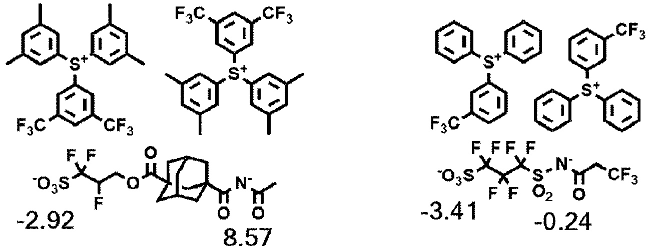

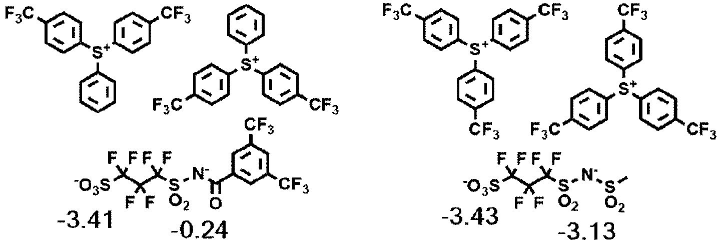

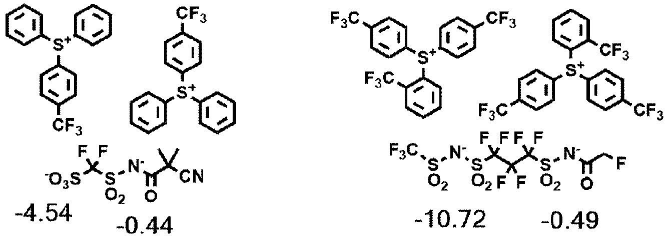

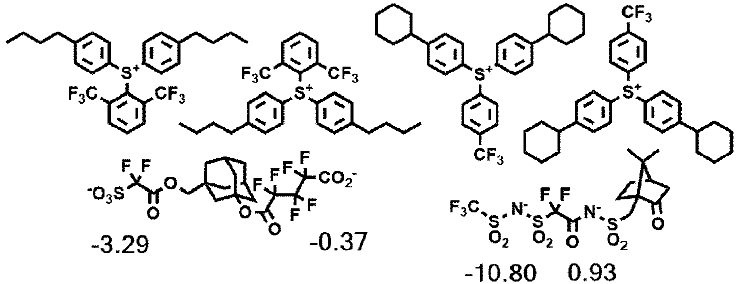

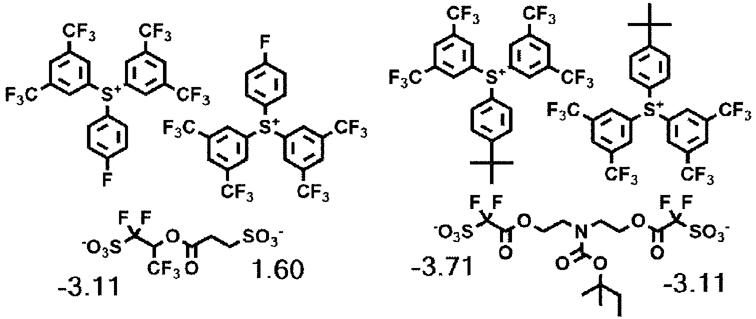

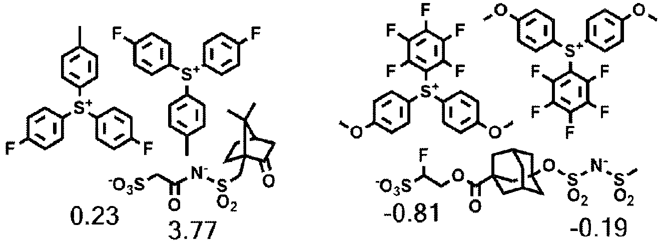

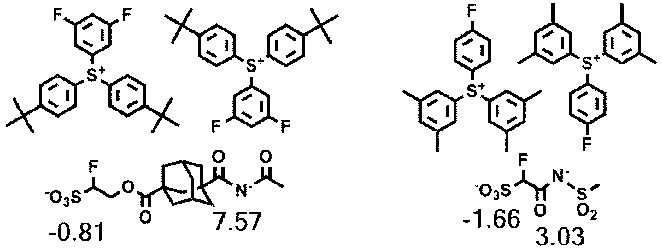

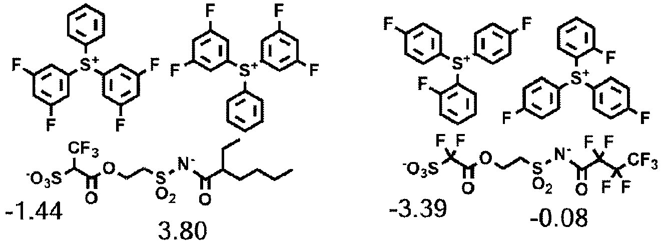

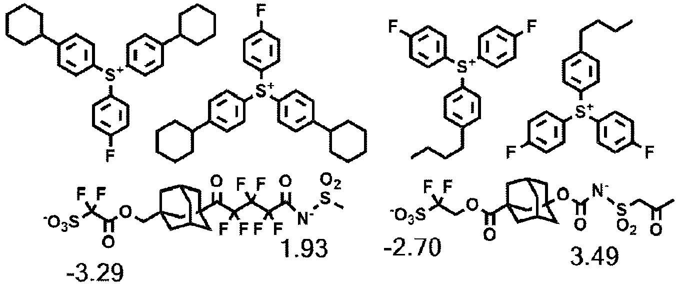

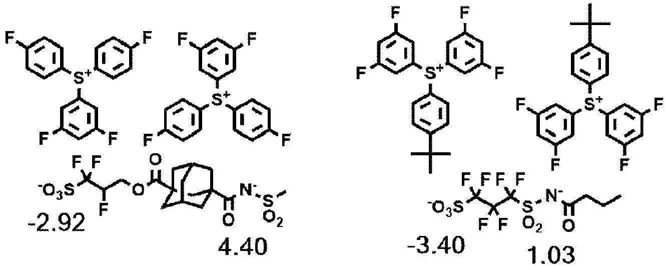

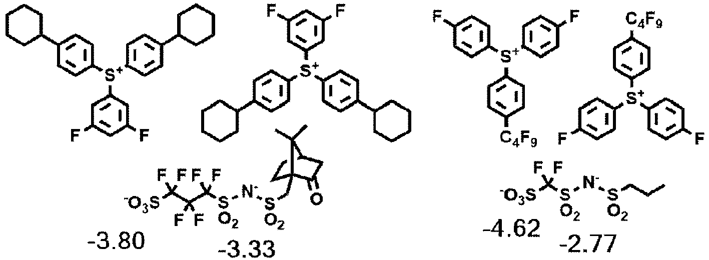

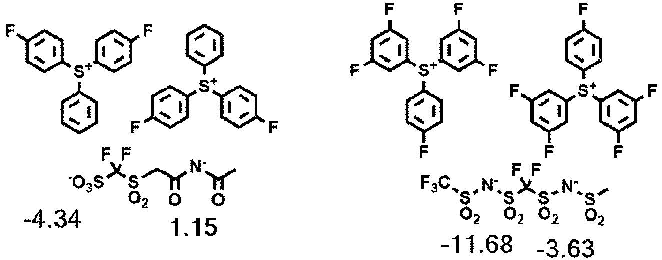

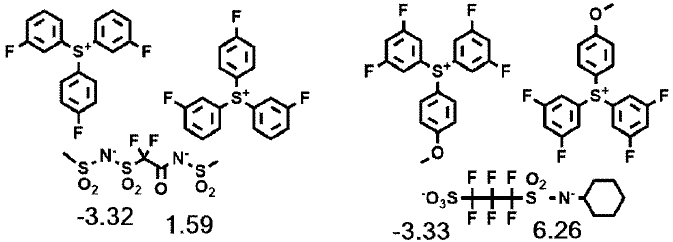

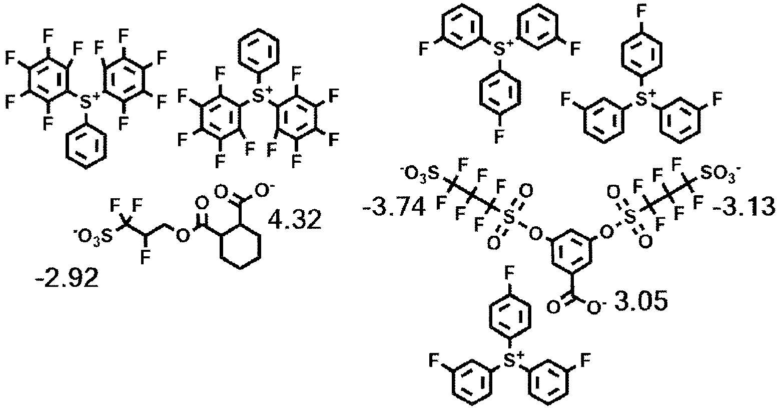

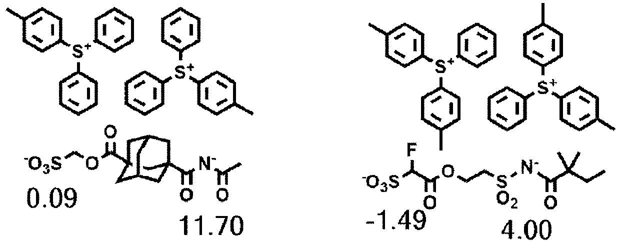

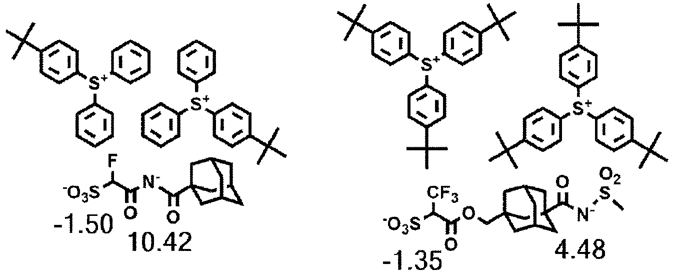

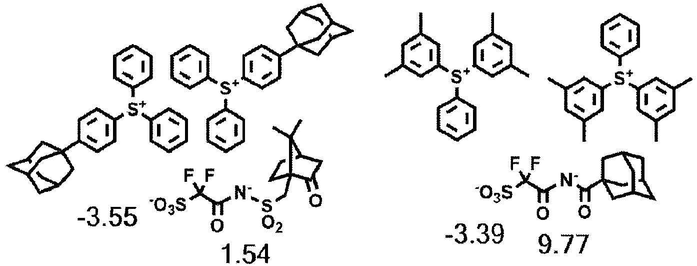

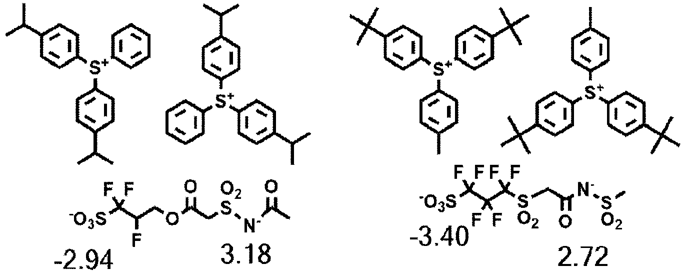

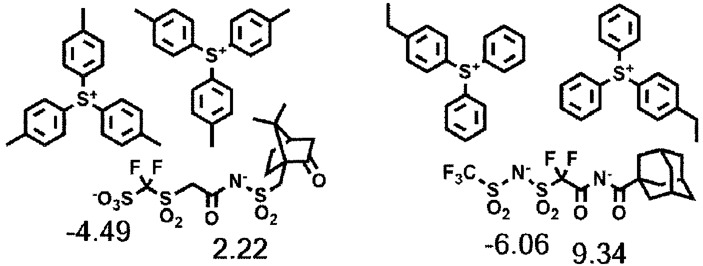

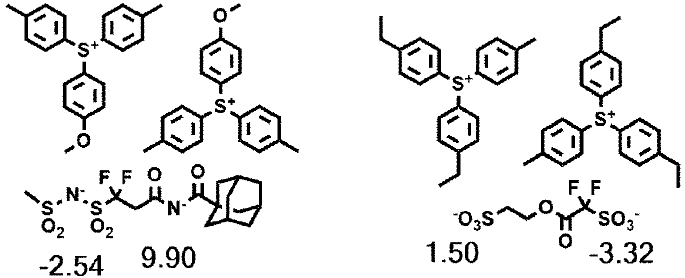

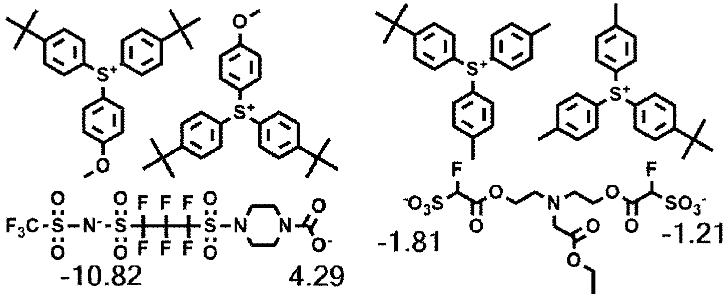

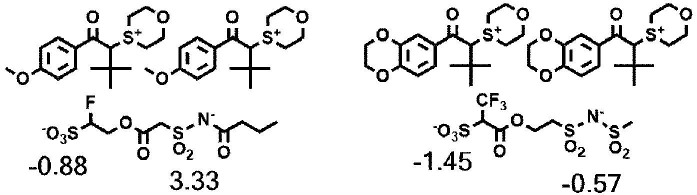

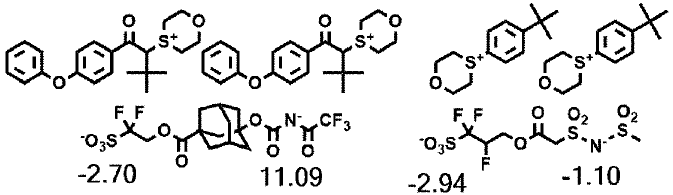

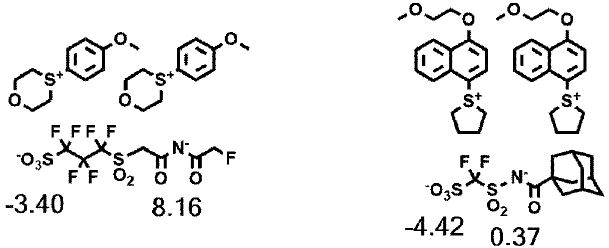

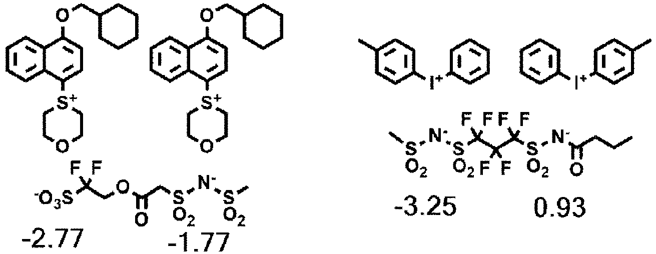

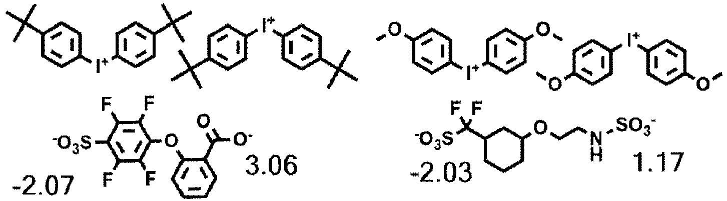

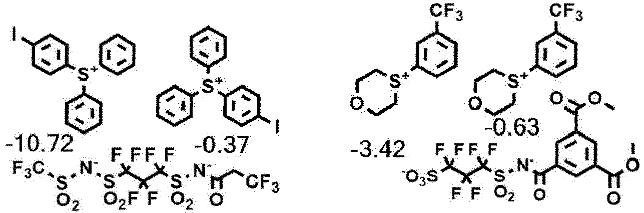

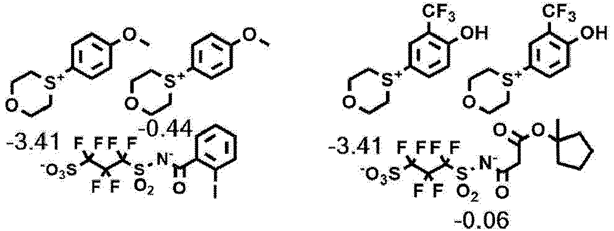

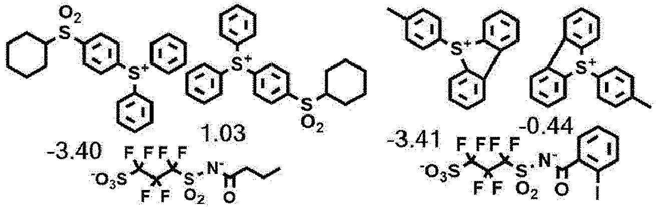

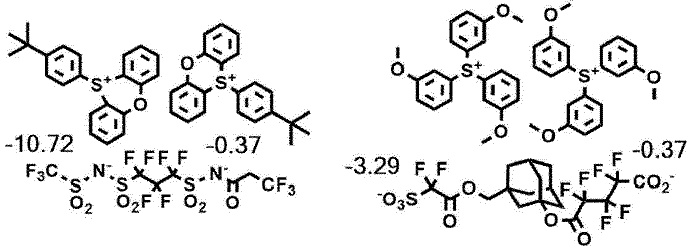

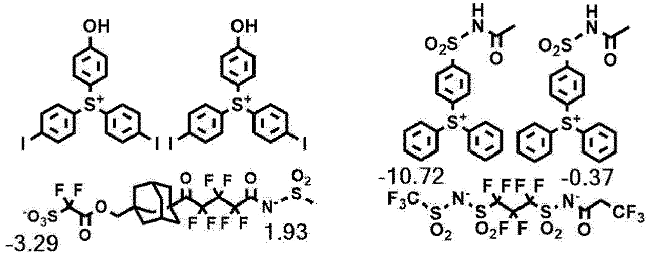

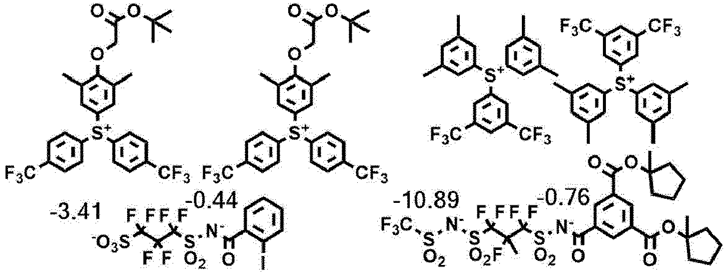

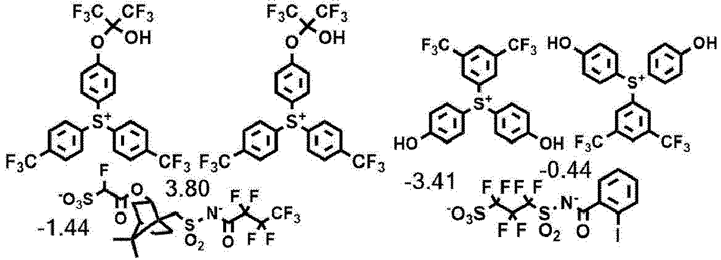

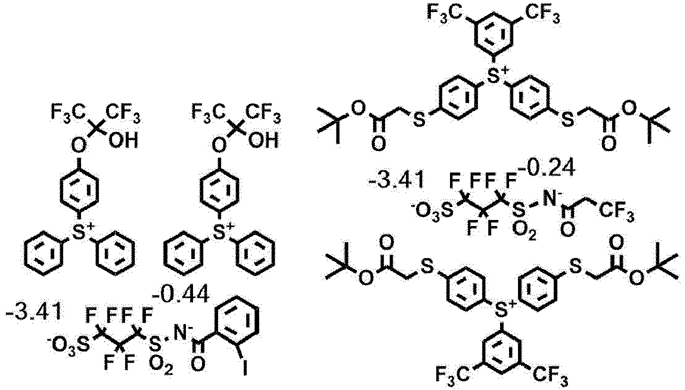

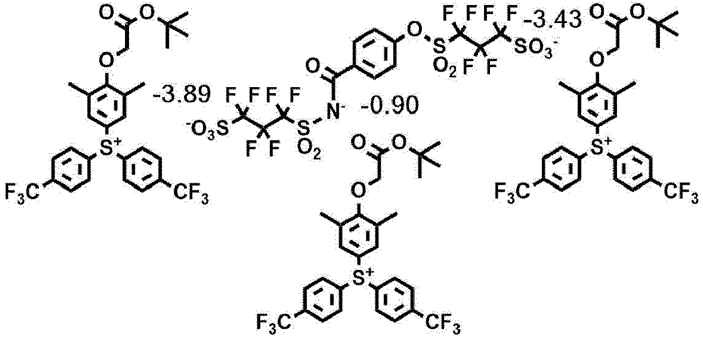

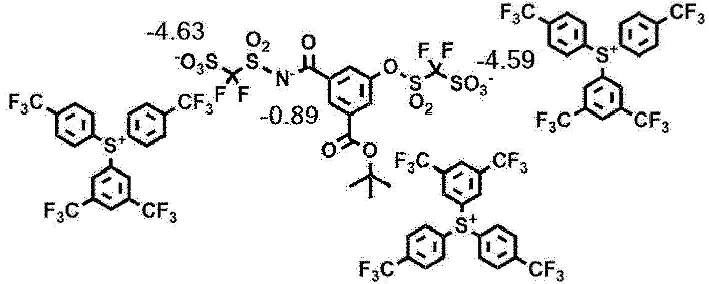

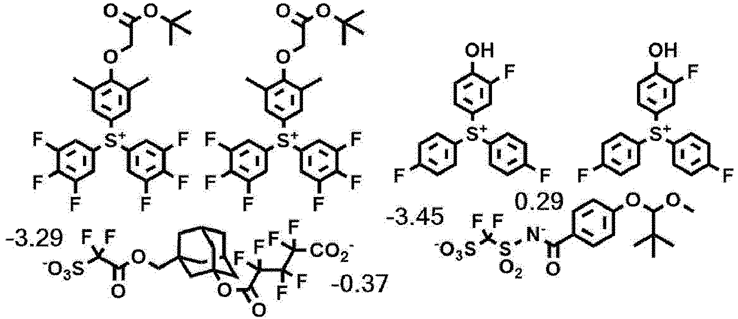

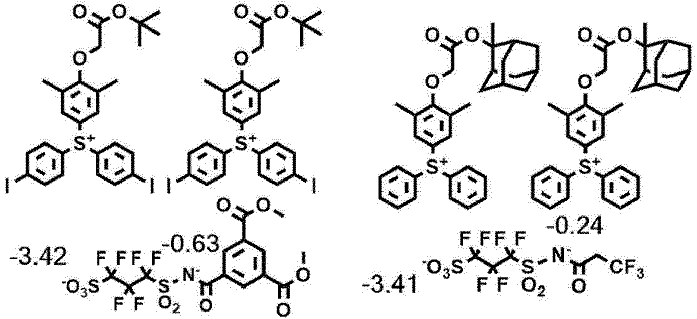

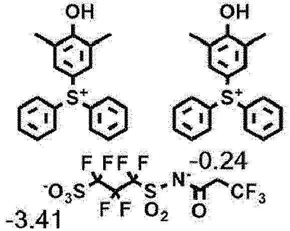

### <Acid-Decomposable Resin (Resin (A))>

The resist composition of the embodiment of the present invention includes a resin (hereinafter also referred to as an "acid-decomposable resin" or a "resin (A)") having a polarity that increases through decomposition by the action of an acid.

That is, in the pattern forming method of an embodiment of the present invention which will be described later, typically, in a case where an alkali developer is adopted as the developer, a positive tone pattern is suitably formed, and in a case where an organic developer is adopted as the developer, a negative tone pattern is suitably formed.

The resin (A) usually includes a repeating unit having a group having a polarity that increases due to decomposition by the action of an acid (hereinafter also referred to as an "acid-decomposable group"), and preferably includes a repeating unit having an acid-decomposable group.

### (Repeating Unit Having Acid-Decomposable Group)

The acid-decomposable group is a group that decomposes by the action of an acid to produce a polar group. The acid-decomposable group preferably has a structure in which the polar group is protected by a leaving group that leaves by the action of an acid. That is, the resin (A) has a repeating unit having a group that decomposes by the action of an acid to produce a polar group. A resin having this repeating unit has an increased polarity by the action of an acid, and thus has an increased solubility in an alkali developer, and a decreased solubility in an organic solvent.

As the polar group, an alkali-soluble group is preferable, and examples thereof include an acidic group such as a carboxyl group, a phenolic hydroxyl group, a fluorinated alcohol group, a sulfonic acid group, a phosphoric acid group, a sulfonamide group, a sulfonylimide group, an (alkylsulfonyl)(alkylcarbonyl)methylene group, an (alkylsulfonyl)(alkylcarbonyl)imide group, a bis(alkylcarbonyl)methylene group, a bis(alkylcarbonyl)imide group, a bis(alkylsulfonyl)methylene group, a bis(alkylsulfonyl)imide group, a tris(alkylcarbonyl)methylene group, and a tris(alkylsulfonyl)methylene group, and an alcoholic hydroxyl group.

Among those, as the polar group, the carboxyl group, the phenolic hydroxyl group, the fluorinated alcohol group (preferably a hexafluoroisopropanol group), or the sulfonic acid group is preferable.

Examples of the leaving group that leaves by the action of an acid include groups represented by Formulae (Y1) to (Y4).

Formula (Y1): -C(Rx₁)(Rx₂)(Rx₃)

Formula (Y2): -C(=O)OC(Rx₁)(Rx₂)(Rx₃)

Formula (Y3): -C(R₃₆)(R₃₇)(OR₃₈)

Formula (Y4): -C(Rn)(H)(Ar)

In Formula (Y1) and Formula (Y2), Rxi to Rx₃ each independently represent an (linear or branched) alkyl group or (monocyclic or polycyclic) cycloalkyl group, an (linear or branched) alkenyl group, or an (monocyclic or polycyclic) aryl group. In a case where possible, these groups preferably have a fluorine atom or a group having a fluorine atom as a substituent.

Furthermore, in a case where all of Rxi to Rx₃ are (linear or branched) alkyl groups, it is preferable that at least two of Rx₁, Rx₂, or Rx₃ are methyl groups.

Above all, it is preferable that Rx₁ to Rx₃ each independently represent a linear or branched alkyl group, and it is more preferable that Rxi to Rx₃ each independently represent the linear alkyl group.

Two of Rxi to Rx₃ may be bonded to each other to form a monocycle or a polycycle.

As the alkyl group of each of Rxi to Rx₃, an alkyl group having 1 to 5 carbon atoms, such as a methyl group, an ethyl group, an n-propyl group, an isopropyl group, an n-butyl group, an isobutyl group, and a t-butyl group, is preferable.

As the cycloalkyl group of each of Rx₁ to Rx₃, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

As the aryl group of each of Rx₁ to Rx₃, an aryl group having 6 to 10 carbon atoms is preferable, and examples thereof include a phenyl group, a naphthyl group, and an anthryl group.

As the alkenyl group of each of Rx₁ to Rx₃, a vinyl group is preferable.

As a ring formed by the bonding of two of Rx₁ to Rx₃, a cycloalkyl group is preferable. As the cycloalkyl group formed by the bonding of two of Rx₁ to Rx₃, a monocyclic cycloalkyl group such as a cyclopentyl group or a cyclohexyl group, or a polycyclic cycloalkyl group such as a norbornyl group, a tetracyclodecanyl group, a tetracyclododecanyl group, or an adamantyl group is preferable, and a monocyclic cycloalkyl group having 5 or 6 carbon atoms is more preferable.

In the cycloalkyl group formed by the bonding of two of Rxi to Rx₃, for example, one of the methylene groups constituting the ring may be substituted with a heteroatom such as an oxygen atom, a group having a heteroatom, such as a carbonyl group, or a vinylidene group. In addition, in the cycloalkyl group, one or more of the ethylene groups constituting the cycloalkane ring may be substituted with a vinylene group.

With regard to the group represented by Formula (Y1) or Formula (Y2), for example, an aspect in which Rx₁ is a methyl group or an ethyl group, and Rx₂ and Rx₃ are bonded to each other to form a cycloalkyl group is also preferable.

In Formula (Y3), R₃₆ to R₃₈ each independently represent a hydrogen atom or a monovalent organic group. R₃₇ and R₃₈ may be bonded to each other to form a ring. Examples of the monovalent organic group include an alkyl group, a cycloalkyl group, an aryl group, an aralkyl group, and an alkenyl group. It is also preferable that R₃₆ is the hydrogen atom.

Furthermore, the alkyl group, the cycloalkyl group, the aryl group, and the aralkyl group may include a heteroatom such as an oxygen atom, and/or a group having a heteroatom, such as a carbonyl group. For example, in the alkyl group, the cycloalkyl group, the aryl group, and the aralkyl group, one or more of the methylene groups may be substituted with a heteroatom such as an oxygen atom and/or a group having a heteroatom, such as a carbonyl group.

In addition, R₃₈ and another substituent contained in the main chain of the repeating unit may be bonded to each other to form a ring. A group formed by the mutual bonding of R₃₈ and another substituent on the main chain of the repeating unit is preferably an alkylene group such as a methylene group.

As Formula (Y3), a group represented by Formula (Y3-1) is preferable.

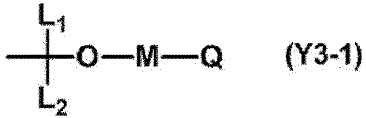

Here, L₁ and L₂ each independently represent a hydrogen atom, an alkyl group, a cycloalkyl group, an aryl group, or a group formed by combination thereof (for example, a group formed by combination of an alkyl group and an aryl group).

M represents a single bond or a divalent linking group.

Q represents an alkyl group which may include a heteroatom, a cycloalkyl group which may include a heteroatom, an aryl group which may include a heteroatom, an amino group, an ammonium group, a mercapto group, a cyano group, an aldehyde group, or a group formed by combination thereof (for example, a group formed by combination of an alkyl group and a cycloalkyl group).

In the alkyl group and the cycloalkyl group, for example, one of the methylene groups may be substituted with a heteroatom such as an oxygen atom or a group having a heteroatom, such as a carbonyl group.

In addition, it is preferable that one of L₁ or L₂ is a hydrogen atom, and the other is an alkyl group, a cycloalkyl group, an aryl group, or a group formed by combination of an alkylene group and an aryl group.

At least two of Q, M, or L₁ may be bonded to each other to form a ring (preferably a 5- or 6-membered ring). Q may be bonded to a part of the acid group protected by the group represented by the formula (Y3-1) to form a ring. Q may be bonded to the main chain of the repeating unit to form a ring.

From the viewpoint of pattern miniaturization, L₂ is preferably a secondary or tertiary alkyl group, and more preferably the tertiary alkyl group. Examples of the secondary alkyl group include an isopropyl group, a cyclohexyl group, and a norbornyl group, and examples of the tertiary alkyl group include a tert-butyl group and an adamantane group. In these aspects, since the glass transition temperature (Tg) and the activation energy are increased, it is possible to suppress fogging in addition to ensuring film hardness.

In Formula (Y4), Ar represents an aromatic ring group. Rn represents an alkyl group, a cycloalkyl group, or an aryl group. Rn and Ar may be bonded to each other to form a non-aromatic ring. Ar is more preferably the aryl group.

From the viewpoint that the acid decomposability of the repeating unit is excellent, in a case where a non-aromatic ring is directly bonded to a polar group (or a residue thereof) in a leaving group that protects the polar group, it is also preferable that a ring member atom adjacent to the ring member atom directly bonded to the polar group (or a residue thereof) in the non-aromatic ring has no halogen atom such as a fluorine atom as a substituent.

The leaving group that leaves by the action of an acid may be a group represented by "-O(Ry₁)(Ry₁)(Ry₃)" in General Formula (B) which will be described later.

In addition, the leaving group that leaves by the action of an acid may be a 2-cyclopentenyl group having a substituent (an alkyl group and the like), such as a 3-methyl-2-cyclopentenyl group, and a cyclohexyl group having a substituent (an alkyl group and the like), such as a 1,1,4,4-tetramethylcyclohexyl group.

As the repeating unit having an acid-decomposable group, a repeating unit represented by Formula (A) is also preferable.

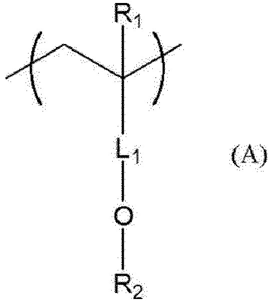

L₁ represents a divalent linking group which may have a fluorine atom or an iodine atom, R₁ represents a hydrogen atom, a fluorine atom, an iodine atom, a fluorine atom, an alkyl group which may have an iodine atom, or an aryl group which may have a fluorine atom or an iodine atom, and R₂ represents a leaving group that leaves by the action of an acid and may have a fluorine atom or an iodine atom. It should be noted that at least one of L₁, R₁, or R₂ has a fluorine atom or an iodine atom.

L₁ represents a divalent linking group which may have a fluorine atom or an iodine atom. Examples of the divalent linking group which may have a fluorine atom or an iodine atom include -CO-, -O-, -S-, -SO-, -SO₂-, a hydrocarbon group which may have a fluorine atom or an iodine atom (for example, an alkylene group, a cycloalkylene group, an alkenylene group, and an arylene group), and a linking group formed by the linking of a plurality of these groups. Among those, as Li, -CO- or -arylene group-alkylene group having a fluorine atom or an iodine atom- is preferable.

As the arylene group, a phenylene group is preferable.

The alkylene group may be linear or branched. The number of carbon atoms of the alkylene group is not particularly limited, but is preferably 1 to 10, and more preferably 1 to 3.

The total number of fluorine atoms and iodine atoms included in the alkylene group having a fluorine atom or an iodine atom is not particularly limited, but is preferably 2 or more, more preferably 2 to 10, and still more preferably 3 to 6.

R₁ represents a hydrogen atom, a fluorine atom, an iodine atom, an alkyl group which may have a fluorine atom or an iodine atom, or an aryl group which may have a fluorine atom or an iodine atom.

The alkyl group may be linear or branched. The number of carbon atoms of the alkyl group is not particularly limited, but is preferably 1 to 10, and more preferably 1 to 3.

The total number of fluorine atoms and iodine atoms included in the alkyl group having a fluorine atom or an iodine atom is not particularly limited, but is preferably 1 or more, more preferably 1 to 5, and still more preferably 1 to 3.

The alkyl group may include a heteroatom such as an oxygen atom, other than a halogen atom.

R₂ represents a leaving group that leaves by the action of an acid and may have a fluorine atom or an iodine atom.

Examples of the leaving group include the above-mentioned groups represented by Formulae (Y1) to (Y4).

As the repeating unit having an acid-decomposable group, a repeating unit having an acid-decomposable group including an unsaturated bond is also preferable.

The repeating unit having an acid-decomposable group including an unsaturated bond is preferably a repeating unit represented by General Formula (B).

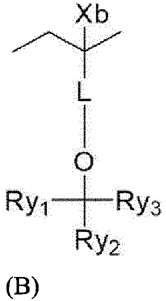

In General Formula (B),
Xb represents a hydrogen atom, a halogen atom, or an alkyl group which may have a substituent.
L represents a single bond, or a divalent linking group which may have a substituent.
Ry₁ to Ry₃ each independently represent a linear or branched alkyl group, a monocyclic or polycyclic cycloalkyl group, an alkenyl group, an alkynyl group, or a monocyclic or polycyclic aryl group. It should be noted that at least one of Ry₁, Ry₂, or Ry₃ represents an alkenyl group, an alkynyl group, a monocyclic or polycyclic cycloalkenyl group, or a monocyclic or polycyclic aryl group.

Two of Ry₁ to Ry₃ may be bonded to each other to form a monocycle or polycycle (a monocyclic or polycyclic cycloalkyl group, a cycloalkenyl group, or the like).

In a case where two of Ry₁ to Ry₃ are bonded to each other to form a cycloalkenyl group and the cycloalkenyl group forms a vinylene group with β carbon and γ carbon with respect to -O- specified in General Formula (B), the remaining one of Ry₁ to Ry₃ may be a hydrogen atom.

Examples of the alkyl group which may have a substituent, represented by Xb, include a methyl group or a group represented by -CH₂-R₁₁. R₁₁ represents a halogen atom (a fluorine atom or the like), a hydroxyl group, or a monovalent organic group, examples thereof include an alkyl group having 5 or less carbon atoms, which may be substituted with a halogen atom, an acyl group having 5 or less carbon atoms, which may be substituted with a halogen atom, and an alkoxy group having 5 or less carbon atoms, which may be substituted with a halogen atom; and an alkyl group having 3 or less carbon atoms is preferable, and a methyl group is more preferable. As Xb, a hydrogen atom, a fluorine atom, a methyl group, a trifluoromethyl group, or a hydroxymethyl group is preferable.

Examples of the divalent linking group of L include an -Rt- group, a -CO- group, a -COO-Rt- group, a -COO-Rt-CO- group, an -Rt-CO- group, and an -O-Rt- group. In the formulae, Rt represents an alkylene group, a cycloalkylene group, or an aromatic ring group.

L is preferably the -Rt- group, the -CO- group, the -COO-Rt-CO- group, or the -Rt-CO- group. Rt may have a substituent such as, for example, a halogen atom, a hydroxyl group, or an alkoxy group, and is preferably an aromatic group.

As the alkyl group of each of Ry₁ to Ry₃, an alkyl group having 1 to 4 carbon atoms, such as a methyl group, an ethyl group, an n-propyl group, an isopropyl group, an n-butyl group, an isobutyl group, and a t-butyl group, is preferable.

As the cycloalkyl group of each of Ry₁ to Ry₃, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

As the aryl group of each of Ry₁ to Ry₃, an aryl group having 6 to 10 carbon atoms is preferable, and examples thereof include a phenyl group, a naphthyl group, and an anthryl group.

As the alkenyl group of each of Ry₁ to Ry₃, a vinyl group is preferable.

As the alkynyl group of each of Ry₁ to Ry₃, an ethynyl group is preferable.

As the cycloalkenyl group of each of Ry₁ to Ry₃, a structure including a double bond in a part of a monocyclic cycloalkyl group such as a cyclopentyl group and a cyclohexyl group is preferable.

As the cycloalkyl group formed by the bonding of two of Ry₁ to Ry₃, a monocyclic cycloalkyl group such as a cyclopentyl group and a cyclohexyl group is preferable, and in addition, a polycyclic cycloalkyl group such as a norbornyl group, a tetracyclodecanyl group, a tetracyclododecanyl group, and an adamantyl group is also preferable. Among those, a monocyclic cycloalkyl group having 5 or 6 carbon atoms is preferable.

In the cycloalkyl group or cycloalkenyl group formed by the bonding of two of Ry₁ to Ry₃, for example, one of the methylene groups constituting the ring may be substituted with a heteroatom such as an oxygen atom, a group having a heteroatom, such as a carbonyl group, an -SO₂- group, and an -SO₃- group, or a vinylidene group, or a combination thereof. In addition, in the cycloalkyl group or cycloalkenyl group, one or more of the ethylene groups constituting the cycloalkane ring or the cycloalkene ring may be substituted with a vinylene group.

In the repeating unit represented by General Formula (B), for example, an aspect in which Ry₁ is a methyl group, an ethyl group, a vinyl group, an allyl group, or an aryl group, and Ry₂ and Rx₃ are bonded to each other to form the above-mentioned cycloalkyl group and cycloalkenyl group is preferable.

In a case where each of the groups has a substituent, examples of the substituent include an alkyl group (having 1 to 4 carbon atoms), a halogen atom, a hydroxyl group, an alkoxy group (having 1 to 4 carbon atoms), a carboxyl group, and an alkoxycarbonyl group (having 2 to 6 carbon atoms). The substituent preferably has 8 or less carbon atoms.

As the repeating unit represented by General Formula (B), an acid-decomposable (meth)acrylic acid tertiary ester-based repeating unit (a repeating unit in which Xb represents a hydrogen atom or a methyl group, and L represents a -CO- group), an acid-decomposable hydroxystyrene tertiary alkyl ether-based repeating unit (a repeating unit in which Xb represents a hydrogen atom or a methyl group and L represents a phenyl group), or an acid-decomposable styrenecarboxylic acid tertiary ester-based repeating unit (a repeating unit in which Xb represents a hydrogen atom or a methyl group, and L represents a -Rt-CO- group (Rt is an aromatic group)) is preferable.

The content of the repeating unit having an acid-decomposable group including an unsaturated bond is preferably 15% by mole or more, more preferably 20% by mole or more, and still more preferably 30% by mole or more with respect to all repeating units in the resin (A). In addition, an upper limit value thereof is preferably 80% by mole or less, more preferably 70% by mole or less, and particularly preferably 60% by mole or less.

Specific examples of the repeating unit having an acid-decomposable group including an unsaturated bond are shown below, but the present invention is not limited thereto. Furthermore, in the formula, Xb and L₁ each represent any of the substituents or linking groups described above, Ar represents an aromatic group, R represents a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR‴ or -COOR‴: R‴ is an alkyl group having 1 to 20 carbon atoms or a fluorinated alkyl group) or a substituent such as a carboxyl group, R' represents a linear or branched alkyl group, a monocyclic or polycyclic cycloalkyl group, an alkenyl group, an alkynyl group, a monocyclic group, or a polycyclic aryl group, Q represents a heteroatom such as an oxygen atom, a group having a heteroatom, such as a carbonyl group, an -SO₂- group, and an -SO₃- group, or a vinylidene group, or a combination thereof, 1, n, and m represents an integer of 0 or more.

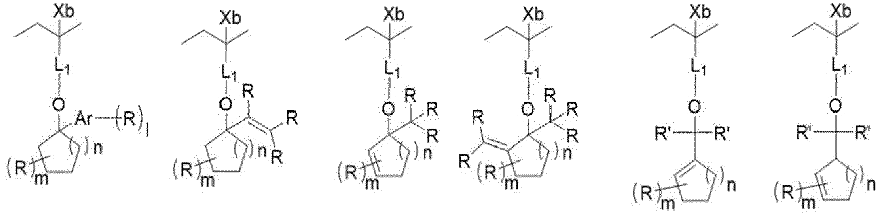

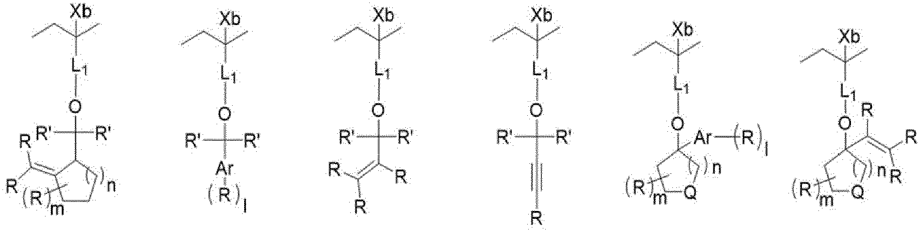

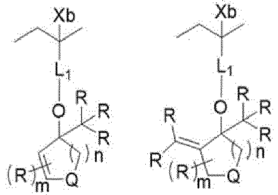

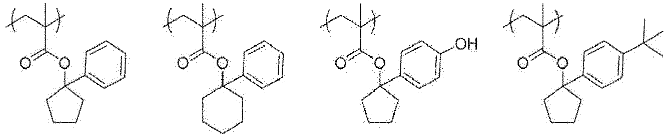

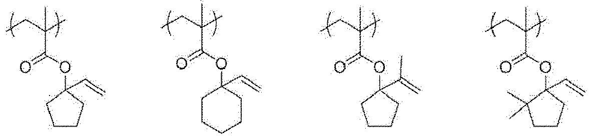

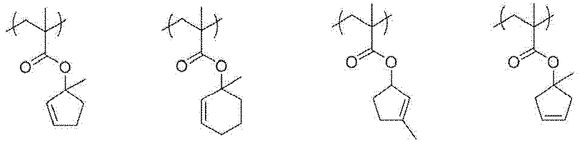

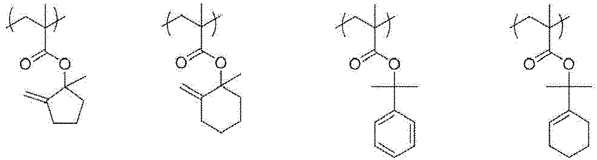

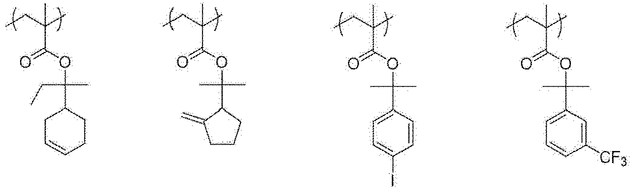

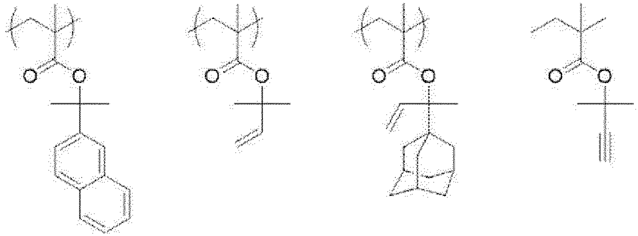

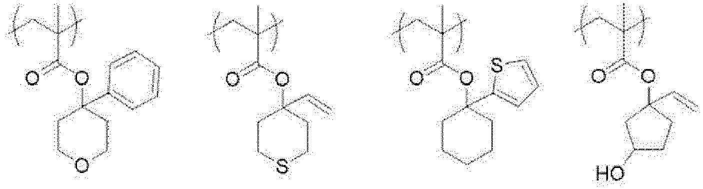

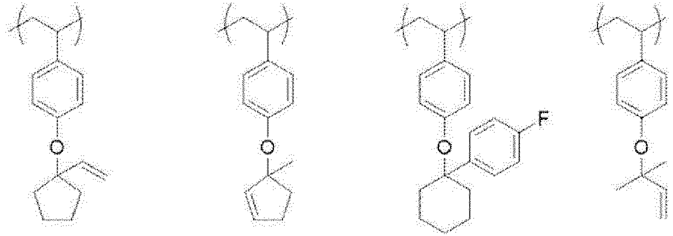

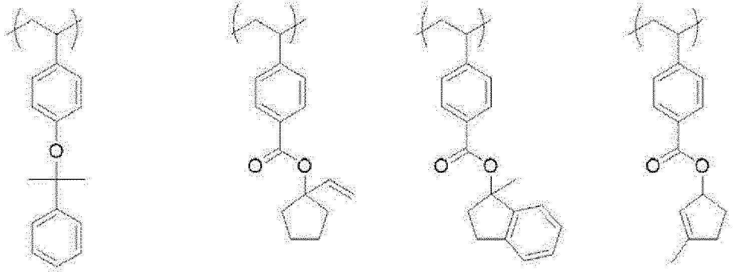

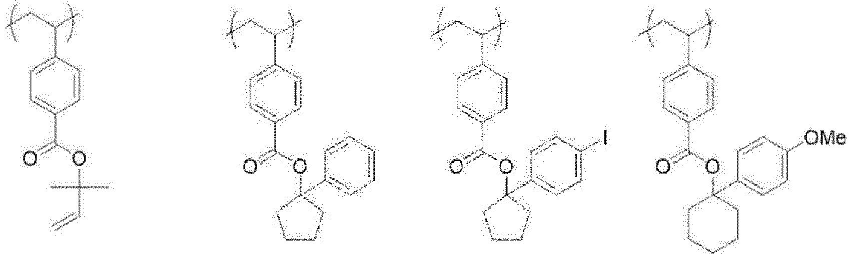

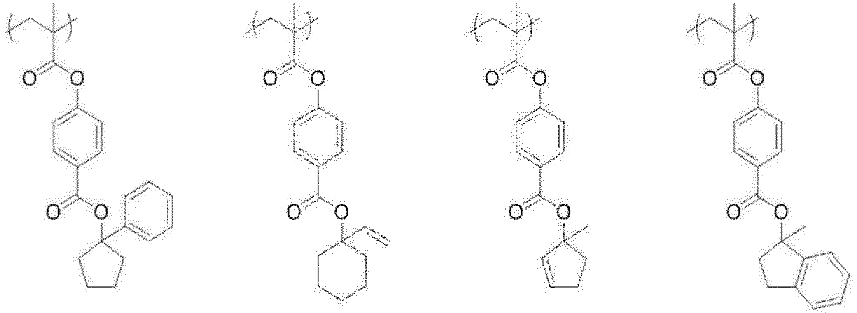

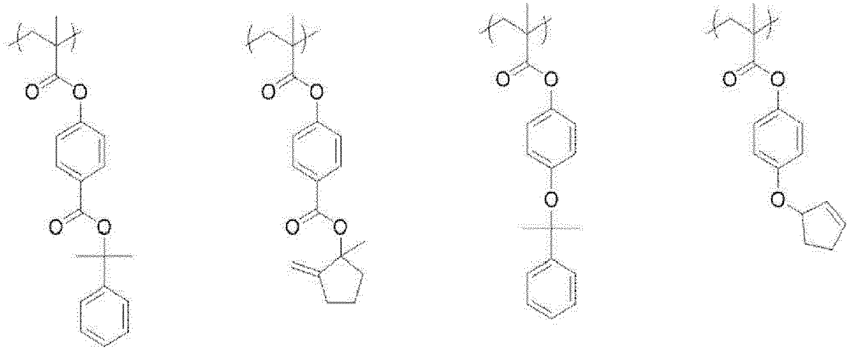

As the repeating unit having an acid-decomposable group, for example, the repeating units described in paragraphs [0068] to [0108] of JP2014-010245A, the contents of which are incorporated herein by reference, can also be used.

Specific examples of the repeating unit having an acid-decomposable group are shown below, but the present invention is not limited thereto. Furthermore, in the formulae, Xai represents H, CH₃, CF₃, or CH₂OH, and Rxa and Rxb each represent a linear or branched alkyl group having 1 to 5 carbon atoms.

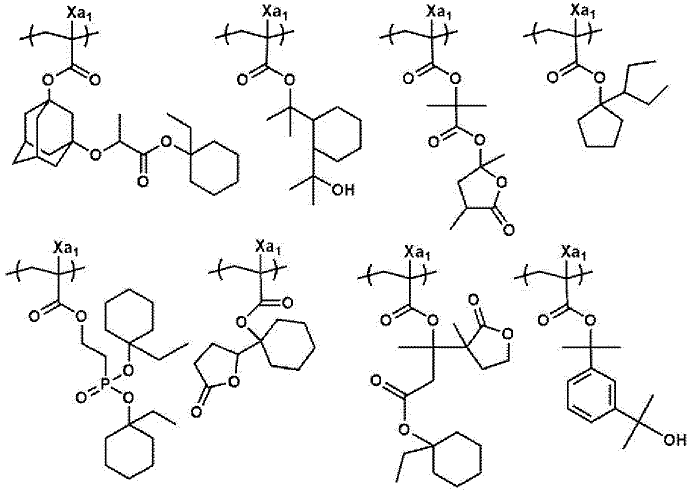

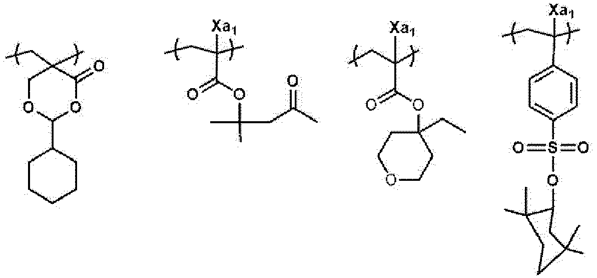

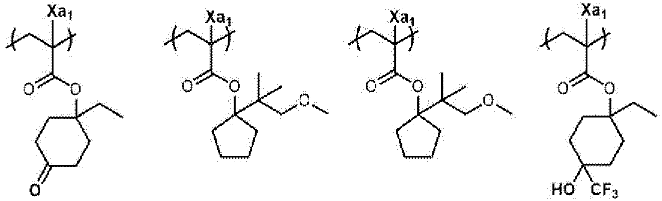

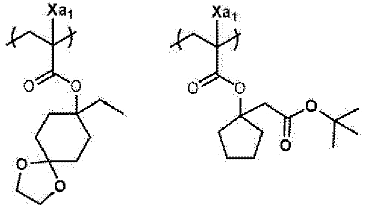

A content of the repeating unit having an acid-decomposable group is preferably 15% to 95% by mole, more preferably 20% to 90% by mole, and still more preferably 20% to 85% by mole with respect to all repeating units in the resin (A).

### (Repeating Unit Having Lactone Group, Sultone Group, or Carbonate Group)

The resin (A) may have a repeating unit having at least one selected from the group consisting of a lactone group, a sultone group, and a carbonate group (hereinafter also collectively referred to as a "repeating unit having a lactone group, a sultone group, or a carbonate group").

It is also preferable that the repeating unit having a lactone group, a sultone group, or a carbonate group has no acid group such as a hexafluoropropanol group.

The lactone group or the sultone group may have a lactone structure or a sultone structure. The lactone structure or the sultone structure is preferably a 5- to 7-membered ring lactone structure or a 5- to 7-membered ring sultone structure. Among those, the structure is more preferably a 5- to 7-membered ring lactone structure with which another ring structure is fused so as to form a bicyclo structure or a spiro structure or a 5- to 7-membered ring sultone structure with which another ring structure is fused so as to form a bicyclo structure or a spiro structure.

The resin (A) preferably has a repeating unit having a lactone group or a sultone group, formed by extracting one or more hydrogen atoms from a ring member atom of a lactone structure represented by any of General Formulae (LC1-1) to (LC1-21) or a sultone structure represented by any of General Formulae (SL1-1) to (SL1-3).

In addition, the lactone group or the sultone group may be bonded directly to the main chain. For example, a ring member atom of the lactone group or the sultone group may constitute the main chain of the resin (A).

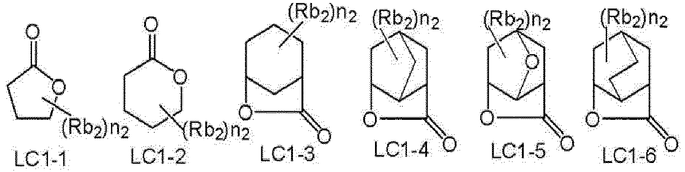

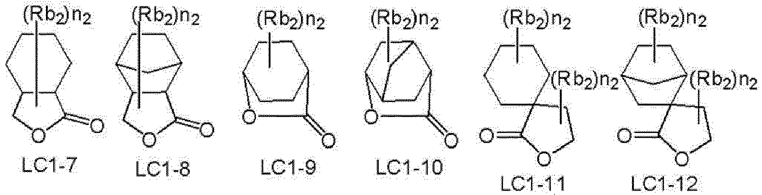

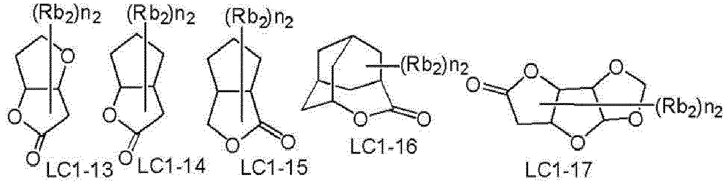

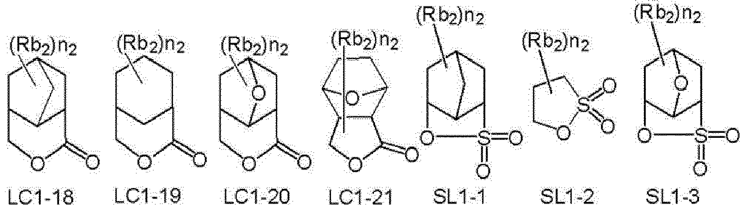

The moiety of the lactone structure or the sultone structure may have a substituent (Rb₂). Preferred examples of the substituent (Rb₂) include an alkyl group having 1 to 8 carbon atoms, a cycloalkyl group having 4 to 7 carbon atoms, an alkoxy group having 1 to 8 carbon atoms, an alkoxycarbonyl group having 1 to 8 carbon atoms, a carboxyl group, a halogen atom, a hydroxyl group, a cyano group, and an acid-decomposable group. n2 represents an integer of 0 to 4. In a case where n2 is 2 or more, Rb₂'s which are present in a plural number may be different from each other, and Rb₂'s which are present in a plural number may be bonded to each other to form a ring.

Examples of the repeating unit having a group having the lactone structure represented by any of General Formulae (LC1-1) to (LC1-21) or the sultone structure represented by any of General Formulae (SL1-1) to (SL1-3) include a repeating unit represented by General Formula (AI).

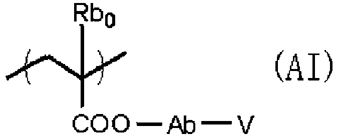

In General Formula (AI), Rbo represents a hydrogen atom, a halogen atom, or an alkyl group having 1 to 4 carbon atoms.

Preferred examples of the substituent which may be contained in the alkyl group of Rbo include a hydroxyl group and a halogen atom.

Examples of the halogen atom of Rbo include a fluorine atom, a chlorine atom, a bromine atom, and an iodine atom. Rbo is preferably the hydrogen atom or a methyl group.

Ab represents a single bond, an alkylene group, a divalent linking group having a monocyclic or polycyclic alicyclic hydrocarbon structure, an ether group, an ester group, a carbonyl group, a carboxyl group, or a divalent group formed by combination thereof. Among those, the single bond or a linking group represented by -Ab₁-CO₂- is preferable. Abi is a linear or branched alkylene group, or a monocyclic or polycyclic cycloalkylene group, and is preferably a methylene group, an ethylene group, a cyclohexylene group, an adamantylene group, or a norbornylene group.

V represents a group formed by extracting one hydrogen atom from a ring member atom of the lactone structure represented by any of General Formulae (LC1-1) to (LC1-21) or a group formed by extracting one hydrogen atom from a ring member atom of the sultone structure represented by any of General Formulae (SL1-1) to (SL1-3).

In a case where an optical isomer is present in the repeating unit having a lactone group or a sultone group, any of optical isomers may be used. In addition, one kind of optical isomers may be used alone or plural kinds of optical isomers may be mixed and used. In a case where one kind of optical isomers is mainly used, an optical purity (ee) thereof is preferably 90 or more, and more preferably 95 or more.

As the carbonate group, a cyclic carbonic acid ester group is preferable.

As the repeating unit having a cyclic carbonic acid ester group, a repeating unit represented by General Formula (A-1) is preferable.

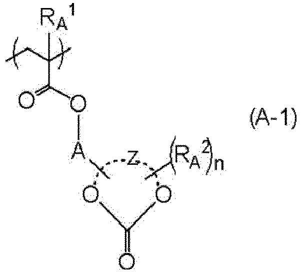

In General Formula (A-1), R_{A}¹ represents a hydrogen atom, a halogen atom, or a monovalent organic group (preferably a methyl group).
n represents an integer of 0 or more.
R_{A}² represents a substituent. In a case where n is 2 or more, R_{A}² which are present in a plural number may be the same as or different from each other.
A represents a single bond or a divalent linking group. As the divalent linking group, an alkylene group, a divalent linking group having a monocyclic or polycyclic alicyclic hydrocarbon structure, an ether group, an ester group, a carbonyl group, a carboxyl group, or a divalent group formed by combination thereof is preferable.
Z represents an atomic group that forms a monocycle or polycycle with a group represented by -O-CO-O- in the formula.

As the repeating unit having a lactone group or a sultone group, for example, the repeating units described in paragraphs [0109] to [0120] of JP2014-010245A, the contents of which are incorporated herein by reference, can also be used.

As the repeating unit having a carbonate group, for example, the repeating units described in paragraphs [0121] to [0132] of JP2014-010245A, the contents of which are incorporated herein by reference, can also be used.

A content of the repeating unit having a lactone group, a sultone group, or a carbonate group is preferably 1% to 70% by mole, more preferably 5% to 65% by mole, and still more preferably 5% to 60% by mole with respect to all repeating units in the resin (A).

### (Repeating Unit Having Acid Group)

The resin (A) may have a repeating unit having an acid group.

As the acid group, an acid group having a pKa of 13 or less is preferable.

As the acid group, for example, a carboxyl group, a phenolic hydroxyl group, a fluorinated alcohol group (preferably a hexafluoroisopropanol group), a sulfonic acid group, a sulfonamide group, or an isopropanol group is preferable.

In addition, in the hexafluoroisopropanol group, one or more (preferably one or two) fluorine atoms may be substituted with a group (an alkoxycarbonyl group and the like) other than a fluorine atom. -C(CF₃)(OH)-CF₂- formed as above is also preferable as the acid group. In addition, one or more fluorine atoms may be substituted with a group other than a fluorine atom to form a ring including -C(CF₃)(OH)-CF₂-.

The repeating unit having an acid group is preferably a repeating unit different from a repeating unit having the structure in which a polar group is protected by the leaving group that leaves by the action of an acid as described above, and a repeating unit having a lactone group, a sultone group, or a carbonate group which will be described later.

The repeating unit having an acid group may have a fluorine atom or an iodine atom.

As the repeating unit having an acid group, a repeating unit represented by Formula (B) is preferable.

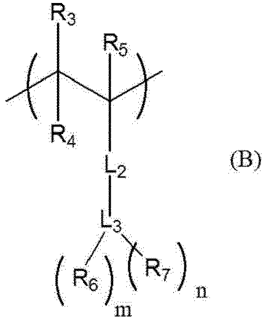

R₃ represents a hydrogen atom, or a monovalent substituent which may have a fluorine atom or an iodine atom.

The monovalent substituent which may have a fluorine atom or an iodine atom is preferably a group represented by -L₄-R₈. L₄ represents a single bond or an ester group. R₈ is an alkyl group which may have a fluorine atom or an iodine atom, a cycloalkyl group which may have a fluorine atom or an iodine atom, an aryl group which may have a fluorine atom or an iodine atom, or a group formed by combination thereof.
R₄ and R₅ each independently represent a hydrogen atom, a fluorine atom, an iodine atom, or an alkyl group which may have a fluorine atom or an iodine atom.
L₂ represents a single bond or an ester group.
L₃ represents an (n + m + l)-valent aromatic hydrocarbon ring group or an (n + m + l)-valent alicyclic hydrocarbon ring group. Examples of the aromatic hydrocarbon ring group include a benzene ring group and a naphthalene ring group. The alicyclic hydrocarbon ring group may be either a monocycle or a polycycle, and examples thereof include a cycloalkyl ring group.
R₆ represents a hydroxyl group or a fluorinated alcohol group (preferably a hexafluoroisopropanol group). Furthermore, in a case where R₆ is a hydroxyl group, L₃ is preferably the (n + m + l)-valent aromatic hydrocarbon ring group.
R₇ represents a halogen atom. Examples of the halogen atom include a fluorine atom, a chlorine atom, a bromine atom, or an iodine atom.
m represents an integer of 1 or more. m is preferably an integer of 1 to 3 and more preferably an integer of 1 or 2.
n represents 0 or an integer of 1 or more. n is preferably an integer of 0 to 4.

Furthermore, (n + m + 1) is preferably an integer of 1 to 5.

The repeating unit having an acid group may be a repeating unit consisting of (meth)acrylic acid.

As the repeating unit having an acid group, for example, the repeating units described in paragraphs [0050] to [0075] of JP2014-010245A, the contents of which are incorporated herein by reference, can also be used.

The repeating unit having an acid group is exemplified below.

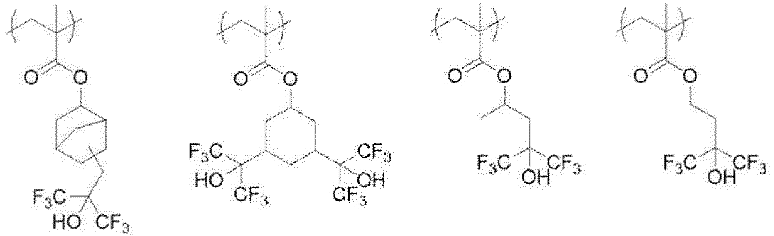

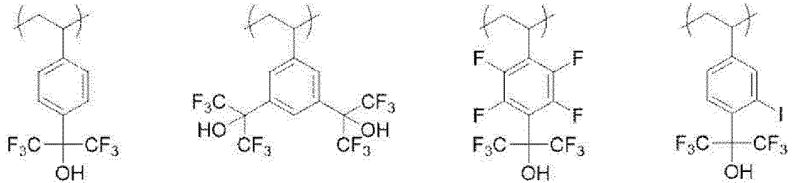

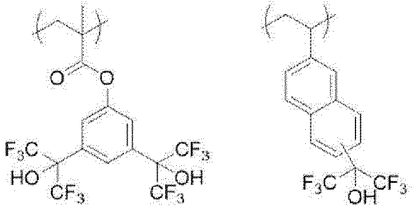

A content of the repeating unit having an acid group is preferably 10% to 70% by mole, more preferably 15% to 65% by mole, and still more preferably 20% to 60% by mole with respect to all repeating units in the resin (A).

### (Repeating Unit Having Photoacid Generating Group)

The resin (A) may have, as a repeating unit other than those above, a repeating unit having a group that generates an acid upon irradiation with actinic rays or radiation (hereinafter also referred to as a "photoacid generating group").

In this case, it can be considered that the repeating unit having this photoacid generating group corresponds to the photoacid generator.

Examples of such the repeating unit include a repeating unit represented by General Formula (4).

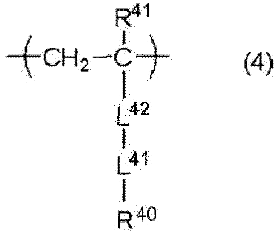

R⁴¹ represents a hydrogen atom or a methyl group. L⁴¹ represents a single bond or a divalent linking group. L⁴² represents a divalent linking group. R⁴⁰ represents a structural moiety that decomposes upon irradiation with actinic rays or radiation to generate an acid in a side chain.

In addition, examples of the repeating unit represented by General Formula (4) include the repeating units described in paragraphs [0094] to [0105] of JP2014-041327A.

A content of the repeating unit having a photoacid generating group is preferably 1% to 40% by mole, more preferably 5% to 35% by mole, and still more preferably 5% to 30% by mole with respect to all repeating units in the resin (A).

### (Repeating Unit Having Hydroxyl Group or Cyano Group)

The resin (A) may have a repeating unit having a hydroxyl group or a cyano group. As a result, the adhesiveness to a substrate and the affinity for a developer are improved.

The repeating unit having a hydroxyl group or a cyano group is preferably a repeating unit having an alicyclic hydrocarbon structure substituted with a hydroxyl group or a cyano group.

The repeating unit having a hydroxyl group or a cyano group preferably has no acid-decomposable group.

The hydroxyl group in the repeating unit having a hydroxyl group or a cyano group is preferably not a hydroxyl group constituting an acid group.

Examples of the repeating unit having a hydroxyl group or a cyano group include repeating units represented by General Formulae (AIIa) to (AIId).

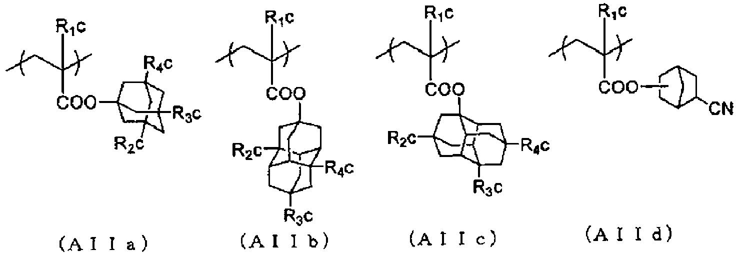

### In General Formulae (AIIa) to (AIId),

R_{1c} represents a hydrogen atom, a methyl group, a trifluoromethyl group, or a hydroxymethyl group.

R_{2c} to R_{4c} each independently represent a hydrogen atom or a hydroxyl group. It should be noted that at least one of R_{2c}, ..., or R_{4c} represents the hydroxyl group. It is preferable that one or two of R_{2c} to R_{4c} are hydroxyl groups and the remaining groups are hydrogen atoms. It is more preferable that two of R_{2c} to R_{4c} are hydroxyl groups and the remaining groups are hydrogen atoms.

As the repeating unit having a hydroxyl group or a cyano group, for example, the repeating units described in paragraphs [0133] to [0142] of JP2014-010245A, the contents of which are incorporated herein by reference, can also be used.

A content of the repeating unit having a hydroxyl group is preferably 5% to 60% by mole, more preferably 10% to 55% by mole, and still more preferably 15% to 55% by mole with respect to all repeating units in the resin (A).

### (Repeating Unit Having Fluorine Atom or Iodine Atom)

The resin (A) may have a repeating unit having a fluorine atom or an iodine atom, in addition to "(Repeating Unit Having Acid-Decomposable Group)" and "(Repeating Unit Having Acid Group)", each mentioned above. In addition, (Repeating Unit Having Fluorine Atom or Iodine Atom) as mentioned herein is preferably different from such another repeating unit as mentioned above.

As the repeating unit having a fluorine atom or an iodine atom, a repeating unit represented by General Formula (C) is preferable. 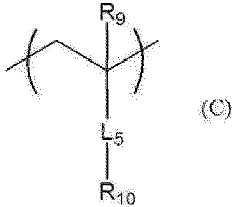
L₅ represents a single bond or an ester group.
R₉ represents a hydrogen atom, or an alkyl group which may have a fluorine atom or an iodine atom.
R₁₀ represents a hydrogen atom, an alkyl group which may have a fluorine atom or an iodine atom, a cycloalkyl group which may have a fluorine atom or an iodine atom, an aryl group which may have a fluorine atom or an iodine atom, or a group formed by combination thereof.

The content of the repeating unit having a fluorine atom or an iodine atom is preferably 0% by mole or more, more preferably 5% by mole or more, and still more preferably 10% by mole or more with respect to all repeating units in the resin (A). In addition, an upper limit value thereof is preferably 50% by mole or less, more preferably 45% by mole or less, and still more preferably 40% by mole or less.

### (Repeating Unit for Reducing Motility of Main Chain)

The resin (A) preferably has a high glass transition temperature (Tg) from the viewpoint that excessive diffusion of an acid generated or pattern collapse during development can be suppressed. Tg is preferably higher than 90°C, more preferably higher than 100°C, still more preferably higher than 110°C, and particularly preferably higher than 125°C. In addition, since an excessive increase in Tg causes a decrease in the dissolution rate in a developer, Tg is preferably 400°C or lower, and more preferably 350°C or lower.

It is preferable to reduce the motility of the main chain of the resin (A) in order to increase the Tg of the resin (A). Examples of a method for reducing the motility of the main chain of the resin (A) include the following (a) to (e) methods.
(a) Introduction of a bulky substituent into the main chain
(b) Introduction of a plurality of substituents into the main chain
(c) Introduction of a substituent that induces an interaction between the resins (A) near the main chain
(d) Formation of the main chain in a cyclic structure
(e) Linking of a cyclic structure to the main chain

As an example of a specific unit for accomplishing (a) above, a method of introducing a repeating unit represented by Formula (A) into the resin (A) may be mentioned.

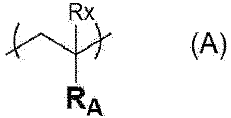

In Formula (A), R_{A} represents a group having a polycyclic structure. Rₓ represents a hydrogen atom, a methyl group, or an ethyl group. The group having a polycyclic structure is a group having a plurality of ring structures, and the plurality of ring structures may or may not be fused.

Specific examples of the repeating unit represented by Formula (A) include the following repeating units.

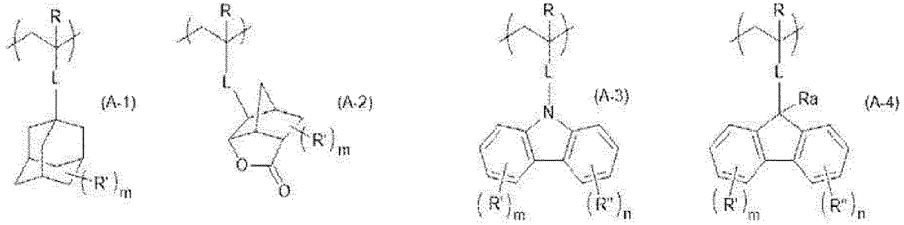

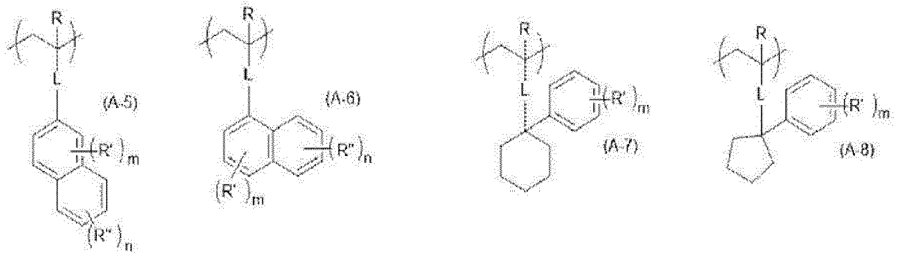

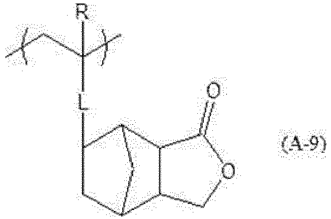

In the formulae, R represents a hydrogen atom, a methyl group, or an ethyl group.

Ra represents a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR‴ or -COOR‴: R‴ is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by Ra may be substituted with a fluorine atom or an iodine atom.

Moreover, R' and R" each independently represent an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR‴ or -COOR‴: R‴ is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by each of R' and R" may be substituted with a fluorine atom or an iodine atom.

L represents a single bond or a divalent linking group. Examples of the divalent linking group include -COO-, -CO-, -O-, -S-, -SO-, -SO₂-, -NH-, -CO-NH-, -CO-NH-CO-, an alkylene group, a cycloalkylene group, an alkenylene group, and a linking group in which a plurality of these groups are linked.

m and n each independently represent an integer of 0 or more. An upper limit of each of m and n is not particularly limited, but is 2 or less in many cases, and 1 or less in more cases.

A content of the repeating unit represented by Formula (A) is preferably 5% to 60%by mole, and more preferably 5% to 55% by mole with respect to all repeating units in the resin (A).

As an example of a specific unit for accomplishing (b) above, a method of introducing a repeating unit represented by Formula (B) into the resin (A) may be mentioned.

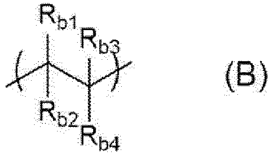

In Formula (B), R_{b1} to R_{b4} each independently represent a hydrogen atom or an organic group, and at least two or more of R_{b1}, ..., or R_{b4} represent an organic group.

Furthermore, in a case where at least one of the organic groups is a group in which a ring structure is directly linked to the main chain in the repeating unit, the types of the other organic groups are not particularly limited.

In addition, in a case where none of the organic groups is a group in which a ring structure is directly linked to the main chain in the repeating unit, at least two or more of the organic groups are substituents having three or more constituent atoms excluding hydrogen atoms.

Specific examples of the repeating unit represented by Formula (B) include the following repeating units.

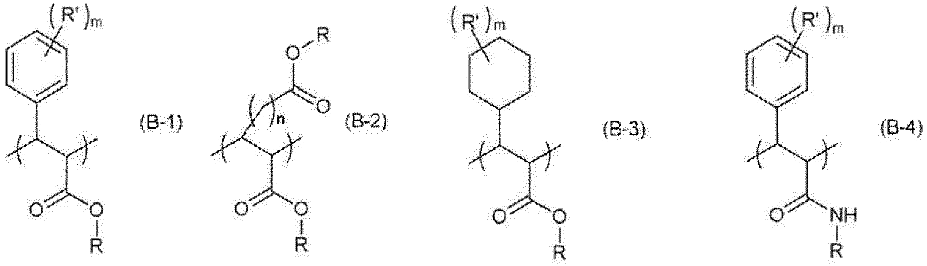

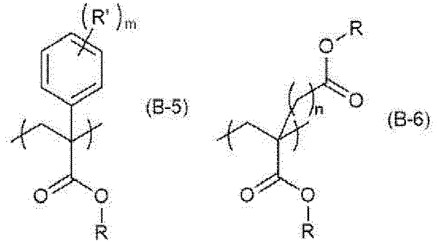

In the formula, R's each independently represent a hydrogen atom or an organic group. Examples of the organic group include an organic group such as an alkyl group, a cycloalkyl group, an aryl group, an aralkyl group, and an alkenyl group, each of which may have a substituent. R may be the above-mentioned leaving group.

R"s each independently represent an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by R' is preferably substituted with a fluorine atom or an iodine atom.

m represents an integer of 0 or more. An upper limit of m is not particularly limited, but is 2 or less in many cases, and 1 or less in more cases.

n represents an integer of 0 or more. An upper limit of n is not particularly limited, but is 10 or less in many cases, and 4 or less in more cases.

A content of the repeating unit represented by Formula (B) is preferably 5% to 60% by mole, and more preferably 10% to 55% by mole with respect to all repeating units in the resin (A).

As an example of a specific unit for accomplishing (c) above, a method of introducing a repeating unit represented by Formula (C) into the resin (A) may be mentioned.

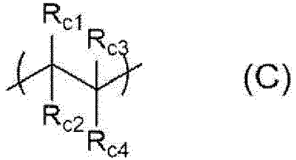

In Formula (C), R_{c1} to R_{c4} each independently represent a hydrogen atom or an organic group, and at least one of R_{c1}, ..., or R_{c4} is a group having a hydrogen-bonding hydrogen atom with a number of atoms of 3 or less from the main chain carbon. Among those, it is preferable that the group has hydrogen-bonding hydrogen atoms with a number of atoms of 2 or less (on a side closer to the vicinity of the main chain) to induce an interaction between the main chains of the resin (A).

Specific examples of the repeating unit represented by Formula (C) include the following repeating units.

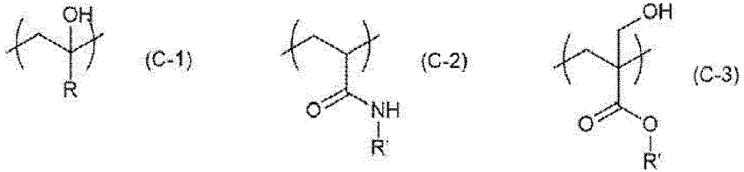

In the formula, R represents an organic group. Examples of the organic group include an alkyl group, a cycloalkyl group, an aryl group, an aralkyl group, an alkenyl group, and an ester group (-OCOR or -COOR: R represents an alkyl group or fluorinated alkyl group having 1 to 20 carbon atoms), each of which may have a substituent.

R' represents a hydrogen atom or an organic group. Examples of the organic group include an organic group such as an alkyl group, a cycloalkyl group, an aryl group, an aralkyl group, and an alkenyl group. In addition, it also preferable that a hydrogen atom in the organic group is substituted with a fluorine atom or an iodine atom.

### • Repeating Unit Represented by Formula (D)

As an example of a specific unit for accomplishing (D) above, a method of introducing a repeating unit represented by Formula (D) into the resin (A) may be mentioned.

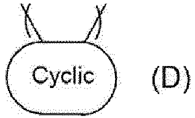

In Formula (D), "Cyclic" is a group that forms a main chain with a cyclic structure. The number of the ring-constituting atoms is not particularly limited.

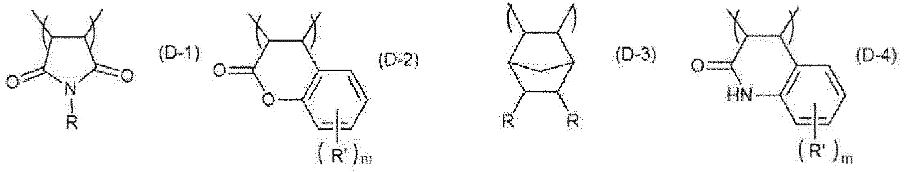

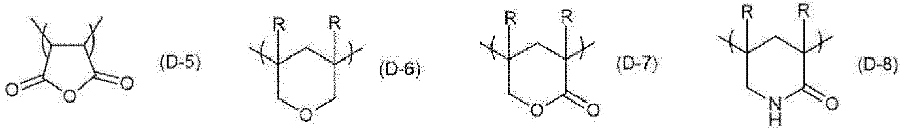

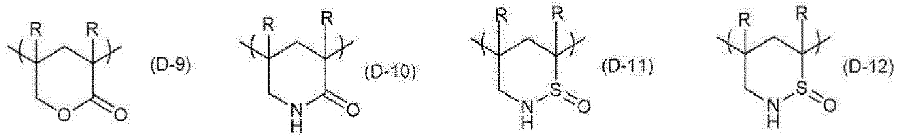

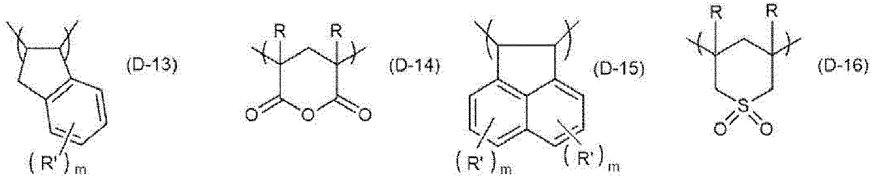

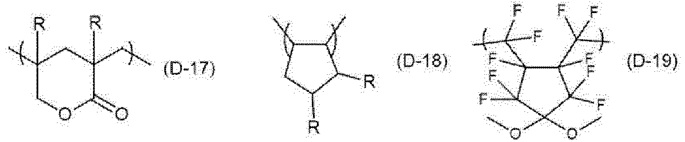

In the formula, R's each independently represent a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an alkylsulfonyl group, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, the alkenyl group, and the alkylsulfonyl group may each have a substituent. In addition, the hydrogen atom bonded to the carbon atom in the group represented by R is preferably substituted with a fluorine atom or an iodine atom.

In the formula, R"s each independently represent an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by R' may be substituted with a fluorine atom or an iodine atom.

m represents an integer of 0 or more. An upper limit of m is not particularly limited, but is 2 or less in many cases, and 1 or less in more cases.

A content of the repeating unit represented by Formula (D) is preferably 5% to 60% by mole, and more preferably 10% to 55% by mole with respect to all repeating units in the resin (A).

### • Repeating Unit Represented by Formula (E)

As an example of a specific unit for accomplishing (e) above, a method of introducing a repeating unit represented by Formula (E) into the resin (A) may be mentioned.

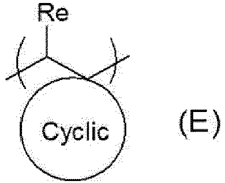

In Formula (E), Re's each independently represent a hydrogen atom or a substituent. Examples of the substituent include an alkyl group, a cycloalkyl group, an aryl group, an aralkyl group, and an alkenyl group, which may have a substituent.

"Cyclic" is a cyclic group including a carbon atom of the main chain. The number of atoms included in the cyclic group is not particularly limited.

Specific examples of the repeating unit represented by Formula (E) include the following repeating units.

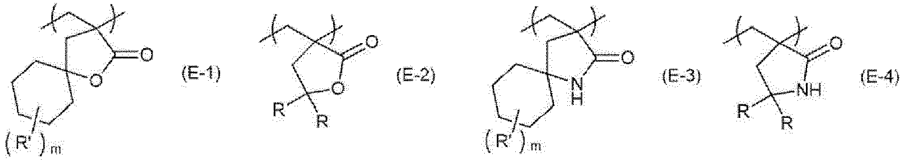

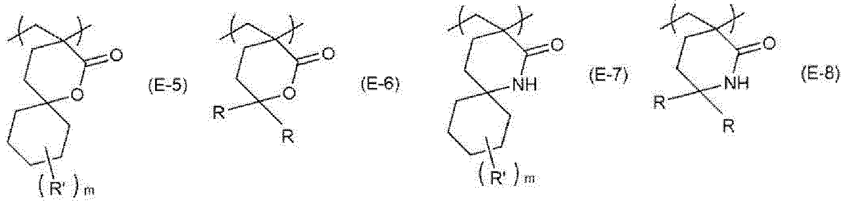

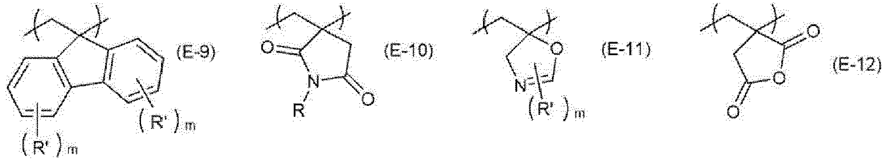

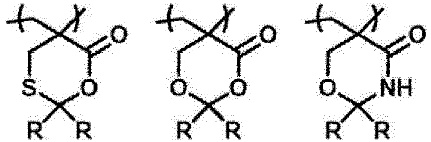

In the formula, R's each independently represent a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, the hydrogen atom bonded to the carbon atom in the group represented by R may be substituted with a fluorine atom or an iodine atom.

R"s each independently represent a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR": R" is an alkyl group or fluorinated alkyl group having 1 to 20 carbon atoms), or a carboxyl group. Furthermore, the alkyl group, the cycloalkyl group, the aryl group, the aralkyl group, and the alkenyl group may each have a substituent. In addition, a hydrogen atom bonded to the carbon atom in the group represented by R' may be substituted with a fluorine atom or an iodine atom.

m represents an integer of 0 or more. An upper limit of m is not particularly limited, but is 2 or less in many cases, and 1 or less in more cases.

In addition, two R's bonded to the same carbon atom may be bonded to each other to form a ring.

In Formula (E-2), Formula (E-4), Formula (E-6), and Formula (E-8), two R's may be combined to form an "=O".

A content of the repeating unit represented by Formula (E) is preferably 5% to 60%by mole, and more preferably 10% to 55% by mole with respect to all repeating units in the resin (A).

A method of increasing the Tg of the resin (A) is not limited to other methods, and examples thereof include the method of introducing a repeating unit described in paragraphs [0105] to [0128] of JP2019-045864A.

The resin (A) may have a variety of repeating structural units, in addition to the repeating structural units described above, for the purpose of adjusting dry etching resistance, suitability for a standard developer, adhesiveness to a substrate, a resist profile, resolving power, heat resistance, sensitivity, and the like.

As the resin (A), all repeating units is also preferably composed of (meth)acrylate-based repeating units (particularly in a case where the composition is used as an actinic ray-sensitive or radiation-sensitive resin composition for ArF). In this case, any of a resin in which all of the repeating units are methacrylate-based repeating units, a resin in which all of the repeating units are acrylate-based repeating units, and a resin in which all of the repeating units are methacrylate-based repeating units and acrylate-based repeating units can be used. It is preferable that the amount of the acrylate-based repeating units is 50% by mole or less of all repeating units.

The resin (A) can be synthesized in accordance with an ordinary method (for example, radical polymerization).

A weight-average molecular weight of the resin (A) as a value expressed in terms of polystyrene by a GPC method is preferably 1,000 to 200,000, more preferably 3,000 to 20,000, and still more preferably 5,000 to 15,000. By setting the weight-average molecular weight of the resin (A) to 1,000 to 200,000, deterioration of heat resistance and dry etching resistance can be further suppressed. In addition, deterioration of developability and deterioration of film forming property due to high viscosity can also be further suppressed.

A dispersity (molecular weight distribution) of the resin (A) is usually 1 to 5, preferably 1 to 3, more preferably 1.2 to 3.0, and still more preferably 1.2 to 2.0. The smaller the dispersity, the more excellent the resolution and the resist shape, and the smoother the side wall of the resist pattern, the more excellent the roughness.

In the composition of the embodiment of the present invention, a content of the resin (A) is preferably 50% to 99.9% by mass, and more preferably 60% to 99.0% by mass with respect to the total solid content of the composition.

Furthermore, the solid content is intended to be components excluding the solvent in the composition, and any of components other than the solvent are regarded as the solid content even in a case where they are liquid components.

In addition, the resin (A) may be used alone or in combination of two or more kinds thereof.

### <Another Photoacid Generator>

The resist composition may include another photoacid generator (a compound which does not correspond to the specific compound and generates an acid upon irradiation with actinic rays or radiation) which does not correspond to the specific compound. Such another photoacid generator is a compound which generates an acid upon exposure (preferably exposure to EUV light and/or ArF).

Such another photoacid generator may be in a form of a low-molecular-weight compound or a form incorporated into a part of a polymer. Furthermore, a combination of the form of a low-molecular-weight compound and the form incorporated into a part of a polymer may also be used.

In a case where such another photoacid generator is in the form of a low-molecular-weight compound, the molecular weight is preferably 3,000 or less, more preferably 2,000 or less, and still more preferably 1,000 or less.

In a case where such another photoacid generator is in the form incorporated into a part of a polymer, it may be incorporated into a part of the resin (A) or into a resin that is different from the resin (A).

In the present invention, the photoacid generator is preferably in the form of the low-molecular-weight compound.

Such another photoacid generator is not particularly limited, and above all, a compound which generates an organic acid is preferable, and examples of the organic acid include the same ones as the organic acids described as the organic acid which can be generated by the specific compound.

Examples of such another photoacid generator include a sulfonium salt compound, an iodonium salt compound, a diazonium salt compound, a phosphonium salt compound, an imidosulfonate compound, an oxime sulfonate compound, a diazodisulfone compound, a disulfone compound, and an o-nitrobenzyl sulfonate compound, each of which is other than the specific compound.

Such another photoacid generator may be a zwitterion.

As such another photoacid generators, known compounds that generate an acid upon irradiation with actinic rays or radiation can be used singly or as a mixture thereof, appropriately selected and used. For example, the known compounds disclosed in paragraphs [0125] to [0319] of the specification of US2016/0070167A1, paragraphs [0086] to [0094] of the specification of US2015/0004544A1, and paragraphs [0323] to [0402] of the specification of US2016/0237190A1 may be used.

In a case where the resist composition includes such another photoacid generator, a content thereof is not particularly limited, but from the viewpoint that the effect of the present invention is more excellent, the content is preferably 0.1% to 40% by mass or more, more preferably 0.5% to 20% by mass or more, and still more preferably 1% to 10% by mass or more with respect to a total solid content of the composition.

Such another photoacid generator may be used alone or in combination of two or more kinds thereof.

### <Solvent>

The resist composition may include a solvent.

The solvent preferably includes at least one of (M1) propylene glycol monoalkyl ether carboxylate (propylene glycol monomethyl ether acetate (PGMEA) and the like), or (M2) at least one selected from the group consisting of a propylene glycol monoalkyl ether (propylene glycol monomethyl ether (PGME), propylene glycol monoethyl ether (PGEE), and the like), a lactic acid ester (ethyl lactate and the like), an acetic acid ester, an alkoxypropionic acid ester, a chain ketone, a cyclic ketone (2-heptanone, cyclohexanone, cyclopentanone, and the like), a lactone (γ-butyrolactone and the like), and an alkylene carbonate (propylene carbonate and the like). Furthermore, this solvent may further include components other than the components (M1) and (M2).

It is preferable that the solvent includes the component (M1). The solvent is more preferably formed of substantially only the component (M1) or is a mixed solvent of the component (M1) and other components. In a case where the solvent is the mixed solvent, it is still more preferable that the solvent includes both the component (M1) and the component (M2).

A mass ratio (M1/M2) of the component (M1) to the component (M2) is preferably "100/0" to "0/100", more preferably "100/0" to "15/85", still more preferably "100/0" to "40/60", and particularly preferably "100/0" to "60/40".

As described above, the solvent may further include components other than the components (M1) and (M2). In this case, a content of the components other than the components (M1) and (M2) is preferably 5% to 30% by mass with respect to the total mass of the solvent.

A content of the solvent in the resist composition is preferably set so that the concentration of solid contents is 0.5% to 30% by mass, and more preferably set so that the concentration of solid contents is 1% to 20% by mass. With this content, the coating property of the resist composition can be further improved.

Furthermore, the solid content means all the components excluding the solvent.

### <Acid Diffusion Control Agent>

The resist composition may further include an acid diffusion control agent. The acid diffusion control agent acts as a quencher that traps an acid generated from a photoacid generator and functions to control the phenomenon of acid diffusion in the resist film.

The acid diffusion control agent may be, for example, a basic compound.

The basic compound is preferably a compound having a structure represented by each of General Formula (A) to General Formula (E).

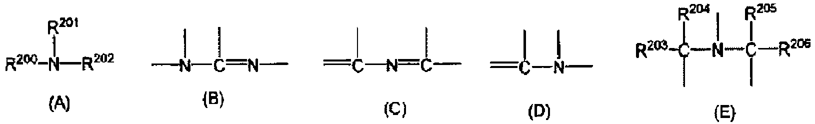

In General Formula (A) and General Formula (E), R²⁰⁰, R²⁰¹, and R²⁰² may be the same as or different from each other, and each represent a hydrogen atom, an alkyl group (preferably having 1 to 20 carbon atoms), a cycloalkyl group (preferably having 3 to 20 carbon atoms), or an aryl group (preferably having 6 to 20 carbon atoms), in which R²⁰¹ and R²⁰² may be bonded to each other to form a ring.

With regard to the alkyl group, the alkyl group having a substituent is preferably an aminoalkyl group having 1 to 20 carbon atoms, a hydroxyalkyl group having 1 to 20 carbon atoms, or a cyanoalkyl group having 1 to 20 carbon atoms.

R²⁰³, R²⁰⁴, R²⁰⁵, and R²⁰⁶ may be the same as or different from each other, and each represent an alkyl group having 1 to 20 carbon atoms.

It is more preferable that the alkyl groups in General Formula (A) and General Formula (E) are unsubstituted.

### (Compound (PA) Which Has Proton-Accepting Functional Group and Generates Compound That Decomposes upon Irradiation with Actinic Rays or Radiation to Exhibit Deterioration in Proton-Accepting Properties, No Proton-Accepting Properties, or Change from Proton-Accepting Properties to Acidic Properties)

The resist composition may include a compound (hereinafter also referred to as a "compound (PA)") which has a proton-accepting functional group and generates a compound that decomposes upon irradiation with actinic rays or radiation to exhibit deterioration in proton-accepting properties, no proton-accepting properties, or a change from the proton-accepting properties to acidic properties as an acid diffusion control agent.

The proton-accepting functional group refers to a functional group having a group or electron capable of electrostatically interacting with a proton, and for example, means a functional group with a macrocyclic structure, such as a cyclic polyether, or a functional group having a nitrogen atom having an unshared electron pair not contributing to π-conjugation. The nitrogen atom having an unshared electron pair not contributing to π-conjugation is, for example, a nitrogen atom having a partial structure represented by the following general formula.

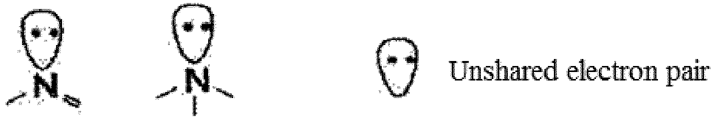

Preferred examples of the partial structure of the proton-accepting functional group include a crown ether structure, an azacrown ether structure, primary to tertiary amine structures, a pyridine structure, an imidazole structure, and a pyrazine structure.

The compound (PA) decomposes upon irradiation with actinic rays or radiation to generate a compound exhibiting deterioration in proton-accepting properties, no proton-accepting properties, or a change from the proton-accepting properties to acidic properties. Here, an expression of generating a compound which exhibits deterioration in proton-accepting properties, no proton-accepting properties, or a change from the proton-accepting properties to acidic properties is a change of proton-accepting properties due to the proton being added to the proton-accepting functional group. Specifically, the expression means a decrease in the equilibrium constant at chemical equilibrium in a case where a proton adduct is generated from the compound (PA) having the proton-accepting functional group and the proton.

With regard to the compound (PA), reference can be made to those described in paragraphs [0421] to [0428] of JP2014-41328A or paragraphs [0108] to [0116] of JP2014-134686A, the contents of which are incorporated herein by reference.

A low-molecular-weight compound having a nitrogen atom and a group that leaves by the action of an acid can also be used as an acid diffusion control agent. The low-molecular-weight compound is preferably an amine derivative having, on the nitrogen atom, a group that leaves by the action of an acid.

The group that leaves by the action of an acid is preferably an acetal group, a carbonate group, a carbamate group, a tertiary ester group, a tertiary hydroxyl group, or a hemiaminal ether group, and more preferably the carbamate group or the hemiaminal ether group.

The molecular weight of the low-molecular-weight compound is preferably 100 to 1,000, more preferably 100 to 700, and still more preferably 100 to 500.

The low-molecular-weight compound may have a carbamate group having a protective group on the nitrogen atom.

Examples of the acid diffusion control agent include the compounds (amine compounds, amide group-containing compounds, urea compounds, nitrogen-containing heterocyclic compounds, and the like) described in paragraphs [0140] to [0144] of JP2013-11833A.

As the acid diffusion control agent, for example, the contents described in paragraphs [0123] to [0159] of JP2018-155788A can also be incorporated.

Specific examples of the acid diffusion control agent are shown below, but the present invention is not limited.

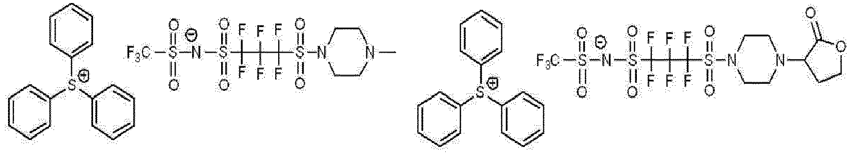

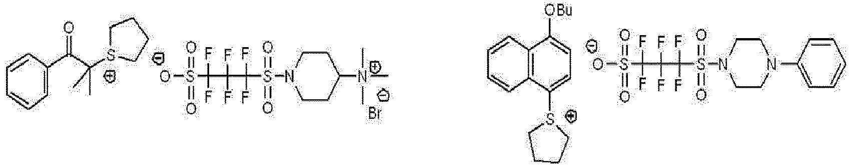

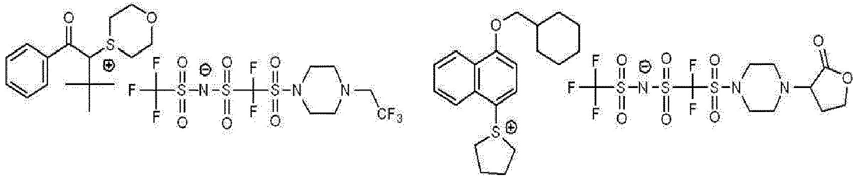

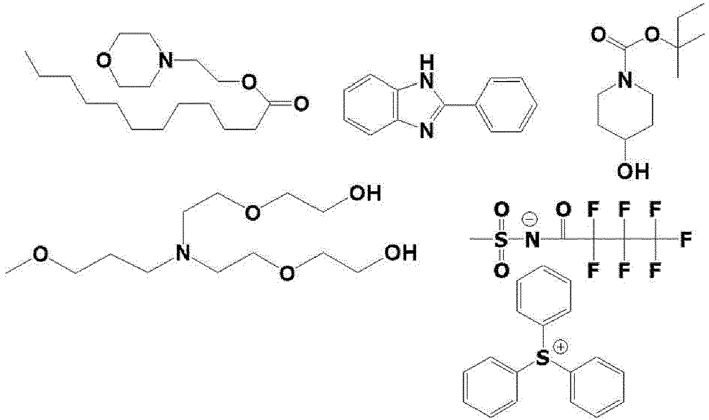

In a case where the resist composition includes an acid diffusion control agent, a content of the acid diffusion control agent is preferably 0.001% to 15% by mass, and more preferably 0.01% to 8% by mass with respect to a total solid content of the resist composition.

The acid diffusion control agents may be used alone or in combination of two or more kinds thereof.

### <Hydrophobic Resin>

The resist composition may include a hydrophobic resin different from the resin (A), in addition to the resin (A).

Although it is preferable that the hydrophobic resin is designed to be unevenly distributed on a surface of the resist film, it does not necessarily need to have a hydrophilic group in the molecule as different from the surfactant, and does not need to contribute to uniform mixing of polar materials and non-polar materials.

Examples of the effect caused by the addition of the hydrophobic resin include a control of static and dynamic contact angles of a surface of the resist film with respect to water and suppression of out gas.

The hydrophobic resin preferably has any one or more of a "fluorine atom", a "silicon atom", and a "CH₃ partial structure which is contained in a side chain moiety of a resin" from the viewpoint of uneven distribution on the film surface layer, and more preferably has two or more kinds thereof. In addition, the hydrophobic resin preferably has a hydrocarbon group having 5 or more carbon atoms. These groups may be contained in the main chain of the resin or may be substituted in a side chain.

With regard to the hydrophobic resin, reference can be made to the description in paragraphs [0315] to [0415] of JP2014-010245A, the contents of which are incorporated herein by reference.

Furthermore, for the hydrophobic resin, the resins described in JP2011-248019A, JP2010-175859A, and JP2012-032544A can also be preferably used, in addition to the resins described above.

In a case where the resist composition includes a hydrophobic resin, a content of the hydrophobic resin is preferably 0.01% to 20% by mass, and more preferably 0.1% to 15% by mass with respect to the total solid content of the resist composition.

### <Surfactant>

The resist composition may include a surfactant. In a case where the surfactant is included, it is possible to form a pattern having more excellent adhesiveness and fewer development defects.

The surfactant is preferably a fluorine-based and/or silicon-based surfactant.

Examples of the fluorine-based and/or silicon-based surfactant include the surfactants described in paragraph [0276] of the specification of US2008/0248425A. In addition, EFTOP EF301 or EF303 (manufactured by Shin-Akita Chemical Co., Ltd.); FLUORAD FC430, 431, and 4430 (manufactured by Sumitomo 3M inc.); MEGAFACE F171, F173, F176, F189, F113, F110, F177, F120, and R08 (manufactured by DIC Corporation); SURFLON S-382, SC101, 102, 103, 104, 105, or 106 (manufactured by Asahi Glass Co., Ltd.); TROYSOL S-366 (manufactured by Troy Corporation); GF-300 or GF-150 (manufactured by Toagosei Co., Ltd.); SURFLON S-393 (manufactured by AGC Seimi Chemical Co., Ltd.); EFTOP EF121, EF122A, EF122B, RF122C, EF125M, EF135M, EF351, EF352, EF801, EF802, or EF601 (manufactured by JEMCO Inc.); PF636, PF656, PF6320, and PF6520 (manufactured by OMNOVA Solutions Inc.); KH-20 (manufactured by Asahi Kasei Corporation); or FTX-204G, 208G, 218G, 230G, 204D, 208D, 212D, 218D, and 222D (manufactured by NEOS COMPANY LIMITED) may be used. In addition, a polysiloxane polymer KP-341 (manufactured by Shin-Etsu Chemical Co., Ltd.) can also be used as the silicon-based surfactant.

Moreover, in addition to the known surfactants as shown above, a surfactant may be synthesized using a fluoroaliphatic compound manufactured using a telomerization method (also referred to as a telomer method) or an oligomerization method (also referred to as an oligomer method). Specifically, a polymer including a fluoroaliphatic group derived from fluoroaliphatic compound may be used as the surfactant. This fluoroaliphatic compound can be synthesized, for example, by the method described in JP2002-90991A.

In addition, a surfactant other than the fluorine-based surfactant and/or the silicon-based surfactants described in paragraph [0280] of the specification of US2008/0248425A may be used.

The surfactants may be used alone or in combination of two or more kinds thereof.

In a case where the resist composition includes a surfactant, a content of the surfactant is preferably 0.0001% to 2% by mass, and more preferably 0.0005% to 1% by mass with respect to the total solid content of the composition.

### <Other Additives>

The resist composition may further include, in addition to the components, a dissolution inhibiting compound, a dye, a plasticizer, a photosensitizer, a light absorber, and/or a compound promoting a solubility in a developer (for example, a phenol compound having a molecular weight of 1,000 or less or an alicyclic or aliphatic compound including a carboxylic acid group), or the like.

### [Resist Film and Pattern Forming Method]

The procedure of the pattern forming method using the resist composition is not particularly limited, but preferably has the following steps.
Step 1: A step of forming a resist film on a substrate, using a resist composition
Step 2: A step of exposing the resist film
Step 3: A step of developing the exposed resist film, using a developer, to form a pattern

Hereinafter, the procedure of each of the steps will be described in detail.

### <Step 1: Resist Film Forming Step>

The step 1 is a step of forming a resist film on a substrate, using a resist composition.

The definition of the resist composition is as described above.

Hereinafter, a specific example of the method for preparing the resist composition will be shown.

In the resist composition used in the pattern forming method of the embodiment of the present invention, it is preferable that the content of metal atoms is reduced.

Hereinafter, first, a specific example of a method for reducing the content of the metal atoms in the resist composition will be described, and then a specific example of a method for preparing the resist composition will be described.

Examples of the method for reducing the content of the metal atoms in the resist composition include a method for adjusting the content by filtration using a filter. As for the filter pore diameter, the pore size is preferably less than 100 nm, more preferably 10 nm or less, and still more preferably 5 nm or less. As the filter, a polytetrafluoroethylene-made, polyethylene-made, or nylon-made filter is preferable. The filter may include a composite material in which the filter material is combined with an ion exchange medium. As the filter, a filter which has been washed with an organic solvent in advance may be used. In the step of filter filtration, plural kinds of filters connected in series or in parallel may be used. In a case of using the plural kinds of filters, a combination of filters having different pore diameters and/or materials may be used. In addition, various materials may be filtered plural times, and the step of filtering plural times may be a circulatory filtration step.

In addition, examples of a method for reducing the content of the metal atoms in the resist composition include a method of selecting raw materials having a low content of metals as raw materials constituting various materials in the resist composition, a method of subjecting raw materials constituting various materials in the resist composition to filter filtration, and a method of performing distillation under the condition for suppressing the contamination as much as possible by, for example, lining the inside of a device with TEFLON (registered trademark).

Moreover, as the method for reducing the content of the metal atoms in the resist composition, removal with an adsorbing material may be performed, in addition to the above-mentioned filter filtration, and the filter filtration and the adsorbing material may be used in combination. As the adsorbing material, known adsorbing materials can be used, and for example, inorganic adsorbing materials such as silica gel and zeolite, and organic adsorbing materials such as activated carbon can be used.

In addition, in order to reduce the content of the metal atoms in the resist composition, it is necessary to prevent the incorporation of metal impurities in the production process. Sufficient removal of metal impurities from a production device can be confirmed by measuring the content of metal components included in a washing liquid used to wash the production device.

Next, a specific example of the method for preparing the resist composition will be described.

In the production of the resist composition, for example, it is preferable to dissolve various components such as the resin and the photoacid generator as described above in a solvent, and then perform filtration (which may be circulatory filtration) using a plurality of filters having different materials. For example, it is preferable to connect a polyethylene-made filter with a pore diameter of 50 nm, a nylon-made filter with a pore diameter of 10 nm, and a polyethylene-made filter with a pore diameter of 3 to 5 nm in permuted connection, and then perform filtration. As for the filtration, a method of performing circulatory filtration twice or more is also preferable. Furthermore, the filtration step also has an effect of reducing the content of the metal atoms in the resist composition. A smaller pressure difference among the filters is more preferable, and the pressure difference is generally 0.1 MPa or less, preferably 0.05 MPa or less, and more preferably 0.01 MPa or less. A smaller pressure difference between the filter and the charging nozzle is more preferable, and the pressure difference is generally 0.5 MPa or less, preferably 0.2 MPa or less, and more preferably 0.1 MPa or less.

In addition, as a method for performing circulatory filtration using a filter in the production of the resist composition, for example, a method of performing circulatory filtration twice or more using a polytetrafluoroethylene-made filter having a pore diameter of 50 nm is also preferable.

It is preferable to subject the inside of a device for producing the resist composition to gas replacement with an inert gas such as nitrogen. With this, it is possible to suppress dissolution of an active gas such as oxygen in the composition.

After being filtered by a filter, the resist composition is charged into a clean container. It is preferable that the composition charged in the container is subjected to cold storage. This enables performance deterioration caused by the lapse of time to be suppressed. A shorter time from completion of the charge of the resist composition into the container to initiation of cold storage is more preferable, and the time is generally 24 hours or shorter, preferably 16 hours or shorter, more preferably 12 hours or shorter, and still more preferably 10 hours or shorter. The storage temperature is preferably 0°C to 15°C, more preferably 0°C to 10°C, and still more preferably 0°C to 5°C.

Next, a method of forming a resist film on a substrate, using the composition will be described.

Examples of a method in which a resist film is formed on a substrate, using a resist composition, include a method in which a resist composition is applied onto a substrate.

The resist composition can be applied onto a substrate (for example, silicon and silicon dioxide coating) as used in the manufacture of integrated circuit elements by a suitable application method such as ones using a spinner or a coater. The application method is preferably spin application using a spinner. A rotation speed upon the spin application using a spinner is preferably 1,000 to 3,000 rpm.

After the application of the resist composition, the substrate may be dried to form a resist film. In addition, various underlying films (an inorganic film, an organic film, or an antireflection film) may be formed on the underlayer of the resist film.

Examples of the drying method include a method of heating and drying. The heating can be carried out using a unit included in an ordinary exposure machine and/or an ordinary development machine, and may also be carried out using a hot plate or the like. A heating temperature is preferably 80°C to 150°C, more preferably 80°C to 140°C, and still more preferably 80°C to 130°C. A heating time is preferably 30 to 1,000 seconds, more preferably 60 to 800 seconds, and still more preferably 60 to 600 seconds.

A film thickness of the resist film is not particularly limited, but is preferably 10 to 65 nm, and more preferably 15 to 50 nm from the viewpoint that a fine pattern having higher accuracy can be formed.

Moreover, a topcoat may be formed on the upper layer of the resist film, using the topcoat composition.

It is preferable that the topcoat composition is not mixed with the resist film and can be uniformly applied onto the upper layer of the resist film.

The topcoat composition includes, for example, a resin, an additive, and a solvent.

The topcoat is not particularly limited, a topcoat known in the related art can be formed by the methods known in the related art, and the topcoat can be formed, based on the description in paragraphs [0072] to [0082] of JP2014-059543A, for example.

It is preferable that a topcoat including a basic compound as described in JP2013-61648A, for example, is formed on a resist film. Specific examples of the basic compound which can be included in the topcoat include a basic compound which may be included in the resist composition.

In addition, it is also preferable that the topcoat includes a compound which includes at least one group or bond selected from the group consisting of an ether bond, a thioether bond, a hydroxyl group, a thiol group, a carbonyl bond, and an ester bond.

### <Step 2: Exposing Step>

The step 2 is a step of exposing the resist film.

Examples of an exposing method include a method in which a resist film formed is irradiated with light through a predetermined mask.

It is preferable to perform baking (heating) before performing development after the exposure. The baking accelerates a reaction in the exposed area, and the sensitivity and the pattern shape are improved.

A heating temperature is preferably 80°C to 150°C, more preferably 80°C to 140°C, and still more preferably 80°C to 130°C.

A heating time is preferably 10 to 1,000 seconds, more preferably 10 to 180 seconds, and still more preferably 30 to 120 seconds.

The heating can be carried out using a unit included in an ordinary exposure machine and/or an ordinary development machine, and may also be performed using a hot plate or the like.

This step is also referred to as a post-exposure baking.

### <Step 3: Developing Step>

The step 3 is a step of developing the exposed resist film, using a developer, to form a pattern.

The developer may be either an alkali developer or a developer containing an organic solvent (hereinafter also referred to as an organic developer).

Examples of the developing method include a method in which a substrate is immersed in a tank filled with a developer for a certain period of time (a dip method), a method in which development is performed by heaping a developer up onto the surface of a substrate by surface tension, and then leaving it to stand for a certain period of time (a puddle method), a method in which a developer is sprayed on the surface of a substrate (a spray method), and a method in which a developer is continuously jetted onto a substrate rotating at a constant rate while scanning a developer jetting nozzle at a constant rate (a dynamic dispense method).

Furthermore, after the step of performing development, a step of stopping the development may be carried out while substituting the solvent with another solvent.

A developing time is not particularly limited as long as it is a period of time where the unexposed area of a resin is sufficiently dissolved, and is preferably 10 to 300 seconds, and more preferably 20 to 120 seconds.

A temperature of the developer is preferably 0°C to 50°C, and more preferably 15°C to 35°C.

As the alkali developer, it is preferable to use an aqueous alkali solution including an alkali. The type of the aqueous alkali solution is not particularly limited, but examples thereof include an aqueous alkali solution including a quaternary ammonium salt typified by tetramethylammonium hydroxide, an inorganic alkali, a primary amine, a secondary amine, a tertiary amine, an alcoholamine, a cyclic amine, or the like. Among those, the aqueous solutions of the quaternary ammonium salts typified by tetramethylammonium hydroxide (TMAH) are preferable as the alkali developer. An appropriate amount of an alcohol, a surfactant, or the like may be added to the alkali developer. The alkali concentration of the alkali developer is usually 0.1% to 20% by mass. Furthermore, the pH of the alkali developer is usually 10.0 to 15.0.

The organic developer is preferably a developer containing at least one organic solvent selected from the group consisting of a ketone-based solvent, an ester-based solvent, an alcohol-based solvent, an amide-based solvent, an ether-based solvent, and a hydrocarbon-based solvent.

A plurality of the solvents may be mixed or the solvent may be used in admixture with a solvent other than those described above or water. The moisture content in the entire developer is preferably less than 50% by mass, more preferably less than 20% by mass, and still more preferably less than 10% by mass, and particularly preferably moisture is not substantially contained.

A content of the organic solvent with respect to the organic developer is preferably from 50% by mass to 100% by mass, more preferably from 80% by mass to 100% by mass, still more preferably from 90% by mass to 100% by mass, and particularly preferably from 95% by mass to 100% by mass with respect to the total amount of the developer.

### <Other Steps>

It is preferable that the pattern forming method includes a step of performing washing using a rinsing liquid after the step 3.

Examples of the rinsing liquid used in the rinsing step after the step of performing development using an alkali developer include pure water. Furthermore, an appropriate amount of a surfactant may be added to pure water.

An appropriate amount of a surfactant may be added to the rinsing liquid.

The rinsing liquid used in the rinsing step after the developing step with an organic developer is not particularly limited as long as the rinsing liquid does not dissolve the pattern, and a solution including a common organic solvent can be used. As the rinsing liquid, a rinsing liquid containing at least one organic solvent selected from the group consisting of a hydrocarbon-based solvent, a ketone-based solvent, an ester-based solvent, an alcohol-based solvent, an amide-based solvent, and an ether-based solvent is preferably used.

A method for the rinsing step is not particularly limited, and examples thereof include a method in which a rinsing liquid is continuously jetted on a substrate rotated at a constant rate (a rotation application method), a method in which a substrate is immersed in a tank filled with a rinsing liquid for a certain period of time (a dip method), and a method in which a rinsing liquid is sprayed on a substrate surface (a spray method).

Furthermore, the pattern forming method of the embodiment of the present invention may include a heating step (postbaking) after the rinsing step. By the present step, the developer and the rinsing liquid remaining between and inside the patterns are removed by baking. In addition, the present step also has an effect that a resist pattern is annealed and the surface roughness of the pattern is improved. The heating step after the rinsing step is usually performed at 40°C to 250°C (preferably 90°C to 200°C) for usually 10 seconds to 3 minutes (preferably 30 seconds to 120 seconds).

In addition, an etching treatment on the substrate may be carried out using a pattern formed as a mask.

For etching, any of known methods can be used, and various conditions and the like are appropriately determined according to the type of a substrate, usage, and the like. Etching can be carried out, for example, in accordance with Journal of The International Society for Optical Engineering (Proc. of SPIE), Vol. 6924, 692420 (2008), JP2009-267112A, and the like. In addition, the etching can also be carried out in accordance with "Chapter 4 Etching" in "Semiconductor Process Text Book, 4^{th} Ed., published in 2007, publisher: SEMI Japan".

Various materials (for example, a developer, a rinsing liquid, a composition for forming an antireflection film, and a composition for forming a topcoat) other than the resist composition used in the pattern forming method of the embodiment of the present invention preferably have smaller amounts of impurities such as a metal (for example, Na, K, Ca, Fe, Cu, Mg, Al, Li, Cr, Ni, Sn, Ag, As, Au, Ba, Cd, Co, Pb, Ti, V, W, and Zn). The content of the impurities included in these materials is preferably for example, 1 ppm by mass or less.

Examples of a method for reducing impurities such as a metal in various materials other than the resist composition include filtration using a filter. As for the filter pore diameter, the pore size is preferably less than 100 nm, more preferably 10 nm or less, and still more preferably 5 nm or less. As the filter, a polytetrafluoroethylene-made, polyethylene-made, or nylon-made filter is preferable. The filter may include a composite material in which the filter material is combined with an ion exchange medium. As the filter, a filter which has been washed with an organic solvent in advance may be used. In the step of filter filtration, plural kinds of filters connected in series or in parallel may be used. In a case of using the plural kinds of filters, a combination of filters having different pore diameters and/or materials may be used. In addition, various materials may be filtered plural times, and the step of filtering plural times may be a circulatory filtration step.

In addition, examples of a method for reducing impurities such as a metal in various materials other than the resist composition include a method of selecting raw materials having a low content of metals as raw materials constituting various materials, a method of subjecting raw materials constituting various materials to filter filtration, and a method of performing distillation under the condition for suppressing the contamination as much as possible by, for example, lining the inside of a device with TEFLON (registered trademark).

Moreover, as the method for reducing impurities such as a metal in various materials other than the resist composition, removal of impurities with an adsorbing material may be performed, in addition to the above-mentioned filter filtration, and the filter filtration and the adsorbing material may be used in combination. As the adsorbing material, known adsorbing materials can be used, and for example, inorganic adsorbing materials such as silica gel and zeolite, and organic adsorbing materials such as activated carbon can be used. It is necessary to prevent the incorporation of metal impurities in the production process in order to reduce the impurities such as a metal included in the various materials other than the resist composition. Sufficient removal of metal impurities from a production device can be confirmed by measuring the content of metal components included in a washing liquid used to wash the production device.

A conductive compound may be added to an organic treatment liquid such as a rinsing liquid in order to prevent breakdown of chemical liquid pipes and various parts (a filter, an O-ring, a tube, or the like) due to electrostatic charging, and subsequently generated electrostatic discharging. The conductive compound is not particularly limited, but examples thereof include methanol. The addition amount is not particularly limited, but from the viewpoint that preferred development characteristics or rinsing characteristics are maintained, the addition amount is preferably 10% by mass or less, and more preferably 5% by mass or less.

For members of the chemical liquid pipe, various pipes coated with stainless steel (SUS), or a polyethylene, polypropylene, or fluorine resin (a polytetrafluoroethylene or perfluoroalkoxy resin, or the like) that has been subjected to an antistatic treatment can be used. In the same manner, for the filter or the O-ring, polyethylene, polypropylene, or a fluorine resin (a polytetrafluoroethylene or perfluoroalkoxy resin, or the like) that has been subjected to an antistatic treatment can be used.

Moreover, the present invention further relates to a method for manufacturing an electronic device, including the pattern forming method, and an electronic device manufactured by the manufacturing method.

The electronic device of an embodiment of the present invention is suitably mounted on electric and electronic equipment (for example, home appliances, office automation (OA)-related equipment, media-related equipment, optical equipment, telecommunication equipment, and the like).

### Examples

Hereinbelow, the present invention will be described in more detail with reference to Examples. The materials, the amounts of materials used, the proportions, the treatment details, the treatment procedure, and the like shown in Examples below may be modified as appropriate as long as the modifications do not depart from the spirit of the present invention. Therefore, the scope of the present invention should not be construed as being limited to Examples shown below.

### [Production of Composition]

Components included in actinic ray-sensitive or radiation-sensitive resin composition (hereinafter also referred to as a "resist composition") used in Examples or Comparative Examples, and production procedure therefor are shown below.

### <Photoacid Generator (Specific Compound and Another Photoacid Generator)>

(Synthesis of X-1)
X-1 which is a specific compound was synthesized, based on the following scheme.

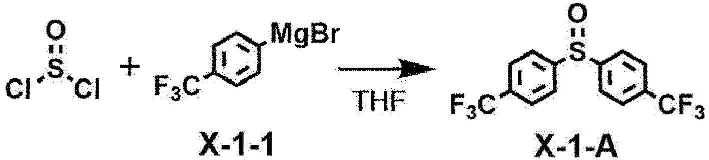

Magnesium (18.0 g) was added to tetrahydrofuran (500 mL) to obtain a mixture. 4-Bromobenzotrifluoride (151.5 g) was added dropwise to the obtained mixture. Then, the mixture was stirred for 1 hour to prepare X-1-1.

Thionyl chloride (37.7 g) was added to tetrahydrofuran (500 mL) to obtain a mixed liquid. The obtained mixed liquid was cooled to 0°C, and X-1-1 prepared above was added dropwise to the mixed liquid. After stirring the mixed liquid for 1 hour, 1 N hydrochloric acid (600 mL) was added to the mixed liquid while maintaining the temperature of the mixed liquid at 0°C.

A reaction product produced in the mixed liquid was extracted with ethyl acetate (600 mL). The obtained organic phase was washed with a saturated aqueous sodium hydrogen carbonate solution (500 mL) and water (500 mL), and then the solvent was evaporated from the organic phase. The obtained concentrate was washed with hexane (300 mL) and filtered to obtain X-1-A (60 g) as a filtrate (yield 56%).

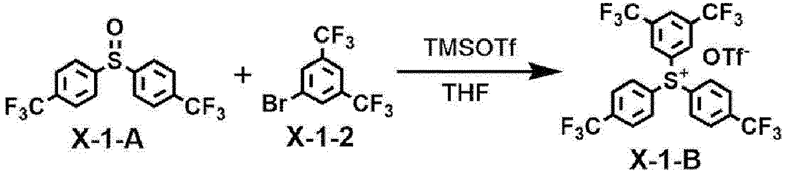

Magnesium (5.0 g) was added to tetrahydrofuran (170 mL) to obtain a mixture. 1-Bromo-3,5-bis(trifluoromethyl)benzene (52.0 g) was added dropwise to the obtained mixture. Then, the mixture was stirred for 1 hour to prepare X-1-2.

X-1-A (20.0 g) was added to tetrahydrofuran (170 mL) to obtain a mixed liquid. The obtained mixed liquid was cooled to 0°C, and trimethylsilyl trifluoromethanesulfonate (91.9 g) was added dropwise to the mixed liquid. Subsequently, X-1-2 prepared above was added dropwise to the mixed liquid, and then the mixed liquid was stirred for 1 hour.

Water (500 mL) was added to the mixed liquid while maintaining the temperature of the mixed liquid at 0°C, and then a reaction product produced in the mixed liquid was extracted with methylene chloride (500 mL).

The obtained organic phase was washed with water (500 mL), and then the solvent was evaporated from the organic phase.

The obtained concentrate was washed with diisopropyl ether (300 mL) and filtered to obtain X-1-B (20 g) as a filtrate (yield 58%).

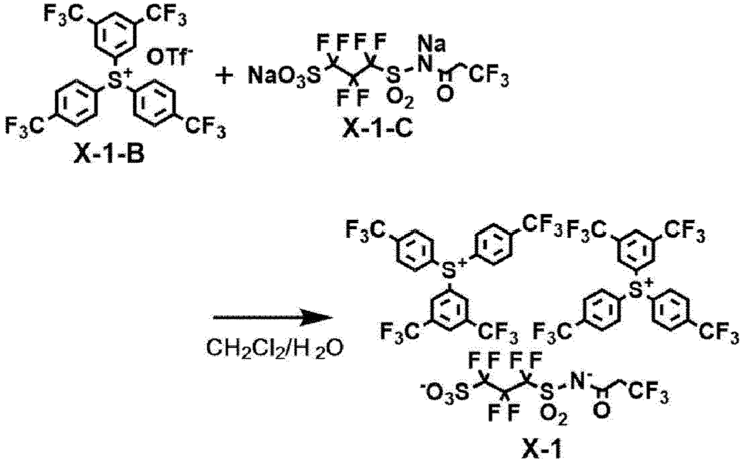

Methylene chloride (100 mL) and water (100 mL) were mixed to obtain a mixed liquid. X-1-B (10.0 g) and X-1-C (4.4 g) were added to the mixed liquid. After stirring the mixed liquid for 1 hour, the aqueous phase was removed from the mixed liquid. The remaining organic phase was washed with a 1%-by-mass aqueous potassium carbonate solution (100 mL), 0.01 N hydrochloric acid (100 mL), and water (100 mL). The solvent was evaporated from the organic phase to obtain X-1 (13.8 g) (yield 99%).

Other specific compounds were synthesized with reference to the synthesis method. Photoacid generators used in Examples are shown below.

Furthermore, X-1 to X-20 correspond to the specific compounds, and B-1 to B-6, and Z-1 do not correspond to the specific compounds.

In addition, X-1 to X-15 correspond to the compounds represented by General Formula (Ib).

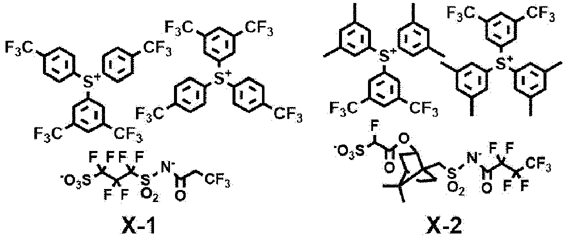

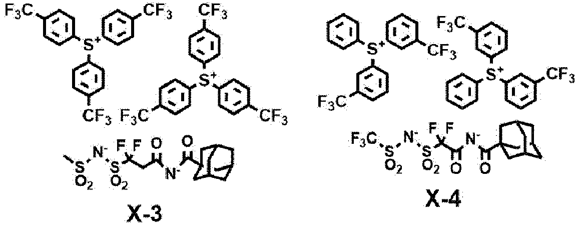

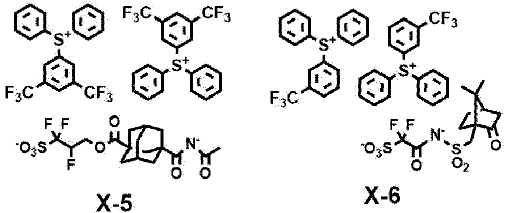

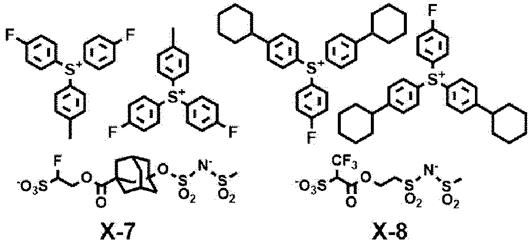

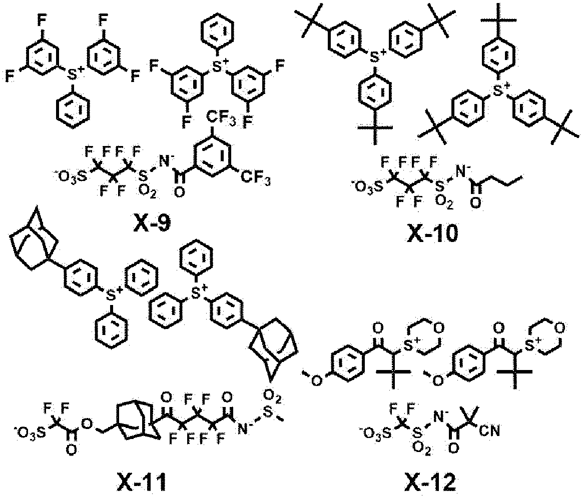

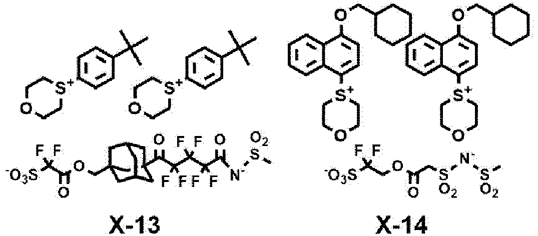

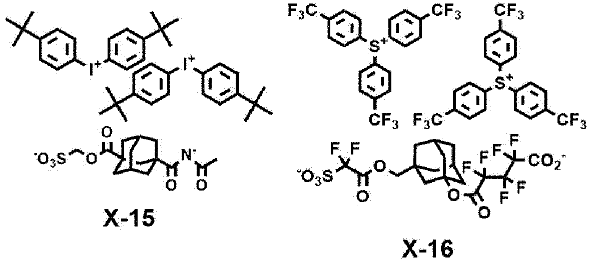

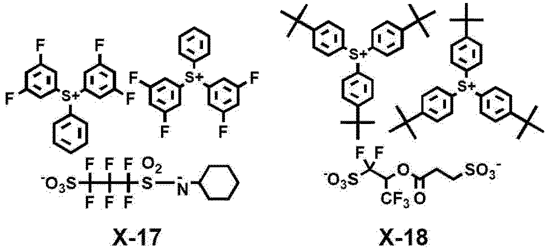

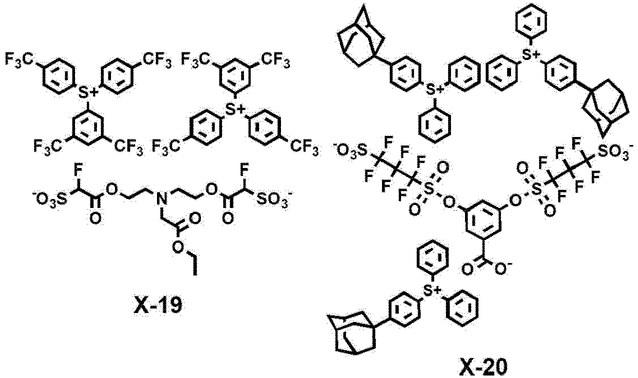

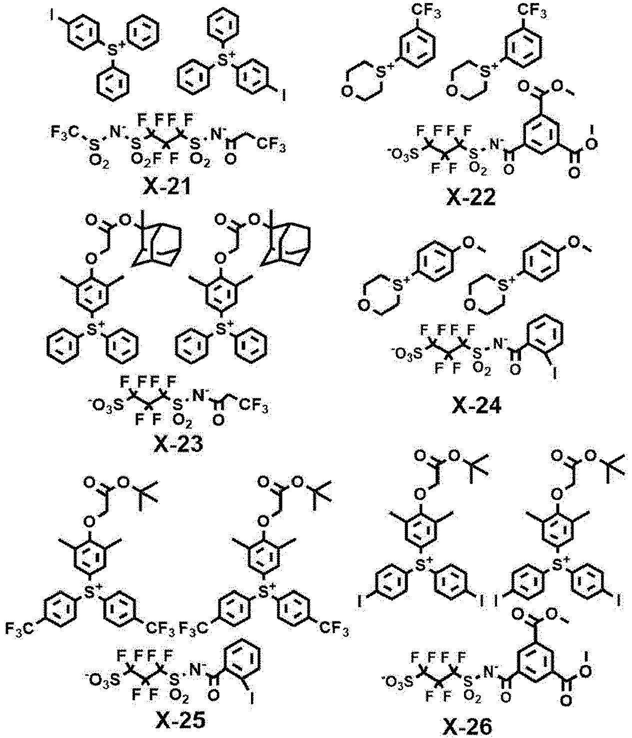

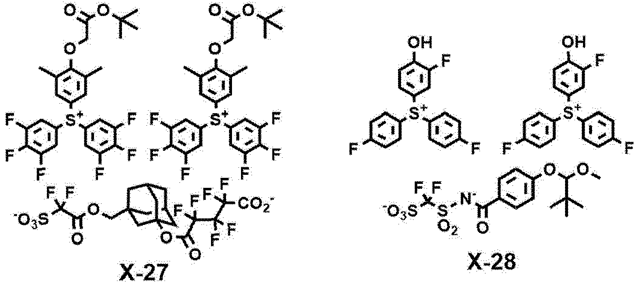

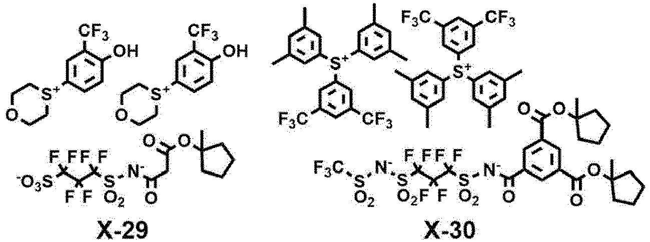

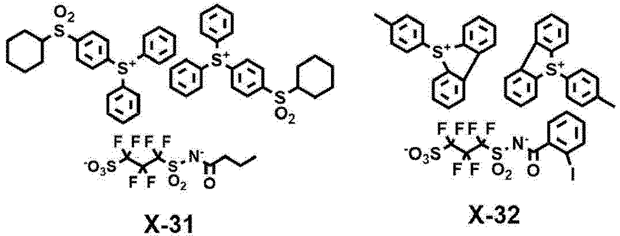

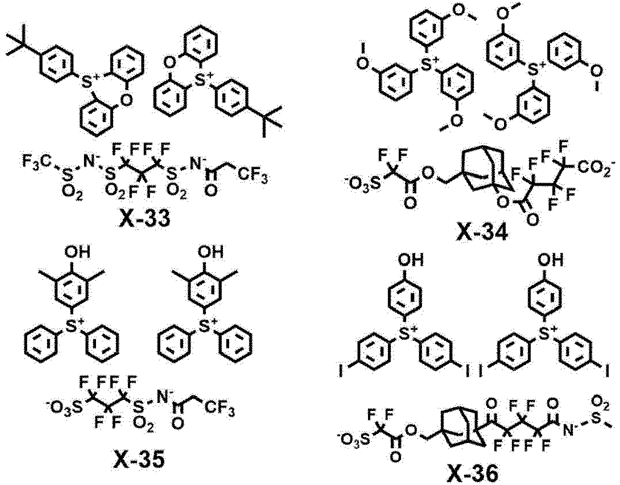

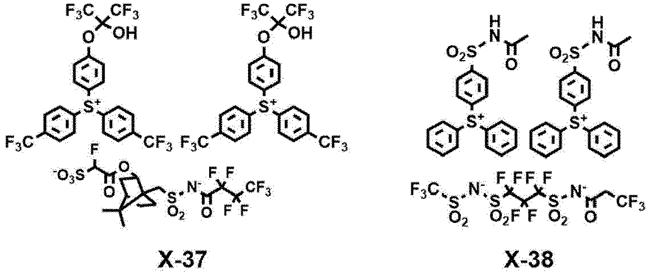

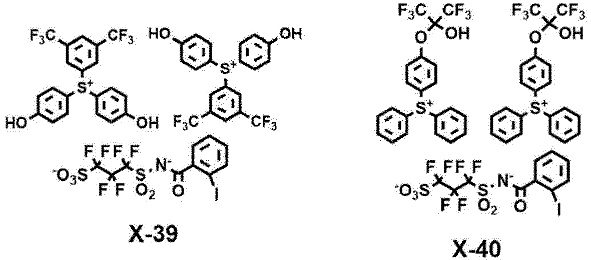

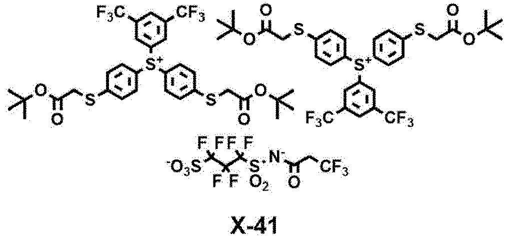

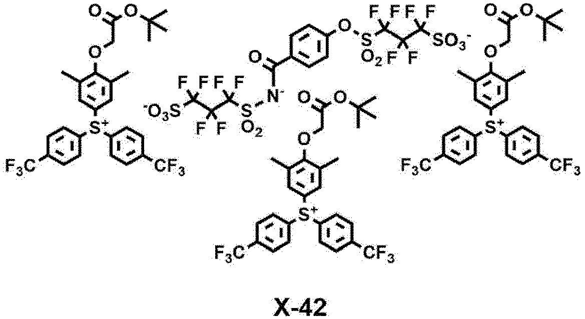

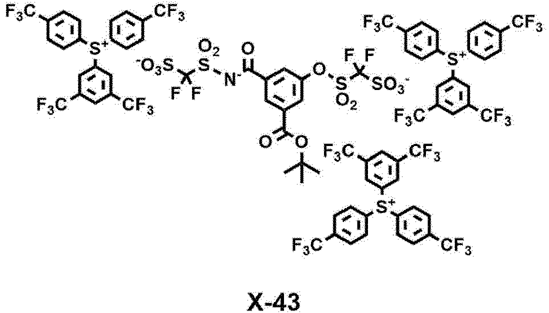

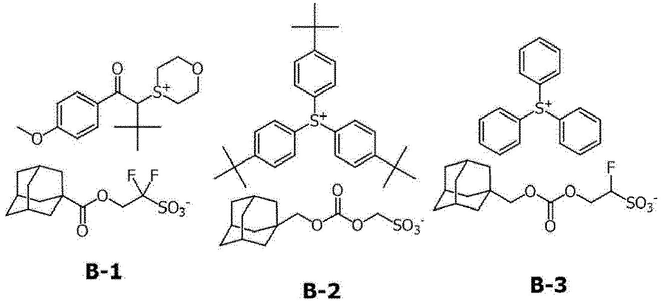

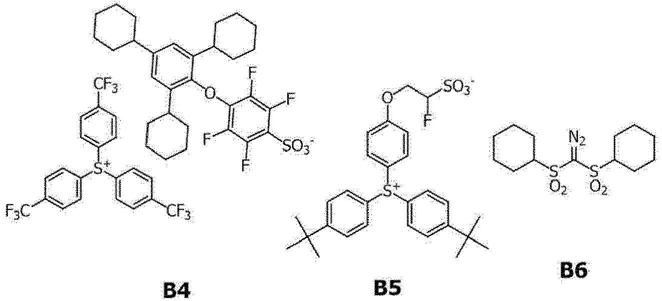

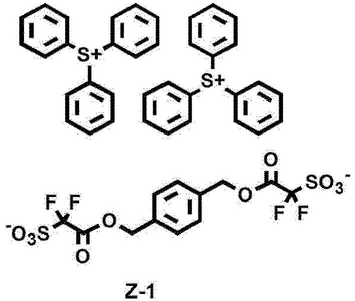

### <Acid-Decomposable Resin (Resin (A))>

An acid-decomposable resin (resin (A)) used for the production of a resist composition is shown below.

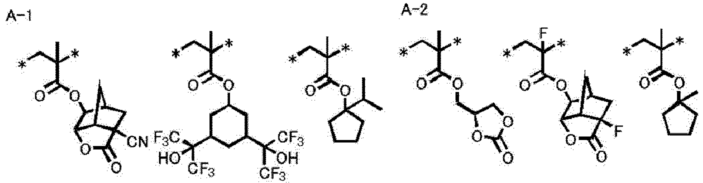

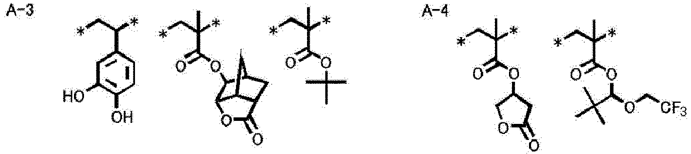

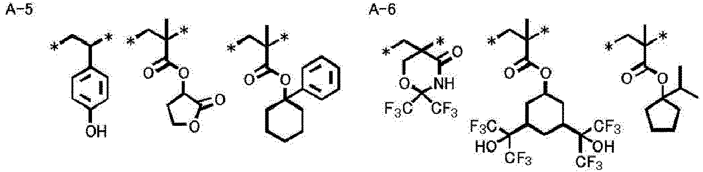

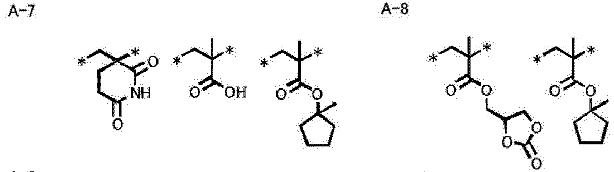

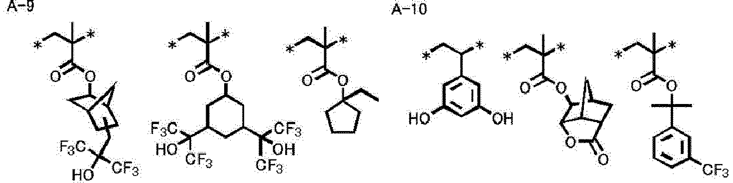

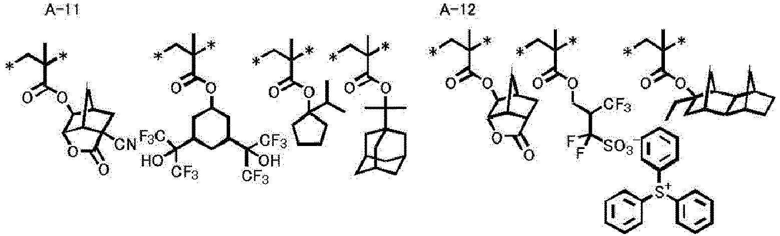

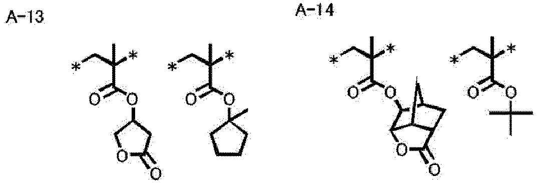

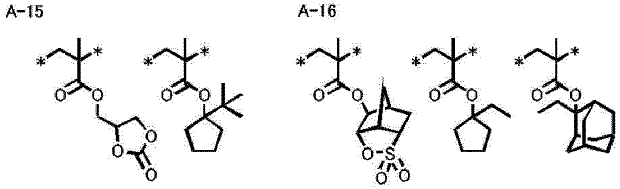

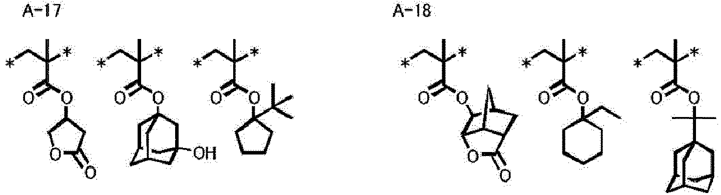

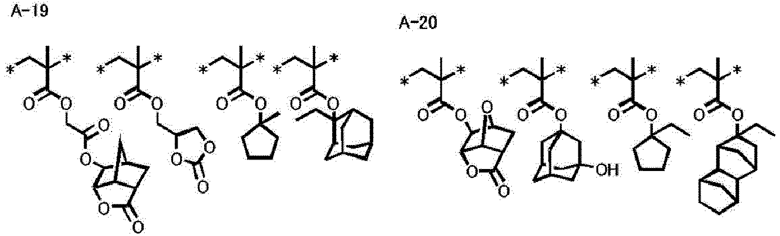

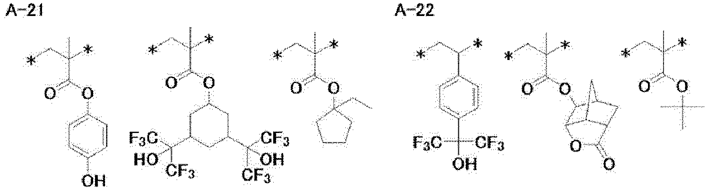

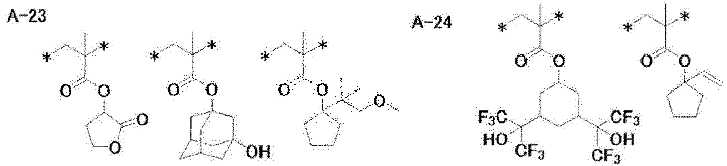

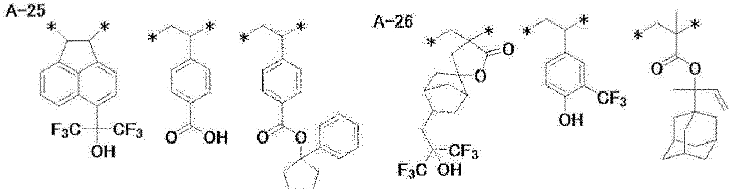

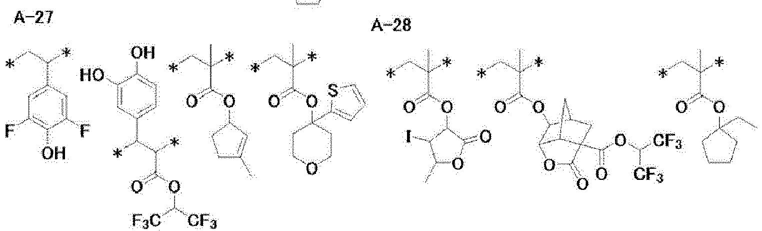

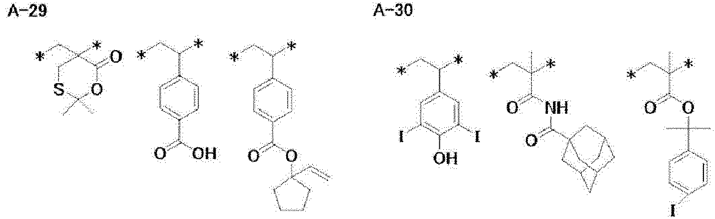

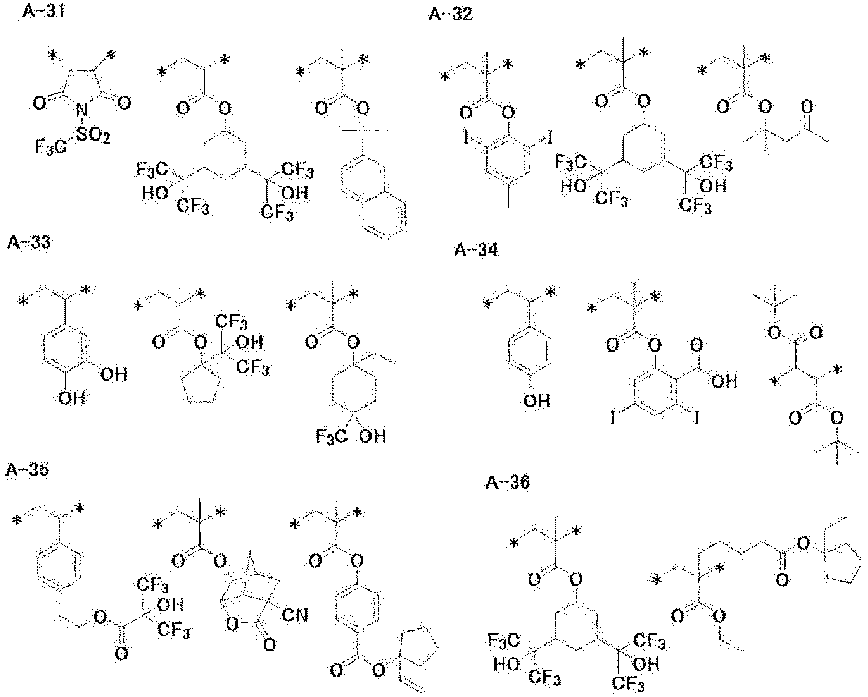

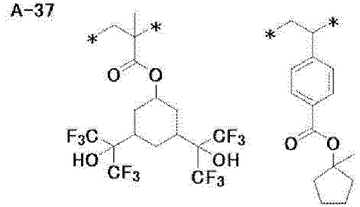

The molar ratios of the repeating units constituting each resin shown above (corresponding in order from the left), and the weight-average molecular weight (Mw) and the dispersity (Mw/Mn) of each resin are shown in the following table.

**[Table 1]**

| Table 1 | Molar ratio of repeating unit | | | | Mw | Mw/Mn |
|---|---|---|---|---|---|---|
| Resin A-1 | 25 | 30 | 45 | - | 6,000 | 1.71 |
| Resin A-2 | 45 | 15 | 40 | - | 10,200 | 1.64 |
| Resin A-3 | 40 | 20 | 40 | - | 7,500 | 1.54 |
| Resin A-4 | 60 | 40 | - | - | 6,800 | 1.52 |
| Resin A-5 | 40 | 10 | 50 | - | 6,500 | 1.63 |
| Resin A-6 | 15 | 40 | 45 | - | 5,900 | 1.59 |
| Resin A-7 | 40 | 20 | 40 | - | 5,100 | 1.51 |
| Resin A-8 | 40 | 60 | - | - | 6,200 | 1.39 |
| Resin A-9 | 25 | 30 | 45 | - | 7,500 | 1.54 |
| Resin A-10 | 30 | 20 | 50 | - | 7,000 | 1.61 |
| Resin A-11 | 20 | 40 | 35 | 5 | 6,500 | 1.63 |
| Resin A-12 | 40 | 20 | 40 | - | 7,000 | 1.73 |
| Resin A-13 | 50 | 50 | - | - | 7,600 | 1.49 |
| Resin A-14 | 40 | 60 | - | - | 9,500 | 1.68 |
| Resin A-15 | 50 | 50 | - | - | 8,500 | 1.63 |
| Resin A-16 | 15 | 45 | 40 | - | 6,400 | 1.51 |
| Resin A-17 | 40 | 10 | 50 | - | 12,000 | 1.49 |
| Resin A-18 | 50 | 30 | 20 | - | 8,000 | 1.65 |
| Resin A-19 | 25 | 30 | 30 | 15 | 8,500 | 1.61 |
| Resin A-20 | 30 | 50 | 10 | 10 | 5,000 | 1.61 |
| Resin A-21 | 20 | 30 | 50 | - | 6,500 | 1.73 |
| Resin A-22 | 45 | 15 | 40 | - | 7,300 | 1.62 |
| Resin A-23 | 40 | 10 | 50 | - | 7,000 | 1.64 |
| Resin A-24 | 70 | 30 | - | - | 11,000 | 1.71 |
| Resin A-25 | 30 | 10 | 60 | - | 8,500 | 1.68 |
| Resin A-26 | 25 | 25 | 50 | - | 6,000 | 1.69 |
| Resin A-27 | 30 | 10 | 50 | 10 | 7,100 | 1.59 |
| Resin A-28 | 30 | 30 | 40 | - | 11,000 | 1.71 |
| Resin A-29 | 30 | 20 | 50 | - | 6,000 | 1.68 |
| Resin A-30 | 50 | 5 | 45 | - | 7,000 | 1.53 |
| Resin A-31 | 20 | 40 | 40 | - | 10,000 | 1.57 |
| Resin A-32 | 30 | 15 | 55 | - | 5,500 | 1.55 |
| Resin A-33 | 40 | 30 | 30 | - | 7,500 | 1.63 |
| Resin A-34 | 25 | 35 | 40 | - | 9,200 | 1.71 |
| Resin A-35 | 40 | 10 | 50 | - | 7,000 | 1.65 |
| Resin A-36 | 50 | 50 | - | - | 13,000 | 1.69 |
| Resin A-37 | 65 | 35 | - | - | 8,500 | 1.66 |

### <Acid Diffusion Control Agent>

In a case where the resist composition included an acid diffusion control agent, the following acid diffusion control agent was used.

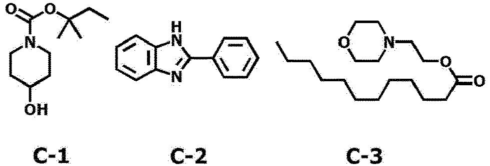

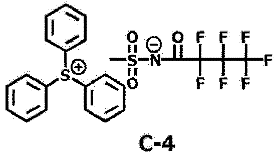

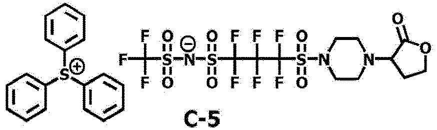

### <Hydrophobic Resin>

In a case where the resist composition included a hydrophobic resin, a hydrophobic resin having a repeating unit based on the following monomer was used.

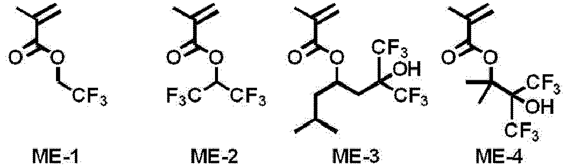

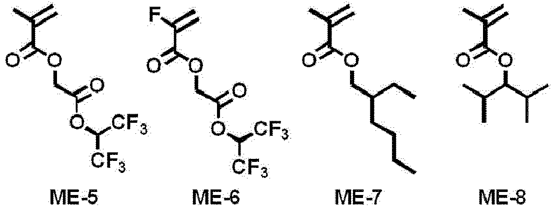

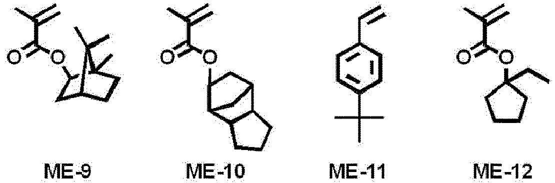

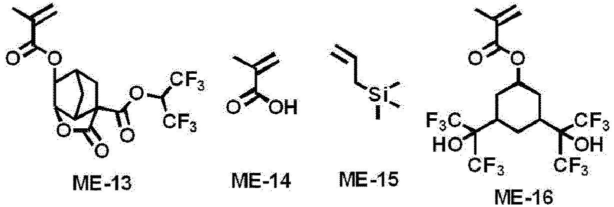

The molar ratios of the repeating units based on the respective monomers, and the weight-average molecular weight (Mw) and the dispersity (Mw/Mn) of each resin in the hydrophobic resin used in the composition are shown in the following table.

**[Table 2]**

| Table 2 | Molar ratio of repeating unit 1 | | Molar ratio of repeating unit 2 | | Molar ratio of repeating unit 3 | | Molar ratio of repeating unit 4 | | Mw | Mw/Mn |
|---|---|---|---|---|---|---|---|---|---|---|
| Resin D-1 | ME-12 | 50 | ME-1 | 50 | - | - | - | - | 12,000 | 1.5 |
| Resin D-2 | ME-2 | 40 | ME-11 | 50 | ME-7 | 5 | ME-14 | 5 | 6,000 | 1.3 |
| Resin D-3 | ME-8 | 50 | ME-2 | 50 | - | - | - | - | 15,000 | 1.5 |
| Resin D-4 | ME-5 | 100 | - | - | - | - | - | - | 23,000 | 1.7 |
| Resin D-5 | ME-11 | 10 | ME-13 | 85 | ME-7 | 5 | - | - | 11,000 | 1.4 |
| Resin D-6 | ME-6 | 80 | ME-9 | 20 | - | - | - | - | 13,000 | 1.4 |
| Resin D-7 | ME-5 | 50 | ME-15 | 50 | - | - | - | - | 12,000 | 1.5 |
| Resin D-8 | ME-2 | 50 | ME-16 | 50 | - | - | - | - | 10,000 | 1.6 |

### <Surfactant>

In a case where the composition included a surfactant, the following surfactants were used.
E-1: MEGAFACE F176 (manufactured by DIC Corporation, fluorine-based surfactant)
E-2: MEGAFACE R08 (manufactured by DIC Corporation, fluorine- and silicon-based surfactant)
E-3: PF656 (manufactured by OMNOVA Solutions Inc., fluorine-based surfactant)

### <Solvent>

Solvents included in the composition are shown below.
F-1: Propylene glycol monomethyl ether acetate (PGMEA)
F-2: Propylene glycol monomethyl ether (PGME)
F-3: Propylene glycol monoethyl ether (PGEE)
F-4: Cyclohexanone
F-5: Cyclopentanone
F-6: 2-Heptanone
F-7: Ethyl lactate
F-8: γ-Butyrolactone
F-9: Propylene carbonate

### <Preparation of Composition>

### (Preparation of Resist Composition for EUV Exposure Test (Re-1 to Re-30, Re-49, and Re-51 to Re-76))

The respective components shown in the following table were mixed so that the concentration of solid contents was 2% by mass. Next, the obtained mixed liquid was filtered initially through a polyethylene-made filter having a pore diameter of 50 nm, then through a nylon-made filter having a pore diameter of 10 nm, and lastly through a polyethylene-made filter having a pore diameter of 5 nm in this order to prepare a resist composition used for a test by EUV exposure (actinic ray-sensitive or radiation-sensitive resin composition).

In addition, in the resist composition, the solid content means all the components excluding the solvent. The obtained resist composition was used in Examples and Comparative Examples.

### (Preparation of Resist Composition for ArF Exposure Test (Re-31 to Re-48, and Re-50))

The respective components shown in the following table were mixed so that the concentration of solid contents was 4% by mass. Next, the obtained mixed liquid was filtered initially through a polyethylene-made filter having a pore diameter of 50 nm, then through a nylon-made filter having a pore diameter of 10 nm, and lastly through a polyethylene-made filter having a pore diameter of 5 nm in this order to prepare a resist composition used for a test by ArF exposure (actinic ray-sensitive or radiation-sensitive resin composition).

The formulation of each composition (the resist composition for an EUV exposure test and the resist composition for an ArF exposure test) is shown below.

The "Amount" column in the tables indicates a content (% by mass) of each component with respect to a total solid content of the resist composition.

**[Table 3]**

| Table 3-1 | Acid-decomposable resin | | Photoacid generator | | | | Acid diffusion control agent | | Hydrophobic resin | | Resin | | Solvent | |
|---|---|---|---|---|---|---|---|---|---|---|---|---|---|---|
| | Type | Amount | Specific compound | | Another photoacid generator | | Type | Amount | Type | Amount | Type | Amount | Type | Mixing ratio (mass ration) |
| | | | Type | Amount | Type | Amount | | | | | | | | |
| Re-1 | A-3 | 88.7 | X-5 | 11.3 | - | - | - | - | - | - | - | - | F-1/F-8 | 85/15 |
| Rc-2 | A-1 | 76.4 | X-9 | 23.6 | - | - | - | - | - | - | - | - | F-1/F-2 | 70/30 |
| Re-3 | A-6 | 90.8 | X-14 | 9.2 | - | - | - | - | - | - | - | - | F-1/F-7 | 80/20 |
| Re-4 | A-9 | 85.0 | X-11 | 15.0 | - | - | - | - | - | - | - | - | F-4 | 100 |
| Re-5 | A-11 | 88.4 | X-4 | 11.5 | - | - | - | - | - | - | E-3 | 0.1 | F-1/F-9 | 90/10 |
| Rc-6 | A-7 | 83.3 | X-15 | 16.7 | - | - | - | - | - | - | - | - | F-1/F-6 | 40/60 |
| Rc-7 | A-2 | 88.7 | X-7 | 11.3 | - | - | - | - | - | - | - | - | F-1/F-5 | 50/50 |
| Re-8 | A-8 | 87.1 | X-8 | 12.9 | - | - | - | - | - | - | - | - | F-1 | 100 |
| Re-9 | A-10 | 90.2 | X-17 | 9.8 | - | - | - | - | - | - | - | - | F-1/F-2/F-8 | 70/25/5 |
| Re-10 | A-4 | 80.0 | X-19 | 20.0 | - | - | - | - | - | - | - | - | F-1 | 100 |
| Re-11 | A-5 | 85.5 | X-2 | 14.5 | - | - | - | - | - | - | - | - | F-7 | 100 |
| Re-12 | A-6 | 86.8 | X-1 | 13.0 | - | - | - | - | - | - | E-1/E-2 | 0.1/0.1 | F-1/F-2 | 70/30 |
| Re-13 | A-1 | 89.8 | X-6 | 10.2 | - | - | - | - | - | - | - | - | F-1/F-3 | 90/10 |
| Re-14 | A-12 | 93.7 | X-16 | 6.3 | - | - | - | - | - | - | - | - | F-1/F-7 | 80/20 |
| Re-15 | A-10 | 82.2 | X-18 | 17.8 | - | - | - | - | - | - | - | - | F-1/F-8 | 85/15 |
| Re-16 | A-9 | 89.9 | X-12 | 10.1 | - | - | - | - | - | - | - | - | F-4 | 100 |
| Re-17 | A-5 | 84.6 | X-13 | 15.4 | - | - | - | - | - | - | - | - | F-1F-5 | 50/50 |
| Re-18 | A-3 | 87.1 | X-3 | 12.8 | - | - | - | - | - | - | E-1 | 0.1 | F-1 | 100 |
| Re-19 | A-2 | 86.4 | X-10 | 13.6 | - | - | - | - | - | - | - | - | F-1/F-2/F-8 | 70/25/5 |
| Re-20 | A-1 | 83.4 | X-20 | 16.4 | - | - | - | - | - | - | E-1/E-2 | 0.1/0.1 | F-1/F-6 | 40/60 |
| Re-21 | A-9 | 86.5 | X-12 | 10.1 | B-1 | 3.4 | - | - | - | - | - | - | F-4 | 100 |
| Re-22 | A-3/A-6 | 45.9/30.0 | X-3 | 17.1 | B-2 | 6.9 | - | - | - | - | E-1 | 0.1 | F-1 | 100 |
| Rc-23 | A-4 | 76.4 | X-19 | 20.0 | B-5 | 3.6 | - | - | - | - | - | - | F-1 | 100 |
| Rc-24 | A-1 | 84.7 | X-6 | 10.2 | B-6 | 5.1 | - | - | - | - | - | - | F-1/F-3 | 90/10 |
| Re-25 | A-1 | 76.7 | X-9 | 18.3 | B-3/B-4 | 3.0/2.0 | - | - | - | - | - | - | F-1/F-2 | 70/30 |
| Re-26 | A-9 | 84.5 | X-11 | 15.0 | - | - | C-1 | 0.5 | - | - | - | - | F-4 | 100 |
| Re-27 | A-11 | 88.0 | X-4 | 11.5 | - | - | C-2 | 0.4 | - | - | E-3 | 0.1 | F-1/F-9 | 90/10 |
| Re-28 | A-6 | 85.0 | X-1 | 13.0 | - | - | C-3 | 1.8 | - | - | E-1/E-2 | 0.1/0.1 | F-1/F-2 | 70/30 |
| Re-29 | A-3 | 87.9 | X-5 | 11.3 | - | - | C-4 | 0.8 | - | - | - | - | F-1/F-8 | 85/15 |
| Re-30 | A-5 | 83.3 | X-2 | 14.5 | B-4 | 1.1 | C-5 | 1.1 | - | - | - | - | F-7 | 100 |
| Re-31 | A-15 | 83.8 | X-1 | 15.1 | - | - | - | - | D-1 | 1.1 | - | - | F-1/F-8 | 85/15 |
| Re-32 | A-13 | 89.4 | X-6 | 9.9 | - | - | - | - | D-5 | 0.5 | E-1/E-2 | 0.1/0.1 | F-1/F-2 | 70/30 |
| Re-33 | A-20 | 80.5 | X-11 | 16.9 | - | - | - | - | D-4 | 2.5 | E-3 | 0.1 | F-1/F-7 | 80/20 |
| Re-34 | A-18 | 84.3 | X-18 | 13.1 | - | - | - | - | D-3 | 2.5 | E-1 | 0.1 | F-4 | 100 |
| Re-35 | A-14/A-15 | 40.4/40.3 | X-12 | 15.1 | - | - | - | - | D-4 | 4.2 | - | - | F-1/F-9 | 90/10 |
| Re-36 | A-17 | 76.9 | X-8 | 21.6 | - | - | - | - | D-1 | 1.5 | - | - | F-1/F-6 | 40/60 |
| Re-37 | A-16 | 79.9 | X-20 | 18.6 | - | - | - | - | D-6 | 1.5 | - | - | F-1F-5 | 50/50 |
| Re-38 | A-19 | 73.4 | X-3 | 25.8 | - | - | - | - | D-2 | 0.8 | - | - | F-1/F-3 | 70/30 |
| Re-39 | A-15 | 81.6 | X-1 | 15.1 | B-3 | 2.2 | - | - | D-1 | 1.1 | - | | F-1/F-8 | 85/15 |
| Re-40 | A-17 | 75.1 | X-8 | 21.6 | B-1 | 1.8 | - | - | D-1 | 1.5 | - | - | F-1/F-6 | 40/60 |
| Re-41 | A-18 | 81.5 | X-18 | 13.1 | B-2 | 2.8 | - | - | D-3 | 2.5 | E-1 | 0.1 | F-4 | 100 |
| Re-42 | A-14/A-15 | 40.4/40.0 | X-12 | 15.1 | - | - | C-2 | 0.3 | D-4 | 4.2 | - | - | F-1/F-9 | 90/10 |
| Re-43 | A-20 | 80.1 | X-11 | 16.9 | - | - | C-1 | 0.4 | D-4 | 2.5 | E-3 | 0.1 | F-1/F-7 | 80/20 |
| Re-44 | A-13 | 88.6 | X-6 | 9.9 | - | - | C-4 | 0.8 | D-5 | 0.5 | E-1/E-2 | 0.1/0.1 | F-1/F-2 | 70/30 |
| Re-45 | A-19 | 73.4 | X-3 | 25.8 | B-1 | 2.3 | C-5 | 1.5 | D-2 | 0.8 | - | - | F-1/F-3 | 70/30 |
| Re-46 | A-15 | 84.9 | X-1 | 15.1 | - | - | - | - | | | - | | F-1/F-8 | 85/15 |
| Re-47 | A-13 | 89.9 | X-6 | 9.9 | - | - | - | - | | | E-1/E-2 | 0.1/0.1 | F-1/F-2 | 70/30 |
| Re-48 | A-20 | 83.0 | X-11 | 16.9 | - | - | - | - | | | E-3 | 0.1 | F-1/F-7 | 80/20 |
| Re-49 | A-6 | 85.6 | - | - | Z-1 | 14.2 | - | - | - | - | E-1/E-2 | 0.1/0.1 | F-1/F-2 | 70/30 |
| Re-50 | A-15 | 84.0 | - | - | Z-1 | 14.9 | - | - | D-1 | 1.1 | - | | F-1/F-8 | 85/15 |

**[Table 4]**

| Table 3-2 | Acid-decomposable resin | | Photoacid generator | | | | Acid diffusion control agent | | Hydrophobic resin | | Resin | | Solvent | |
|---|---|---|---|---|---|---|---|---|---|---|---|---|---|---|
| | Type | Amount | Specific compound | | Another pholoacid generator | | Type | Amount | Type | Amount | Type | Amount | Type | Mixing ratio (mass ration) |
| | | | Type | Amount | Type | Amount | | | | | | | | |
| Re-51 | A-31 | 67.9 | X-22 | 25.0 | B-3 | 4.5 | - | - | D-7 | 2.6 | - | - | F-1/F-7 | 80/20 |
| Re-52 | A-30 | 80.5 | X-32 | 19.5 | - | - | - | - | - | - | - | - | F-1/F-6 | 40/60 |
| Rc-53 | A-22 | 75.9 | X-31 | 22.6 | - | - | - | - | D-6 | 1.5 | - | - | F-1/F-2 | 70/30 |
| Re-54 | A-29 | 83.3 | X-29 | 16.7 | - | - | - | - | - | - | - | - | F-1/F-6 | 40/60 |
| Re-55 | A-28 | 49.8 | X-26 | 50.0 | - | - | - | - | - | - | E-1/ E-2 | 0.1/ 0.1 | F-1/F-8 | 85/15 |
| Re-56 | A-33 | 79.4 | X-33 | 17.5 | - | - | - | - | D-8 | 3.1 | - | - | F-1/F-8 | 85/15 |
| Re-57 | A-36 | 54.1 | X-25 | 44.8 | - | - | - | - | D-1 | 1.1 | - | - | F-1F-5 | 50/50 |
| Re-58 | A-27 | 44.8 | X-41 | 55.2 | - | - | - | - | - | - | - | - | F-1/F-2 | 70/30 |
| Re-59 | A-21 | 69.4 | X-21 | 28.1 | - | - | - | - | D-4 | 2.5 | - | - | F-4 | 100 |
| Re-60 | A-25 | 70.0 | X-27 | 30.0 | - | - | - | - | - | - | - | - | F-1 | 100 |
| Re-61 | A-37 | 77.7 | X-28 | 21.5 | - | - | - | - | D-5 | 0.8 | - | - | F-1/F-9 | 90/10 |
| Re-62 | A-34 | 63.3 | X-43 | 35.6 | - | - | C-4 | 1.1 | - | - | - | - | F-1/F-6 | 40/60 |
| Re-63 | A-35 | 85.4 | X-23 | 14.6 | - | - | - | - | - | - | - | - | F-1/F-5 | 50/50 |
| Re-64 | A-32 | 58.0 | X-42 | 40.0 | - | - | - | - | D-2 | 2 | - | - | F-4 | 100 |
| Re-65 | A-24 | 73.2 | X-34 | 24.3 | - | - | - | - | D-3 | 2.5 | - | - | F-1/F-2/ F-8 | 70/25/5 |
| Re-66 | A-23 | 83.1 | X-24 | 16.9 | - | - | - | - | - | - | - | - | F-1/F-2 | 70/30 |
| Re-67 | A-26 | 57.6 | X-30 | 41.3 | - | - | - | - | D-6 | 1.1 | - | - | F-1 | 100 |
| Re-68 | A-6 | 77.4 | X-35 | 22.6 | - | - | - | - | - | - | - | - | F-1/F-7 | 80/20 |
| Re-69 | A-2 | 68.5 | X-38 | 31.5 | - | - | - | - | - | - | - | - | F-1/F-2/ F-8 | 70/25/5 |
| Re-70 | A-33 | 77.9 | X-39 | 19.0 | - | - | - | - | D-8 | 3.1 | - | - | F-1/F-8 | 85/15 |
| Re-71 | A-22 | 67.3 | X-36 | 31.2 | - | - | - | - | D-6 | 1.5 | - | - | F-1/F-2 | 70/30 |
| Rc-72 | A-1 | 77.6 | X-40 | 22.2 | - | - | - | - | - | - | E-1/ E-2 | 0.1/ 0.1 | F-1/F-6 | 40/60 |
| Re-73 | A-35 | 66.0 | X-37 | 34.0 | - | - | - | - | - | - | - | - | F-1F-5 | 50/50 |
| Re-74 | A-5/ A-24 | 39.8/ 30.0 | X-1 | 30.2 | - | - | - | - | - | - | - | - | F-1/F-8 | 85/15 |
| Re-75 | A-1 | 64.5 | X-2 | 35.5 | - | - | - | - | - | - | - | - | F-7 | 100 |
| Re-76 | A-1 | 39.7 | X-9 | 60.3 | - | - | - | - | - | - | - | - | F-1/F-2 | 70/30 |

### [Production of Topcoat Composition]

In the present Example, in a case where a resist film was manufactured using the resist composition, a topcoat prepared on the resist film was further manufactured as desired.

The components used in the topcoat composition used to form the topcoat and a production procedure therefor are shown below.

### <Resin>

The molar fractions of the repeating units based on the respective monomers, and the weight-average molecular weight (Mw) and the dispersity (Mw/Mn) of each resin of the resin used in the topcoat composition are shown in the following table.

Furthermore, with regard to the structures of the monomers corresponding to the repeating units shown in the table, reference can be made to the above-mentioned monomers shown in the description of <Hydrophobic Resin>.

**[Table 5]**

| Table 4 | Molar ratio of repeating unit 1 | | Molar ratio of repeating unit 2 | | Molar ratio of repeating unit 3 | | Mw | Mw/Mn |
|---|---|---|---|---|---|---|---|---|
| Resin PT-1 | ME-2 | 40 | ME-9 | 30 | ME-7 | 30 | 8,000 | 1.6 |
| Resin PT-2 | ME-2 | 50 | ME-6 | 40 | ME-3 | 10 | 5,000 | 1.5 |
| Resin PT-3 | ME-3 | 30 | ME-4 | 65 | ME-10 | 5 | 8,500 | 1.7 |

### <Additive>

The additives included in the topcoat composition are shown below.

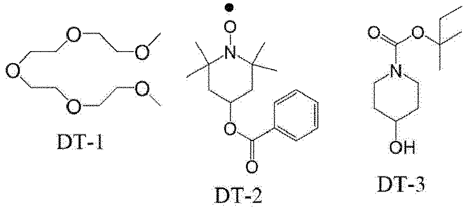

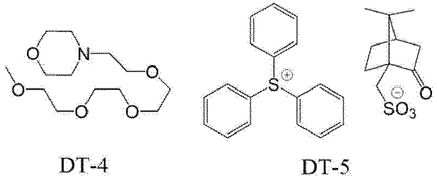

### <Surfactant>

In a case where the topcoat composition included a surfactant, the following surfactant was used.

### E-3: PF656 (manufactured by OMNOVA Solutions Inc., fluorine-based surfactant)

### <Solvent>

Solvents included in the topcoat composition are shown below.
FT-1: 4-Methyl-2-pentanol (MIBC)
FT-2: n-Decane
FT-3: Diisoamyl ether

### <Preparation of Topcoat Composition>

A solution was prepared by dissolving the respective components in a solvent such that a formulation shown in the following table was satisfied and a concentration of solid contents of 3.8% by mass was obtained.

Then, the obtained solution was filtered through a polyethylene filter having a pore size of 0.1 µm to prepare a topcoat composition.

**[Table 6]**

| Table 5 | Resin | | Additive | | Surfactant | | Solvent | |
|---|---|---|---|---|---|---|---|---|
| | Type | Mass (g) | Type | Mass (g) | Type | Mass (g) | Type | Mixing ratio (mass ratio) |
| TC-1 | PT-1 | 10.0 | DT-1/DT-2 | 1.3/0.06 | - | - | FT-1/FT-2 | 70/30 |
| TC-2 | PT-2 | 10.0 | DT-3/DT-4 | 0.04/0.06 | E-3 | 0.005 | FT-1/FT-3 | 75/25 |
| TC-3 | PT-3 | 10.0 | DT-5 | 0.05 | - | - | FT-1/FT-3 | 10/90 |

### [Test]

Using the resist composition prepared as mentioned above, the LWR of a pattern developed under each of the following conditions was evaluated.

Furthermore, in any of the tests, a resist composition which had been left in an environment of 4°C for 3 months after production thereof was used as the resist composition used for pattern formation (actinic ray-sensitive or radiation-sensitive resin composition).

### <EUV Exposure and Organic Solvent Development>

### (Pattern Formation)

A composition for forming an underlayer film, AL412 (manufactured by Brewer Science, Inc.), was applied onto a silicon wafer and baked at 205°C for 60 seconds to form an underlying film having a film thickness of 20 nm. A resist composition shown in Table 6 was applied thereon and baked at 100°C for 60 seconds to form a resist film having a film thickness of 30 nm.

The silicon wafer having the obtained resist film was subjected to patternwise irradiation using an EUV exposure device (manufactured by Exitech Ltd., Micro Exposure Tool, NA 0.3, Quadrupol, outer sigma 0.68, inner sigma 0.36). Furthermore, as the reticle, a mask having a line size = 20 nm and a line: space = 1:1 was used.

The resist film after the exposure was baked at 90°C for 60 seconds, developed with n-butyl acetate for 30 seconds, and spin-dried to obtain a negative tone pattern.

### (Evaluation)

### • LWR

In a case where a 20 nm (1:1) line-and-space pattern formed with an optimum exposure amount by resolving a line pattern having an average line width of 20 nm was observed from the upper part of the pattern using a critical dimension scanning electron microscope (SEM (S-9380II manufactured by Hitachi, Ltd.)). The line width of the pattern was observed at any points (100 points), and a measurement deviation thereof was evaluated with 3σ (nm) and taken as an LWR. A smaller value of LWR indicates better LWR performance. LWR (nm) is preferably 4.2 nm or less, more preferably 3.9 nm or less, and still more preferably 3.5 nm or less.

### • Resolution

In a case where the above-mentioned pattern formation was carried out, the exposure amount was gradually increased or decreased from an optimum exposure amount to form a line-and-space pattern having each line width. At this time, the minimum dimension of a pattern which was resolved without collapsing was determined using a critical dimension scanning electron microscope (SEM (S-9380II, Hitachi, Ltd.)). This was defined as a "limit resolution (nm)". The smaller the limit resolution value, the better the resolution.

In addition, the limit resolution (nm) is preferably 17.0 nm or less, more preferably 14.0 nm or less, and still more preferably 13.0 nm or less.

### The results are shown in the following table.

The "Amount" column in the tables indicates a content (% by mass) of the specific compound with respect to a total solid content of the resist composition.

The "Formula (cation)" column in the tables indicates which of General Formulae (I) to (IV) the organic cation contained in the specific compound used corresponds to.

It should be noted that a case with description of "II-2" means that at least two of the organic cation Ar¹, Ar², or Ar³ are each the organic cation represented by General Formula (II) which is an aromatic hydrocarbon ring group having a substituent.

A case with description of "II-1" means that the organic cation is the cation represented by General Formula (II), which does not correspond to the requirement of "II-2".

A case with description of "-" means that the organic cation did not correspond to any of General Formulae (I) to (IV).

The "Acid group-containing cation" column indicates whether or not the organic cation of the specific compound has an acid group. A case where the present requirement was satisfied was designated as A, and a case where the present requirement was not satisfied was designated as B.

The "Formula (anion)" column indicates whether or not the specific compound used has a group represented by any of General Formulae (B-1) to (B-4) as an anionic moiety. A case where the present requirement was satisfied was designated as A, and a case where the present requirement was not satisfied was designated as B.

**[Table 7]**

| Table 6-1 | Characteristics | | | | | | LWR (nm) after lapse of time (organic solvent development) | Limit resolution (nm) (organic solvent development) |
|---|---|---|---|---|---|---|---|---|
| | Type of Resist composition | Specific compound | | | | | | |
| | | Type | Amount | Formula (cation) | Acid group-containing cation | Formula (anion) | | |
| Example 1-1 | Re-1 | X-5 | 11.3 | II-1 | B | A | 3.8 | 14.6 |
| Example 1-2 | Re-2 | X-9 | 23.6 | III | B | A | 3.4 | 13.1 |
| Example 1-3 | Re-3 | X-14 | 9.2 | - | B | A | 4.2 | 14.5 |
| Example 1-4 | Re-4 | X-11 | 15.0 | IV | B | A | 3.4 | 14.8 |
| Example 1-5 | Re-5 | X-4 | 11.5 | II-2 | B | A | 3.5 | 14.6 |
| Example 1-6 | Re-6 | X-15 | 16.7 | - | B | A | 4.1 | 13.2 |
| Example 1-7 | Re-7 | X-7 | 11.3 | III | B | A | 3.4 | 14.7 |
| Example 1-8 | Re-8 | X-8 | 12.9 | III | B | A | 3.5 | 14.7 |
| Example 1-9 | Re-9 | X-17 | 9.8 | III | B | B | 3.8 | 14.8 |
| Example 1-10 | Re-10 | X-19 | 20.0 | II-2 | B | B | 3.9 | 13.3 |
| Example 1-11 | Re-11 | X-2 | 14.5 | II-2 | B | A | 3.5 | 14.5 |
| Example 1-12 | Re-12 | X-1 | 13.0 | II-2 | B | A | 3.4 | 14.7 |
| Example 1-13 | Re-13 | X-6 | 10.2 | II-1 | B | A | 3.7 | 14.6 |
| Example 1-14 | Re-14 | X-16 | 6.3 | II-2 | B | B | 3.9 | 14.6 |
| Example 1-15 | Re-15 | X-18 | 17.8 | IV | B | B | 3.9 | 13.5 |
| Example 1-16 | Re-16 | X-12 | 10.1 | - | B | A | 4.1 | 14.4 |
| Example 1-17 | Re-17 | X-13 | 15.4 | - | B | A | 4.2 | 14.3 |
| Example 1-18 | Re-18 | X-3 | 12.8 | II-2 | B | A | 3.5 | 14.6 |
| Example 1-19 | Re-19 | X-10 | 13.6 | IV | B | A | 3.4 | 14.7 |
| Example 1-20 | Re-20 | X-20 | 16.4 | IV | B | B | 3.8 | 13.3 |
| Example 1-21 | Re-21 | X-12 | 10.1 | - | B | A | 4.2 | 14.6 |
| Example 1-22 | Re-22 | X-3 | 17.1 | II-2 | B | A | 3.5 | 13.4 |
| Example 1-23 | Re-23 | X-19 | 20.0 | II-2 | B | B | 3.9 | 13.2 |
| Example 1-24 | Re-24 | X-6 | 10.2 | II-1 | B | A | 3.8 | 14.6 |
| Example 1-25 | Re-25 | X-9 | 18.3 | III | B | A | 3.4 | 13.5 |
| Example 1-26 | Re-26 | X-11 | 15.0 | IV | B | A | 3.5 | 14.5 |
| Example 1-27 | Re-27 | X-4 | 11.5 | II-2 | B | A | 3.4 | 14.6 |
| Example 1-28 | Re-28 | X-1 | 13.0 | II-2 | B | A | 3.4 | 14.8 |
| Example 1-29 | Re-29 | X-5 | 11.3 | II-1 | B | A | 3.8 | 14.6 |
| Example 1-30 | Re-30 | X-2 | 14.5 | II-2 | B | A | 3.4 | 14.5 |
| Comparative Example 1 | Re-49 | - | - | - | - | - | 5.2 | 17.8 |

**[Table 8]**

| Table 6-2 | Characteristics | | | | | | LWR (nm) after lapse of time (organic solvent development) | Limit resolution (nm) (organic solvent development) |
|---|---|---|---|---|---|---|---|---|
| | Type of Resist composition | Specific compound | | | | | | |
| | | Type | Amount | Formula (cation) | Acid group-containing cation | Formula (anion) | | |
| Example 1-31 | Re-51 | X-22 | 25.0 | - | B | A | 4.1 | 13.4 |
| Example 1-32 | Re-52 | X-32 | 19.5 | - | B | A | 4.0 | 13.4 |
| Example 1-33 | Re-53 | X-31 | 22.6 | - | B | A | 4.2 | 13.3 |
| Example 1-34 | Re-54 | X-29 | 16.7 | - | A | A | 3.8 | 13.4 |
| Example 1-35 | Re-55 | X-26 | 50.0 | IV | B | A | 3.5 | 13.5 |
| Example 1-36 | Re-56 | X-33 | 17.5 | - | B | A | 4.0 | 13.3 |
| Example 1-37 | Re-57 | X-25 | 44.8 | II-2 | B | A | 3.4 | 13.4 |
| Example 1-38 | Re-58 | X-41 | 55.2 | II-2 | B | A | 3.4 | 13.6 |
| Example 1-39 | Re-59 | X-21 | 28.1 | - | B | A | 4.2 | 13.2 |
| Example 1-40 | Re-60 | X-27 | 30.0 | III | B | B | 3.9 | 13.4 |
| Example 1-41 | Re-61 | X-28 | 21.5 | III | A | A | 3.2 | 13.5 |
| Example 1-42 | Re-62 | X-43 | 35.6 | II-2 | B | A | 3.4 | 12.5 |
| Example 1-43 | Re-63 | X-23 | 14.6 | IV | B | A | 3.4 | 14.6 |
| Example 1-44 | Re-64 | X-42 | 40.0 | II-2 | B | A | 3.5 | 12.2 |
| Example 1-45 | Re-65 | X-34 | 24.3 | - | B | B | 4.4 | 13.5 |
| Example 1-46 | Re-66 | X-24 | 16.9 | - | B | A | 4.1 | 13.6 |
| Example 1-47 | Re-67 | X-30 | 41.3 | II-2 | B | A | 3.5 | 13.5 |
| Example 1-48 | Re-68 | X-35 | 22.6 | IV | A | A | 3.2 | 13.3 |
| Example 1-49 | Re-69 | X-38 | 31.5 | - | A | A | 3.9 | 13.6 |
| Example 1-50 | Re-70 | X-39 | 19.0 | II-2 | A | A | 3.2 | 13.4 |
| Example 1-51 | Re-71 | X-36 | 31.2 | - | A | A | 3.8 | 13.2 |
| Example 1-52 | Re-72 | X-40 | 22.2 | II-1 | A | A | 3.3 | 13.5 |
| Example 1-53 | Re-73 | X-37 | 34.0 | II-2 | A | A | 3.2 | 13.4 |
| Example 1-54 | Re-74 | X-1 | 30.2 | II-2 | B | A | 3.4 | 13.4 |
| Example 1-55 | Re-75 | X-2 | 35.5 | II-2 | B | A | 3.4 | 13.3 |
| Example 1-56 | Re-76 | X-9 | 60.3 | III | B | A | 3.5 | 13.6 |

As shown in the table, it was confirmed that in a case where a pattern is obtained by performing EUV exposure and organic solvent development, the resist composition of the embodiment of the present invention is capable of forming a pattern having excellent LWR performance even with a use of the resist composition that has been stored for a long period of time.

In addition, it was confirmed that in a case where the specific compound has an organic cation represented by any of General Formulae (II) to (IV), the effect of the present invention is more excellent (the comparison between Examples in which any of "II-1", "II-2", "III", and "IV" in the "Formula (cation)" column is described and Examples in which "-" is described, among Examples in which the contents in the "Acid group-containing cation" column and the "Formula (anion)" are the same as each other, and the like).

It was confirmed that in a case where the specific compound has a group represented by any of General Formulae (B-1) to (B-4) as an anionic moiety (anionic functional group), the effect of the present invention is more excellent (the comparison between Examples in which the requirements in the "Formula (anion)" column are satisfied and Examples in which such requirements are not satisfied, among Examples in which the contents in the descriptions of the "Formula (cation)" column and the "Acid group-containing cation" column are the same as each other, and the like).

It was confirmed that in a case where a specific compound having the organic cation represented by General Formula (II) in which at least two of the organic cation Ar¹, Ar², or Ar³ are each an organic cation represented by General Formula (II) which is an aromatic hydrocarbon ring group having a substituent is used as the specific compound, the effect of the present invention is more excellent, as compared with a case where another specific compound having an organic cation represented by General Formula (II), other than the above specific compound, is used (the comparison between Examples in which "II-2" is described and Examples in which "II-1" is described, each in the "Formula (anion)" column, among Examples in which the contents in the "Acid group-containing cation" column and the "Formula (cation)" column are the same as each other, and the like).

It was confirmed that in a case where the cationic moiety of the specific compound has at least one acid group, the effect of the present invention is more excellent (the comparison between Examples in which the requirements in the "Acid group-containing cation" column are satisfied and Examples in which such requirements are not satisfied, among Examples in which the descriptions of the "Formula (cation)" column and the "Formula (anion)" column are the same as each other, and the like).

It was confirmed that in a case where the content of the specific compound was more than 16.0% by mass with respect to the total solid content of the resist composition, the resolution of the obtained pattern was more excellent (the results of Examples 1-1 to 1-56, and the like).

In addition, it was confirmed that in a case where the content of the specific compound which is compound (II) is more than 20.0% by mass with respect to the total solid content of the resist composition, the resolution of the obtained pattern is more excellent (the results of Examples 1-42 and 1-44, and the like).

### <EUV Exposure and Alkali Development>

### (Pattern Formation)

A composition for forming an underlayer film, AL412 (manufactured by Brewer Science, Inc.), was applied onto a silicon wafer and baked at 205°C for 60 seconds to form an underlying film having a film thickness of 20 nm. A resist composition shown in Table 7 was applied thereon and baked at 100°C for 60 seconds to form a resist film having a film thickness of 30 nm.

The silicon wafer having the obtained resist film was subjected to patternwise irradiation using an EUV exposure device (manufactured by Exitech Ltd., Micro Exposure Tool, NA 0.3, Quadrupol, outer sigma 0.68, inner sigma 0.36). Furthermore, as the reticle, a mask having a line size = 20 nm and a line: space = 1:1 was used.

The resist film after the exposure was baked at 90°C for 60 seconds, developed with an aqueous tetramethylammonium hydroxide solution (2.38%-by-mass) for 30 seconds, and then rinsed with pure water for 30 seconds. Thereafter, the resist film was spin-dried to obtain a positive tone pattern.

### (Evaluation)

The obtained pattern was evaluated in the same manner as in the evaluations of the LWR and the resolution of a pattern in <EUV Exposure and Organic Solvent Development>.

The results are shown in the following table. The meaning of each column in the table is as described above.

**[Table 9]**

| Table 7-1 | Characteristics | | | | | | LWR (nm) after lapse of time (alkali development) | Limit resolution (nm) (alkali development) |
|---|---|---|---|---|---|---|---|---|
| | Type of Resist composition | Specific compound | | | | | | |
| | | Type | Amount | Formula (cation) | Acid group-containing cation | Formula (anion) | | |
| Example 2-1 | Re-1 | X-5 | 11.3 | II-1 | B | A | 3.8 | 14.5 |
| Example 2-2 | Re-2 | X-9 | 23.6 | III | B | A | 3.5 | 13.2 |
| Example 2-3 | Re-3 | X-14 | 9.2 | - | B | A | 4.1 | 14.6 |
| Example 2-4 | Re-4 | X-11 | 15.0 | IV | B | A | 3.5 | 14.5 |
| Example 2-5 | Re-5 | X-4 | 11.5 | II-2 | B | A | 3.5 | 14.5 |
| Example 2-6 | Re-6 | X-15 | 16.7 | - | B | A | 4.2 | 13.3 |
| Example 2-7 | Re-7 | X-7 | 11.3 | III | B | A | 3.4 | 14.6 |
| Example 2-8 | Re-8 | X-8 | 12.9 | III | B | A | 3.5 | 14.7 |
| Example 2-9 | Re-9 | X-17 | 9.8 | III | B | B | 3.9 | 14.6 |
| Example 2-10 | Re-10 | X-19 | 20.0 | II-2 | B | B | 3.8 | 13.2 |
| Example 2-11 | Re-11 | X-2 | 14.5 | II-2 | B | A | 3.4 | 14.7 |
| Example 2-12 | Re-12 | X-1 | 13.0 | II-2 | B | A | 3.4 | 14.4 |
| Example 2-13 | Re-13 | X-6 | 10.2 | II-1 | B | A | 3.8 | 14.6 |
| Example 2-14 | Re-14 | X-16 | 6.3 | II-2 | B | B | 3.9 | 14.6 |
| Example 2-15 | Re-15 | X-18 | 17.8 | IV | B | B | 3.9 | 13.3 |
| Example 2-16 | Re-16 | X-12 | 10.1 | - | B | A | 4.1 | 14.3 |
| Example 2-17 | Re-17 | X-13 | 15.4 | - | B | A | 4.0 | 14.4 |
| Example 2-18 | Re-18 | X-3 | 12.8 | II-2 | B | A | 3.5 | 14.4 |
| Example 2-19 | Re-19 | X-10 | 13.6 | IV | B | A | 3.5 | 14.6 |
| Example 2-20 | Re-20 | X-20 | 16.4 | IV | B | B | 3.9 | 13.2 |
| Example 2-21 | Re-21 | X-12 | 10.1 | - | B | A | 4.2 | 14.7 |
| Example 2-22 | Re-22 | X-3 | 17.1 | II-2 | B | A | 3.4 | 13.3 |
| Example 2-23 | Re-23 | X-19 | 20.0 | II-2 | B | B | 3.9 | 13.3 |
| Example 2-24 | Re-24 | X-6 | 10.2 | II-1 | B | A | 3.8 | 14.8 |
| Example 2-25 | Re-25 | X-9 | 18.3 | III | B | A | 3.5 | 13.3 |
| Example 2-26 | Re-26 | X-11 | 15.0 | IV | B | A | 3.5 | 14.7 |
| Example 2-27 | Re-27 | X-4 | 11.5 | II-2 | B | A | 3.4 | 14.6 |
| Example 2-28 | Re-28 | X-1 | 13.0 | II-2 | B | A | 3.5 | 14.5 |
| Example 2-29 | Re-29 | X-5 | 11.3 | II-1 | B | A | 3.9 | 14.7 |
| Example 2-30 | Re-30 | X-2 | 14.5 | II-2 | B | A | 3.5 | 14.7 |
| Comparative Example 2 | Re-49 | - | - | - | - | - | 5.3 | 17.9 |

**[Table 10]**

| Table 7-2 | Characteristics | | | | | | LWR (nm) after lapse of time (alkali development) | Limit resolution (nm) (alkali development) |
|---|---|---|---|---|---|---|---|---|
| | Type of Resist composition | Specific compound | | | | | | |
| | | Type | Amount | Formula (cation) | Acid group-containing cation | Formula (anion) | | |
| Example 2-31 | Re-51 | X-22 | 25.0 | - | B | A | 4.0 | 13.3 |
| Example 2-32 | Re-52 | X-32 | 19.5 | - | B | A | 4.0 | 13.2 |
| Example 2-33 | Re-53 | X-31 | 22.6 | - | B | A | 4.1 | 13.3 |
| Example 2-34 | Re-54 | X-29 | 16.7 | - | A | A | 3.9 | 13.4 |
| Example 2-35 | Re-55 | X-26 | 50.0 | IV | B | A | 3.5 | 13.5 |
| Example 2-36 | Re-56 | X-33 | 17.5 | - | B | A | 4.3 | 13.3 |
| Example 2-37 | Re-57 | X-25 | 44.8 | II-2 | B | A | 3.4 | 13.3 |
| Example 2-38 | Re-58 | X-41 | 55.2 | II-2 | B | A | 3.5 | 13.5 |
| Example 2-39 | Re-59 | X-21 | 28.1 | - | B | A | 4.2 | 13.6 |
| Example 2-40 | Re-60 | X-27 | 30.0 | III | B | B | 3.8 | 13.4 |
| Example 2-41 | Re-61 | X-28 | 21.5 | III | A | A | 3.2 | 13.2 |
| Example 2-42 | Re-62 | X-43 | 35.6 | II-2 | B | A | 3.5 | 12.4 |
| Example 2-43 | Re-63 | X-23 | 14.6 | IV | B | A | 3.5 | 14.7 |
| Example 2-44 | Re-64 | X-42 | 40.0 | II-2 | B | A | 3.4 | 12.1 |
| Example 2-45 | Re-65 | X-34 | 24.3 | - | B | B | 4.4 | 13.6 |
| Example 2-46 | Re-66 | X-24 | 16.9 | - | B | A | 4.2 | 13.5 |
| Example 2-47 | Re-67 | X-30 | 41.3 | II-2 | B | A | 3.5 | 13.6 |
| Example 2-48 | Re-68 | X-35 | 22.6 | IV | A | A | 3.2 | 13.3 |
| Example 2-49 | Re-69 | X-38 | 31.5 | - | A | A | 3.9 | 13.6 |
| Example 2-50 | Re-70 | X-39 | 19.0 | II-2 | A | A | 3.2 | 13.5 |
| Example 2-51 | Re-71 | X-36 | 31.2 | - | A | A | 3.8 | 13.4 |
| Example 2-52 | Re-72 | X-40 | 22.2 | II-1 | A | A | 3.3 | 13.5 |
| Example 2-53 | Re-73 | X-37 | 34.0 | II-2 | A | A | 3.1 | 13.4 |
| Example 2-54 | Re-74 | X-1 | 30.2 | II-2 | B | A | 3.5 | 13.6 |
| Example 2-55 | Re-75 | X-2 | 35.5 | II-2 | B | A | 3.4 | 13.4 |
| Example 2-56 | Re-76 | X-9 | 60.3 | III | B | A | 3.4 | 13.6 |

As shown in the table, it was confirmed that in a case where a pattern is obtained by performing EUV exposure and alkali development, the resist composition of the embodiment of the present invention is capable of forming a pattern having excellent LWR performance even with a use of the resist composition that has been stored for a long period of time.

In addition, it was confirmed that in a case where the specific compound has an organic cation represented by any of General Formulae (II) to (IV), the effect of the present invention is more excellent (the comparison between Examples in which any of "II-1", "II-2", "III", and "IV" in the "Formula (cation)" column is described and Examples in which "-" is described, among Examples in which the descriptions in the "Acid group-containing cation" column and the "Formula (anion)" column are the same as each other, and the like).

It was confirmed that in a case where the specific compound has a group represented by any of General Formulae (B-1) to (B-4) as an anionic moiety (anionic functional group), the effect of the present invention is more excellent (the comparison between Examples in which the requirements in the "Formula (anion)" column are satisfied and Examples in which such requirements are not satisfied, among Examples in which the contents in the descriptions of the "Formula (cation)" column and the "Acid group-containing cation" column are the same as each other, and the like).

It was confirmed that in a case where a specific compound having the organic cation represented by General Formula (II) in which at least two of the organic cation Ar¹, Ar², or Ar³ are each an organic cation represented by General Formula (II) which is an aromatic hydrocarbon ring group having a substituent is used as the specific compound, the effect of the present invention is more excellent, as compared with a case where another specific compound having an organic cation represented by General Formula (II), other than the above specific compound, is used (the comparison between Examples in which "II-2" is described and Examples in which "II-1" is described, each in the "Formula (anion)" column, among Examples in which the contents in the "Acid group-containing cation" column and the "Formula (cation)" column are the same as each other, and the like).

It was confirmed that in a case where the cationic moiety of the specific compound has at least one acid group, the effect of the present invention is more excellent (the comparison between Examples in which the requirements in the "Acid group-containing cation" column are satisfied and Examples in which such requirements are not satisfied, among Examples in which the descriptions of the "Formula (cation)" column and the "Formula (anion)" column are the same as each other, and the like).

It was confirmed that in a case where the content of the specific compound was more than 16.0% by mass with respect to the total solid content of the resist composition, the resolution of the obtained pattern was more excellent (the results of Examples 2-1 to 2-56, and the like).

In addition, it was confirmed that in a case where the content of the specific compound which is compound (II) is more than 20.0% by mass with respect to the total solid content of the resist composition, the resolution of the obtained pattern is more excellent (the results of Examples 2-42 and 2-44, and the like).

### <ArF Liquid Immersion Exposure and Organic Solvent Development>

### (Pattern Formation)

A composition for forming an organic antireflection film, ARC29SR (manufactured by Brewer Science, Inc.), was applied onto a silicon wafer and baked at 205°C for 60 seconds to form an antireflection film having a film thickness of 98 nm. The resist composition shown in Table 8 was applied thereon and baked at 100°C for 60 seconds to form a resist film (actinic ray-sensitive or radiation-sensitive film) having a film thickness of 90 nm.

Furthermore, in Example 3-16, Example 3-17, and Example 3-18, a topcoat film was formed on the upper layer of the resist film (the types of topcoat compositions used are shown in Table 8). The film thickness of the topcoat film was 100 nm in any case.

The resist film was exposed through a 6% halftone mask having a 1:1 line-and-space pattern with a line width of 45 nm, using an ArF excimer laser liquid immersion scanner (XT1700i, manufactured by ASML, NA 1.20, Dipole, outer sigma: 0.950, inner sigma: 0.850, Y deflection). Ultrapure water was used as the immersion liquid.

The resist film after the exposure was baked at 90°C for 60 seconds, developed with n-butyl acetate for 30 seconds, and then rinsed with 4-methyl-2-pentanol for 30 seconds. Then, the film was spin-dried to obtain a negative tone pattern.

### (Evaluation)

In a case where a 45 nm (1:1) line-and-space pattern formed with an optimum exposure amount by resolving a line pattern having an average line width of 45 nm was observed from the upper part of the pattern using a critical dimension scanning electron microscope (SEM (S-9380II manufactured by Hitachi, Ltd.)). The line width of the pattern was observed at any points (100 points), and a measurement deviation thereof was evaluated with 3σ (nm) and taken as an LWR. A smaller value of LWR indicates better LWR performance. LWR (nm) is preferably 4.0 nm or less, more preferably 2.9 nm or less, and still more preferably 2.5 nm or less.

The results are shown in the following table. The meaning of each column in the table is as described above.

**[Table 11]**

| Table 8 | Characteristics | | | | | LWR (nm) after lapse of time (organic solvent development) |
|---|---|---|---|---|---|---|
| | Type of Resist composition | Specific compound | | | Topcoat composition | |
| | | Type | Formula (cation) | Formula (anion) | | |
| Example 3-1 | Re-31 | X-1 | II-2 | A | - | 2.5 |
| Example 3-2 | Re-32 | X-6 | II-1 | A | - | 2.8 |
| Example 3-3 | Re-33 | X-11 | IV | A | - | 2.4 |
| Example 3-4 | Re-34 | X-18 | IV | B | - | 2.9 |
| Example 3-5 | Re-35 | X-12 | - | A | - | 3.1 |
| Example 3-6 | Re-36 | X-8 | III | A | - | 2.5 |
| Example 3-7 | Re-37 | X-20 | IV | B | - | 2.8 |
| Example 3-8 | Re-38 | X-3 | II-2 | A | - | 2.4 |
| Example 3-9 | Re-39 | X-1 | II-2 | A | - | 2.4 |
| Example 3-10 | Re-40 | X-8 | III | A | - | 2.4 |
| Example 3-11 | Re-41 | X-18 | IV | B | - | 2.9 |
| Example 3-12 | Re-42 | X-12 | - | A | - | 3.1 |
| Example 3-13 | Re-43 | X-11 | IV | A | - | 2.5 |
| Example 3-14 | Re-44 | X-6 | II-1 | A | - | 2.9 |
| Example 3-15 | Re-45 | X-3 | II-2 | A | - | 2.4 |
| Example 3-16 | Re-46 | X-1 | II-2 | A | TC-1 | 2.5 |
| Example 3-17 | Re-47 | X-6 | II-1 | A | TC-2 | 2.8 |
| Example 3-18 | Re-48 | X-11 | IV | A | TC-3 | 2.4 |
| Comparative Example 3 | Re-50 | - | - | - | - | 5.3 |

As shown in the table, it was confirmed that in a case where a pattern is obtained by performing ArF exposure and organic solvent development, the resist composition of the embodiment of the present invention is capable of forming a pattern having excellent LWR performance even with a use of the resist composition that has been stored for a long period of time.

In addition, it was confirmed that in a case where the specific compound has an organic cation represented by any of General Formulae (II) to (IV), the effect of the present invention is more excellent (the comparison between Examples in which any of "II-1", "II-2", "III", and "IV" in the "Formula (cation)" column is described and Examples in which "-" is described, and the like).

It was confirmed that in a case where the specific compound has a group represented by any of General Formulae (B-1) to (B-4) as an anionic moiety (anionic functional group), the effect of the present invention is more excellent (the comparison between Examples in which the requirements in the "Formula (anion)" column are satisfied and Examples in which such requirements are not satisfied, among Examples in which any of "II-2", "III", and "IV" is described in the "Formula (cation)" column, and the like).

It was confirmed that in a case where a specific compound having the organic cation represented by General Formula (II) in which at least two of the organic cation Ar¹, Ar², or Ar³ are each an organic cation represented by General Formula (II) which is an aromatic hydrocarbon ring group having a substituent is used as the specific compound, the effect of the present invention is more excellent, as compared with a case where a specific compound having another organic cation represented by General Formula (II) is used (the comparison between Examples in which "II-2" is described and Examples in which "II-1" is described, each in the "Formula (cation)" column, among Examples in which the requirements in the "Formula (anion)" column are satisfied, and the like).

### <ArF Liquid Immersion Exposure and Alkali Development>

### (Pattern Formation)

A composition for forming an organic antireflection film, ARC29SR (manufactured by Brewer Science, Inc.), was applied onto a silicon wafer and baked at 205°C for 60 seconds to form an antireflection film having a film thickness of 98 nm. A resist composition shown in Table 9 was applied thereon and baked at 100°C for 60 seconds to form a resist film having a film thickness of 90 nm. Furthermore, in Example 4-16, Example 4-17, and Example 4-18, a topcoat film was formed on the upper layer of the resist film (the types of topcoat compositions used are shown in Table 9). The film thickness of the topcoat film was 100 nm in any case.

The resist film was exposed through a 6% halftone mask having a 1:1 line-and-space pattern with a line width of 45 nm, using an ArF excimer laser liquid immersion scanner (XT1700i, manufactured by ASML, NA 1.20, Dipole, outer sigma: 0.950, inner sigma: 0.890, Y deflection). Ultrapure water was used as the immersion liquid.

The resist film after the exposure was baked at 90°C for 60 seconds, developed with an aqueous tetramethylammonium hydroxide solution (2.38%-by-mass) for 30 seconds, and then rinsed with pure water for 30 seconds. Thereafter, the resist film was spin-dried to obtain a positive tone pattern.

### (Evaluation)

The obtained pattern was evaluated in the same manner as in the evaluation of the LWR of a pattern in <ArF Liquid Immersion Exposure and Organic Solvent Development>.

The results are shown in the following table. The meaning of each column in the table is as described above.

**[Table 12]**

| Table 9 | Characteristics | | | | | LWR (nm) after lapse of time (organic solvent development) |
|---|---|---|---|---|---|---|
| | Type of Resist composition | Specific compound | | | Topcoat composition | |
| | | Type | Formula (cation) | Formula (anion) | | |
| Example 4-1 | Re-31 | X-1 | II-2 | A | - | 2.4 |
| Example 4-2 | Re-32 | X-6 | II-1 | A | - | 2.8 |
| Example 4-3 | Re-33 | X-11 | IV | A | - | 2.5 |
| Example 4-4 | Re-34 | X-18 | IV | B | - | 2.9 |
| Example 4-5 | Re-35 | X-12 | - | A | - | 3.0 |
| Example 4-6 | Re-36 | X-8 | III | A | - | 2.4 |
| Example 4-7 | Re-37 | X-20 | IV | B | - | 2.9 |
| Example 4-8 | Re-38 | X-3 | II-2 | A | - | 2.5 |
| Example 4-9 | Re-39 | X-1 | II-2 | A | - | 2.5 |
| Example 4-10 | Re-40 | X-8 | III | A | - | 2.5 |
| Example 4-11 | Re-41 | X-18 | IV | B | - | 2.9 |
| Example 4-12 | Re-42 | X-12 | - | A | - | 3.1 |
| Example 4-13 | Re-43 | X-11 | IV | A | - | 2.5 |
| Example 4-14 | Re-44 | X-6 | II-1 | A | - | 2.8 |
| Example 4-15 | Re-45 | X-3 | II-2 | A | - | 2.5 |
| Example 4-16 | Re-46 | X-1 | II-2 | A | TC-1 | 2.4 |
| Example 4-17 | Re-47 | X-6 | II-1 | A | TC-2 | 2.9 |
| Example 4-18 | Re-48 | X-11 | IV | A | TC-3 | 2.4 |
| Comparative Example 4 | Re-50 | - | - | - | - | 5.1 |

As shown in the table, it was confirmed that in a case where a pattern is obtained by performing ArF exposure and alkali development, the resist composition of the embodiment of the present invention is capable of forming a pattern having excellent LWR performance even with a use of the resist composition that has been stored for a long period of time.

In addition, it was confirmed that in a case where the specific compound has an organic cation represented by any of General Formulae (II) to (IV), the effect of the present invention is more excellent (the comparison between Examples in which any of "II-1", "II-2", "III", and "IV" in the "Formula (cation)" column is described and Examples in which "-" is described, and the like).

It was confirmed that in a case where the specific compound has a group represented by any of General Formulae (B-1) to (B-4) as an anionic moiety (anionic functional group), the effect of the present invention is more excellent (the comparison between Examples in which the requirements in the "Formula (anion)" column are satisfied and Examples in which such requirements are not satisfied, among Examples in which any of "II-2", "III", and "IV" is described in the "Formula (cation)" column, and the like).

It was confirmed that in a case where a specific compound having the organic cation represented by General Formula (II) in which at least two of the organic cation Ar¹, Ar², or Ar³ are each an organic cation represented by General Formula (II) which is an aromatic hydrocarbon ring group having a substituent is used as the specific compound, the effect of the present invention is more excellent, as compared with a case where a specific compound having another organic cation represented by General Formula (II) is used (the comparison between Examples in which "II-2" is described and Examples in which "II-1" is described, each in the "Formula (cation)" column, among Examples in which the requirements in the "Formula (anion)" column are satisfied, and the like).

## Claims

1. An actinic ray-sensitive or radiation-sensitive resin composition comprising:
an acid-decomposable resin; and
a specific compound,
wherein the specific compound has two or more cationic moieties and the same number of anionic moieties as that of the cationic moieties, and at least one of the cationic moieties has a group represented by General Formula (I),
^{∗}-Ar^{x}-(RI)ₙᵢ (I)
in General Formula (I), ^{∗} represents a bonding position,
Ar^{x} represents an aromatic hydrocarbon ring group,
ni represents an integer of 1 or more, and
RI represents a substituent having no cationic functional group.

2. The actinic ray-sensitive or radiation-sensitive resin composition according to claim 1,
wherein the specific compound has one or more organic cations selected from the group consisting of an organic cation represented by General Formula (II), an organic cation represented by General Formula (III), and an organic cation represented by General Formula (IV), 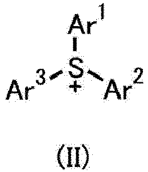
in General Formula (II), Ar¹, Ar², and Ar³ each independently represent an aromatic hydrocarbon ring group which may have a substituent, and
at least one of Ar¹, Ar², or Ar³ represents an aromatic hydrocarbon ring group having a substituent having -CF₃,
provided that all of Ar¹, Ar², and Ar³ have no substituent having a cationic functional group, 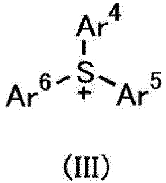
in General Formula (III), Ar⁴, Ar⁵, and Ar⁶ each independently represent an aromatic hydrocarbon ring group which may have a substituent,
at least two of Ar⁴, Ar⁵, or Ar⁶ each represent an aromatic hydrocarbon ring group having a substituent,
at least one of the aromatic hydrocarbon ring groups having a substituent represents an aromatic hydrocarbon ring group having a fluorine atom as the substituent, and
at least two of Ar⁴, Ar⁵, or Ar⁶ represent groups having different structures,
provided that all of Ar⁴, Ar⁵, and Ar⁶ have no substituent having a cationic functional group, 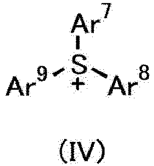
in General Formula (IV), Ar⁷, Ar⁸, and Ar⁹ each independently represent an aromatic hydrocarbon ring group which may have a substituent,
at least one of Ar⁷, Ar⁸, or Ar⁹ represents an aromatic hydrocarbon ring group having one or more specific hydrocarbon groups selected from the group consisting of an alkyl group and a polycyclic cycloalkyl group as the substituent,
provided that all of Ar⁷, Ar⁸, and Ar⁹ have no substituent having a cationic functional group, and
all of Ar⁷, Ar⁸, and Ar⁹ have no substituent having a fluorine atom.

3. The actinic ray-sensitive or radiation-sensitive resin composition according to claim 2,
wherein in General Formula (II), at least two of Ar¹, Ar², or Ar³ each represent an aromatic hydrocarbon ring group having a substituent.

4. The actinic ray-sensitive or radiation-sensitive resin composition according to any one of claims 1 to 3,
wherein the specific compound has an anionic moiety A_{B}⁻ and the anionic moiety A_{B}⁻is a group represented by any of General Formulae (BX-1) to (BX-4), 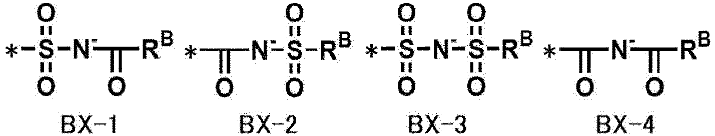
in General Formulae (BX-1) to (BX-4), ^{∗} represents a bonding position, and
in General Formulae (BX-1) to (BX-4), R^{B} represents an organic group.

5. The actinic ray-sensitive or radiation-sensitive resin composition according to claim 4,
wherein the specific compound further has an anionic moiety A_{A}⁻ and the anionic moiety A_{A}⁻ is a group represented by either of General Formula (AX-1) and (AX-2), 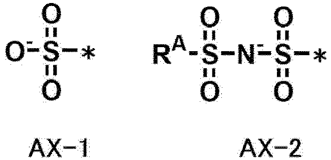
in General Formulae (AX-1) and (AX-2), ^{∗} represents a bonding position, and
in General Formula (AX-2), R^{A} represents an organic group.

6. The actinic ray-sensitive or radiation-sensitive resin composition according to any one of claims 1 to 5,
wherein the cationic moiety has at least one acid group.

7. The actinic ray-sensitive or radiation-sensitive resin composition according to any one of claims 1 to 6,
wherein a content of the specific compound is more than 16.0% by mass with respect to a total solid content of the actinic ray-sensitive or radiation-sensitive resin composition.

8. A resist film formed of the actinic ray-sensitive or radiation-sensitive resin composition according to any one of claims 1 to 7.

9. A pattern forming method comprising:
a step of forming a resist film on a substrate, using the actinic ray-sensitive or radiation-sensitive resin composition according to any one of claims 1 to 7;
a step of exposing the resist film; and
a step of developing the exposed resist film using a developer to form a pattern.

10. A method for manufacturing an electronic device, comprising the pattern forming method according to claim 9.
